# EUROPEAN PATENT APPLICATION

(11) **EP 2 703 007 A1**
(43) Date of publication of application: **05.03.2014**
(21) Application number: 12196199.9
(22) Date of filing: 25.03.2008
(51) Int. Cl.: A61K 39/395, A61K 9/00, A61K 47/18, A61K 47/26, C07K 16/24, A61K 47/12, A61K 47/02

(54) **Antibodies with decreased deamidation profiles**

(30) Priority: 30.03.2007 US 909232 P; 30.03.2007 US 909117 P
(62) Divisional of application: 08744324.8
(71) Applicant: MedImmune, LLC, Gaithersburg, MD 20878 (US)
(72) Inventor: Ram, Kripa, Gaithersburg, MD 20878 (US); Venkat, Raghavan, Gaithersburg, MD 20878 (US)
(74) Representative: Winter, Christopher Spencer

(57) **Abstract**

A stable monoclonal antibody composition with a decreased deamidation profile, wherein the antibody comprises amino acid sequences that predispose the antibody to an elevated deamidation profile.

## Description

### 1. Introduction

The present invention relates to antibodies with decreased deamidation profiles, and methods for producing antibodies with decreased deamidation profiles.

### 2. Background

The stability of protein drugs such as antibodies is adversely affected by many different factors. One of these factors is deamidation. Deamidation is a non-enzymatic chemical reaction in which an amide functional group is removed from an organic compound. The reaction is an important consideration in the degradation of proteins because it alters the amide-containing side chains of the amino acids asparagine and glutamine.

In an example of a biochemical deamidation reaction, the side chain of an asparagine attacks the adjacent peptide group, forming a symmetric succinimide intermediate. The symmetry of the intermediate results in two hydrolysis products, either aspartate or isoaspartate. This process is considered a deamidation reaction because the amide in the asparagine side chain is replaced by a carboxylate group. A similar reaction can also occur in aspartate side chains, yielding a partial conversion to isoaspartate. In the case of glutamine, the rate of deamidation is generally ten fold less than asparagine, however, the mechanism is essentially the same, requiring only water molecules to proceed.

Degradation of proteins and subsequent reduction in protein activity is a recurring problem in the pharmaceutical industry. Accordingly, antibodies that remain stable for extended periods of time and are useful as pharmaceutical agents are desired. To stabilize antibodies, it may be necessary to suppress deamidation of amino acids over time. As discussed above, there are known amino acid sequences that are prone to deamidation. For example, asparagine, such as the asparagine in Asn-Gly containing sequences, is readily deamidated. In addition to glycine adjacent to asparagine, other amino acids have been implicated in facilitating deamidation. At the N+1 position, the amino acids serine, threonine and aspartic acid have also been shown to facilitate deamidation of the adjacent asparagine.

In certain instances, methods of suppressing deamidation by altering amino acids in proteins can be used to improve the value and quality of pharmaceuticals (for example, see U.S. Patent Publication No. 20050171339). In situations when changing the amino acid sequence of a molecule is not desired, other approaches are required. In these situations there is a need for a method of suppressing deamidation of asparagine residues without influencing the activity of proteins, particularly antibodies.

### 3. Summary of the invention

In one embodiment, the invention provides a method of producing an antibody with a decreased deamidation profile, wherein said antibody would otherwise be predisposed to deamidation.

In another embodiment, the invention provides a method of producing an antibody with a decreased deamidation profile, wherein the antibody would otherwise be predisposed to deamidation, the method comprising the following steps: the antibody is produced from cells grown at temperature from about 33°C to about 35°C, the said cells are grown in media with a pH value from 6.7 to 7.1 pH units and the cells are cultured for about 13 to about 19 days.

In another embodiment, the invention provides a stable anti-IFN alpha monoclonal antibody composition with a decreased deamidation profile, wherein the antibody is otherwise predisposed to deamidation.

In another embodiment, the invention provides an antibody composition with a decreased deamidation profile, wherein the antibody is otherwise predisposed to deamidation produced by the process comprising the following steps: the antibody is produced from cells grown at 34°C and the cells are grown in media with a pH of 6.9 units.

In another embodiment, the invention provides an antibody composition with a decreased deamidation profile, wherein the antibody is otherwise predisposed to deamidation produced by the process comprising the following steps: the antibody is produced from cells grown at temperature from about 33°C to about 35°C, the cells are grown in media with a pH value from about 6.7 to about 7.1 pH units, and the cells are cultured for about 13 to about 19 days.

In another embodiment, the invention provide a method of purifying an antibody predisposed to an elevated deamidation profile, wherein the method comprises a wash step during purification for removal of the deamidated species of said antibody.

### 3.1 Brief description of the drawings

Fig 1A. The anti-IFNalpha antibody clone 13H5 heavy chain variable region DNA and amino acid sequences are disclosed. The CDR regions are indicated by the overline.
Fig 1B. The anti-IFNalpha antibody clone 13H5 Kappa chain variable region DNA and amino acid sequences are disclosed. The CDR regions are indicated by the overline.
Fig 2A. The anti-IFNalpha antibody clone 13H7 heavy chain variable region DNA and amino acid sequences are disclosed. The CDR regions are indicated by the overline.
Fig 2B. The anti-IFNalpha antibody clone 13H7 Kappa chain variable region DNA and amino acid sequences are disclosed. The CDR regions are indicated by the overline.
Fig 3A. The anti-IFNalpha antibody clone 7H9 heavy chain variable region DNA and amino acid sequences are disclosed. The CDR regions are indicated by the overline.
Fig 3B. The anti-IFNalpha antibody clone 7H9 Kappa chain variable region DNA and amino acid sequences are disclosed. The CDR regions are indicated by the overline.
Fig 4A. IEC chromatograms of 13H5 species corresponding to various fractions eluted from a column using a linear salt gradient represented in 4B.
Fig 4B. IEC chromatogram of total 13H5 eluted from a column using a linear salt gradient at a gradient slope of 10 column volumes.
Fig 4C. IEC chromatograms of 13H5 species corresponding to various fractions eluted from a column using a linear salt gradient represented in 4D.
Fig 4D. IEC chromatogram of total 13H5 eluted from a column using a linear salt gradient at a gradient slope of 20 column volumes.
Fig 4E. IEC chromatograms of 13H5 species corresponding to various fractions eluted from a column using a linear salt gradient represented in 4F.
Fig 4F. IEC chromatogram of total 13H5 eluted from a column using a linear salt gradient at a gradient slope of 30 column volumes.
Fig 4G. IEC chromatograms of 13H5 species corresponding to various fractions eluted from a column using a linear salt gradient represented in 4H.
Fig 4H. IEC chromatogram of total 13H5 eluted from a column using a linear salt gradient at a gradient slope of 40 column volumes.
[0026] Fig 5. Anti-IFNalpha antibody titres representing actual (dark squares) and estimated intact titre (triangles) and estimated percent deamidation (light squares) of a typical 100L bioreactor run.

### 3.2 Definitions:

In order that the present invention may be more readily understood, certain terms are first defined. Additional definitions are set forth throughout the detailed description.

The terms "interferon alpha", "IFNalpha", "IFNα", "IFN alpha" and "alpha interferon" are used interchangeably and intended to refer to IFN alpha proteins encoded by a functional gene of the interferon alpha gene locus with 75% or greater sequence identity to IFN alpha 1 (Genbank number NP_076918 or protein encoded by Genbank number M_024013). Examples of IFN alpha subtypes include IFN alpha 1, alpha 2a, alpha 2b, alpha 4, alpha 4a, alpha 4b, alpha 5, alpha 6, alpha 7, alpha 8, alpha 10, alpha 13, alpha 14, alpha 16, alpha 17 and alpha 21. The term "interferon alpha" is intended to encompass recombinant forms of the various IFN alpha subtypes, as well as naturally occurring preparations that comprise IFN alpha proteins, such as leukocyte IFN and lymphoblastoid IFN. The term IFN alpha is not intended to encompass IFN omega alone, although a composition that comprises both IFN alpha and IFN omega is encompassed by the term IFN alpha.

The term "anti-interferon alpha antibody" refers to antibodies or antibody fragments specific for polypeptide or polypeptides comprising interferon alpha isoforms family described above. In addition, anti-interferon alpha antibodies of the invention are exemplified in the publications WO 2005/059106 and US 2007/0014724 and the US application serial No. 11/009,410 all entitled "Interferon alpha antibodies and their uses" and which are herein incorporated by reference in their entirety for all purposes. In specific embodiments, anti-interferon alpha antibodies of the invention comprise 13H5, 13H7, and 7H9.

The term "IFN alpha receptor" refers to members of the IFN alpha receptor family of molecules that are receptors for the ligand IFN alpha. Examples of IFN alpha receptors are IFN alpha receptor 1 (Genbank accession number NM_000629) and IFN alpha receptor 2 (Genbank accession numbers: Isoform A, NM_207585.1, Isoform B, NM_000874.3)(Uze et. al. (1990) Cell 60:225; Novick et al. (1994) Cell 77:391).

The term "immune response" refers to the action of, for example, lymphocytes, antigen presenting cells, phagocytic cells, granulocytes, and soluble macromolecules produced by the above cells or the liver (including antibodies, cytokines, and complement) that results in selective damage to, destruction of, or elimination from the human body of invading pathogens, cells or tissues infected with pathogens, cancerous cells, or, in cases of autoimmunity or pathological inflammation, normal human cells or tissues.

A "signal transduction pathway" refers to the biochemical relationship between a variety of signal transduction molecules that play a role in the transmission of a signal from one portion of a cell to another portion of a cell. The phrase "cell surface receptor" includes, for example, molecules and complexes of molecules capable of receiving a signal and the transmission of such a signal across the plasma membrane of a cell. An example of a "cell surface receptor" of the present invention is the IFN alpha receptor 1 or IFN alpha receptor 2.

The term "antibody" as referred to herein includes whole antibodies and any antigen binding fragment (i.e., "antigen-binding portion") or single chains thereof. An "antibody" refers to a glycoprotein comprising at least two heavy (H) chains and two light (L) chains inter-connected by disulfide bonds, or an antigen binding portion thereof. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, (VH or VL)CDR1, FR2, (VH or VL)CDR2, FR3, (VH or VL)CDR3, FR4. The variable regions of the heavy and light chains contain a binding domain that interacts with an antigen. The constant regions of the antibodies may mediate the binding of the immunoglobulin to host tissues or factors, including various cells of the immune system (for example, effector cells) and the first component (C1q) of the classical complement system.

The term "antibody" or "antibodies" includes, but are not limited to, synthetic antibodies, monoclonal antibodies, recombinantly produced antibodies, intrabodies, multispecific antibodies (including bi-specific antibodies), human antibodies, humanized antibodies, chimeric antibodies, synthetic antibodies, single-chain Fvs (scFv) (including bi-specific scFvs), diabodies, BiTE® molcules, single chain antibodies Fab fragments, F(ab') fragments, disulfide-linked Fvs (dsFv), and anti-idiotypic (anti-Id) antibodies, and epitope-binding fragments of any of the above. In particular, antibodies of the present invention include immunoglobulin molecules and immunologically active portions of immunoglobulin molecules, *i.e.*, molecules that contain an antigen binding site that specifically binds to an IFN alpha or IFN alpha antigen (for example, one or more complementarity determining regions (CDRs) of an anti-IFN alpha antibody).

The term "stable" refers to the state of the antibody in the composition with reference to the ability of the antibody to perform its desired function.

The term "antigen-binding portion" of an antibody (or simply "antibody portion"), as used herein, refers to one or more fragments of an antibody that retain the ability to specifically bind to an antigen (for example, IFN alpha). It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see for example, Bird et al. (1988) Science 242:423-426; and Huston et al. (1988) Proc. Natl. Acad. Sci. USA 85:5879-5883). Such single chain antibodies are also intended to be encompassed within the term "antigen-binding portion" of an antibody. These antibody fragments are obtained using conventional techniques known to those with skill in the art, and the fragments are screened for utility in the same manner as are intact antibodies.

An "isolated antibody", as used herein, is intended to refer to an antibody that is substantially free of other antibodies having different antigenic specificities (for example, an isolated antibody that specifically binds IFN alpha is substantially free of antibodies that specifically bind antigens other than IFN alpha). An isolated antibody that specifically binds IFN alpha may, however, have cross-reactivity to other antigens, such as IFN alpha molecules from other species. Moreover, an isolated antibody may be substantially free of other cellular material and/or chemicals.

The terms "monoclonal antibody" or "monoclonal antibody composition" as used herein refer to a preparation of antibody molecules of single molecular composition. A monoclonal antibody composition displays a single binding specificity and affinity for a particular epitope.

The term "human antibody", as used herein, is intended to include antibodies having variable regions in which both the framework and CDR regions are derived from human germline immunoglobulin sequences. Furthermore, if the antibody contains a constant region, the constant region also is derived from human germline immunoglobulin sequences. The human antibodies of the invention may include amino acid residues not encoded by human germline immunoglobulin sequences (for example, mutations introduced by random or site-specific mutagenesis *in vitro* or by somatic mutation *in vivo*).

The term "human monoclonal antibody" refers to antibodies displaying a single binding specificity which have variable regions in which both the framework and CDR regions are derived from human germline immunoglobulin sequences. In one embodiment, the human monoclonal antibodies are produced by a hybridoma which includes a B cell obtained from a transgenic nonhuman animal, for example, a transgenic mouse, having a genome comprising a human heavy chain transgene and a light chain transgene fused to an immortalized cell.

The term "recombinant human antibody", as used herein, includes all human antibodies that are prepared, expressed, created or isolated by recombinant means, such as (a) antibodies isolated from an animal (for example, a mouse) that is transgenic or transchromosomal for human immunoglobulin genes or a hybridoma prepared therefrom, (b) antibodies isolated from a host cell transformed to express the human antibody, for example, from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant human antibodies have variable regions in which the framework and CDR regions are derived from human germline immunoglobulin sequences. In certain embodiments, however, such recombinant human antibodies can be subjected to *in vitro* mutagenesis (or, when an animal transgenic for human Ig sequences is used, *in vivo* somatic mutagenesis) and thus the amino acid sequences of the VH and VL regions of the recombinant antibodies are sequences that, while derived from and related to human germline VH and VL sequences, may not naturally exist within the human antibody germline repertoire *in vivo.*

The term "isotype" refers to the antibody class (for example, IgM or IgG1) that is encoded by the heavy chain constant region genes.

The term "specific binding" or "specifically binds" refers to antibody binding to a predetermined antigen. Typically, the antibody binds with a dissociation constant (KD) of 10⁻⁸ M or less, and binds to the predetermined antigen with a KD that is at least two-fold less than its KD for binding to a non-specific antigen (for example, BSA, casein) other than the predetermined antigen or a closely-related antigen. The phrases "an antibody recognizing an antigen" and " an antibody specific for an antigen" are used interchangeably herein with the term "an antibody which binds specifically to an antigen".

The term "K_{assoc}" or "Kₐ", as used herein, is intended to refer to the association rate of a particular antibody-antigen interaction, whereas the term "K_{dis}" or "K_{d}," as used herein, is intended to refer to the dissociation rate of a particular antibody-antigen interaction. The term "KD", as used herein, is intended to refer to the dissociation constant, which is obtained from the ratio of K_{d} to Kₐ (i.e,. K_{d}/Kₐ) and is expressed as a molar concentration (M). KD values for antibodies can be determined using methods well established in the art. Another method for determining the KD of an antibody is by using surface plasmon resonance, for example, using a biosensor system such as a BIAcore® system.

The term "high affinity" for an IgG antibody refers to an antibody having a KD of 10⁻⁸ M or less, 10⁻⁹ M or less, or 10⁻¹⁰ M or less. However, "high affinity" binding can vary for other antibody isotypes. For example, "high affinity" binding for an IgM isotype refers to an antibody having a KD of 10⁻⁷ M or less, or 10⁻⁸ M or less.

The term "subject" includes any human or nonhuman animal. The term "nonhuman animal" includes all vertebrates, for example, mammals and non-mammals, such as nonhuman primates, sheep, dogs, cats, horses, cows, chickens, amphibians, reptiles, etc.

The term "hydrophobic charge induction chromatography" (or "HCIC") is a type of mixed mode chromatographic process in which the protein of interest in the mixture binds to a dual mode resin through mild hydrophobic interactions in the absence of added salts (for example a lyotropic salts) (Schwart et al. J Chromatogr, 2001;908(1-2):251-63.

The term "hydrophobic charge induction chromatography resin" is a solid phase that contains a ligand which has the combined properties of thiophilic effect (i.e., utilizing the properties of thiophilic chromatography), hydrophobicity and an ionizable group for its separation capability. Thus, an HCIC resin used in a method of the invention contains a ligand that is ionizable and mildly hydrophobic at neutral (physiological) or slightly acidic pH, for example, about pH 5 to 10, or about pH 6 to 9.5. At this pH range, the ligand is predominantly uncharged and binds a protein of interest via mild non-specific hydrophobic interaction. As pH is reduced, the ligand acquires charge and hydrophobic binding is disrupted by electrostatic charge repulsion towards the solute due to the pH shift. Examples of suitable ligands for use in HCIC include any ionizable aromatic or heterocyclic structure (for example those having a pyridine structure, such as 2- aminomethylpyridine, 3-aminomethylpyridine and 4- aminomethylpyridine, 2-mercaptopyridine, 4-mercaptopyridine or 4- mercaptoethylpyridine, mercaptoacids, mercaptoalcohols, imidazolyl based, mercaptomethylimidazole, 2-mercaptobenzimidazole, aminomethylbenzimidazole, histamine, mercaptobenzimidazole, diethylaminopropylamine, aminopropylmorpholine, aminopropylimidazole, aminocaproic acid, nitrohydroxybenzoic acid, - 14 - nitrotyrosine/ethanolamine, dichiorosalicylic acid, dibromotyramine, chlorohydroxyphenylacetic acid, hydroxyphenylacetic acid, tyramine, thiophenol, glutathione, bisuiphate, and dyes, including derivatives thereof see Burton and Harding, Journal of Chromatography A 814: 8 1-81 (1998) and Boschetti, Journal of Biochemical and Biophysical Methods 49: 361-389 (2001), which has an aliphatic chain and at least one sulfur atom on the linker arm and/or ligand structure. A non-limiting example of an HCIC resin includes MEP HYPERCEL® (Pall Corporation; East Hills, NY).

The terms "ion-exchange" and "ion-exchange chromatography" refer to a chromatographic process in which an ionizable solute of interest (for example, a protein of interest in a mixture) interacts with an oppositely charged ligand linked (for example, by covalent attachment) to a solid phase ion exchange material under appropriate conditions of pH and conductivity, such that the solute of interest interacts non-specifically with the charged compound more or less than the solute impurities or contaminants in the mixture. The contaminating solutes in the mixture can be washed from a column of the ion exchange material or are bound to or excluded from the resin, faster or slower than the solute of interest. "Ion- exchange chromatography" specifically includes cation exchange, anion exchange, and mixed mode chromatographies.

The term "cation exchange resin" refers to a solid phase which is negatively charged, and which has free cations for exchange with cations in an aqueous solution passed over or through the solid phase. Any negatively charged ligand attached to the solid phase suitable to form the cation exchange resin can be used, for example, a carboxylate, sulfonate and others as described below. Commercially available cation exchange resins include, but are not limited to, for example, those having a sulfonate based group (for example, MonoS, MiniS, Source 15S and 30S, SP Sepharose Fast Flow,SP Sepharose High Performance from GE Healthcare, Toyopearl SP-650S and SP-650M from Tosoh, Macro-Prep High S from BioRad, Ceramic HyperD 5, Trisacryl M and LS SP and Spherodex LS SP from Pall Technologies,); a sulfoethyl based group (for example, Fractogel SE, from EMD, Poros S- and S-20 from Applied Biosystems); a suiphopropyl based group (for example, TSK Gel SP 5PW and SP-5PW-HR from Tosoh, Poros HS-20 and HS-50 from Applied Biosystems); a sulfoisobutyl based group (for example, (Fractogel EMD SO₃ from EMD); a sulfoxyethyl based group (for example, SE52, SE53 and Express-Ion S from Whatman), a carboxymethyl based group (for example, CM Sepharose Fast Flow from GE Healthcare, Hydrocell CM from Biochrom Labs Inc., Macro-Prep CM from BioRad, Ceramic HyperD CM, Trisacryl M CM, Trisacryl LS CM, from Pall Technologies, Matrx Cellufine C500 and C200 from Millipore, CM52, CM32, CM23 and Express Ion C from Whatman, Toyopearl CM-650S, CM-650M and CM- 650C from Tosoh); sulfonic and carboxylic acid based groups (for example BAKERBOND Carboxy-Sulfon from J.T. Baker); a carboxylic acid based group (for example, WP CBX from J.T Baker, DOWEX MAC-3 from Dow Liquid Separations, Amberlite Weak Cation Exchangers, DOWEX Weak Cation Exchanger, and Diaion Weak Cation Exchangers from Sigma-Aldrich and Fractogel EMD COO- from EMD) ; a sulfonic acid based group (e. g., Hydrocell SP from Biochrom Labs Inc. , DOWEX Fine Mesh Strong Acid Cation Resin from Dow Liquid Separations, UNOsphere 5, WP Sulfonic from J. T. Baker, Sartobind S membrane from Sartorius, Amberlite Strong Cation Exchangers, DOWEX Strong Cation and Diaion Strong Cation Exchanger from Sigma-Aldrich); and a orthophosphate based group (for example, P11 from Whatman).

The term "detergent" refers to ionic, zwitterionic and nonionic surfactants, which are useful for preventing aggregation of proteins and to prevent non-specific interaction or binding of contaminants to the protein of interest, and can be present in various buffers used in the present invention, including sanitization, equilibration, loading, post-load wash(es), elution or strip buffers. In particular embodiments, a detergent is added to a wash buffer. Examples of detergents that can be used in the invention include, but are not limited to polysorbates (for example, polysorbates 20 or 80); poloxamers (for example poloxamer 188); Triton; sodium dodecyl sulfate (SDS); sodium laurel sulfate; sodium octyl glycoside; lauryl-, myristyl-, linoleyl-, or stearyl-sulfobetaine; lauryl-, myristyl- , linoleyl- or stearyl-sarcosine; linoleyl-, myristyl-, or cetyl-betaine; lauroamidopropyl-, cocamidopropyl-, linoleamidopropyl-, myristamidopropyl- , palmidopropyl-, or isostearamidopropyl-betaine (for example lauroamidopropyl); myristamidopropyl-, palmidopropyl-, or isostearamidopropyldimethylamine; sodium methyl cocoyl-, or disodium methyl oleyl-taurate; MONAQUAT series (Mona Industries, Inc., Paterson, N. J.); Igepal CA-630, Pluronic, Triton, BRIJ, Atlas G2127, Genapol, HECAMEG, LUBROL PX, MEGA, NP, THESIT, TOPPS, CHAPS, CHAPSO, DDMAU, EMPIGEN BB, AWITTERGENT and C12B8. The detergent can be added in any working buffer and can also be included in the feed containing the molecule of interest. Detergents can be present in any amount suitable for use in a protein purification process, for example, from about 0.001% to about 20%, and typically from about 0.01% to about 1%. In a particular embodiment, polysorbate 80 is used in a wash buffer for cation exchange chromatography.

The term "buffer" used in the present invention is a solution that resists changes in pH by the addition of acid or base by the action of its acid- base conjugates components. Various buffers can be employed in a method of the present invention depending on the desired pH of the buffer and the particular step in the purification process. Non-limiting examples of buffer components that can be used to control the pH range desirable for a method of the invention include acetate, citrate, histidine, phosphate, ammonium buffers such as ammonium acetate, succinate, 18-MES, CHAPS, MOPS, MOPSO, HEPES, Tris, and the like, as well as combinations of these TRIS-malic acid-NaOH, maleate, chioroacetate, formate, benzoate, propionate, pyridine, piperazine, ADA, PIPES, ACES, BES, TES, tricine, bicine, TAPS, ethanolamine, CHES, CAPS, methylamine, piperidine, o-boric acid, carbonic acid, lactic acid, butaneandioic acid, diethylmalonic acid, glycyiglycine, HEPPS, HEPPSO, imidazole, phenol, POPSO, succinate, TAPS, amine-based, benzylamine, trimethyl or dimethyl or ethyl or phenyl amine, ethylenediamine, or mopholine. Additional components (additives) can be present in a buffer as needed, for example, but not limited to, salts can be used to adjust buffer ionic strength. Non-limiting examples include, sodium chloride, sodium sulfate and potassium chloride; and other additives such as amino acids (such as glycine and histidine), chaotropes (such as urea), alcohols (such as ethanol, marinitol, glycerol, and benzyl alcohol), detergents, and sugars (such as sucrose, mannitol, maltose, trehalose, glucose, and fructose). The buffer components and additives, and the concentrations used, can vary according to the type of chromatography practiced in the invention. The pH and conductivity of the buffers can vary depending on which step in the purification process the buffer is used.

The term "equilibration buffer" refers to a solution used to adjust the pH and conductivity of the chromatography column prior to loading the column with the mixture containing the protein of interest for purification. Suitable buffers that can be used for this purpose are well known in the art, for example, but not limited to, the buffers described above or within, and includes any buffer at pH that is compatible with the selected resin used in the chromatography step for purifying the protein of interest. This buffer is used to load the mixture comprising the polypeptide of interest. The equilibration buffer has a conductivity and/or pH such that the polypeptide of interest is bound to the resin or such that the protein of interest flows through the column while one or more impurities bind to the column.

The term "loading buffer" refers to a solution used to load the mixture containing the protein of interest onto the column. Any appropriate solution can be used as the loading buffer. The conductivity and pH of the loading buffer in the present process is selected such that the protein of interest is bound to the resin while contaminants are able to flow through the column. Optionally, the loading buffer can be buffer exchanged. The loading buffer can also be prepared from a buffered mixture derived from a previous purification step, such as the elution buffer. Suitable buffers for use as a loading buffer with the selected resin are well known in the art, for example, but not limited to, those described above. It shall be appreciated by those having ordinary skill in the art that loading buffers for cation exchange chromatography, anion and HCIC can be used at comparable (if not the same) pH and conductivities.

The terms "wash buffer" or "post load wash", refer to a buffer used to elute one or more impurities from the ion exchange resin prior to eluting the protein of interest. The term "washing", and grammatical variations thereof, is used to describe the passing of an appropriate wash buffer through or over the chromatography resin. In certain embodiments the wash, equilibration, and loading buffers can be the same, but this is not required. The pH and conductivity of the buffer is such that one or more impurities are eluted from the resin while the resin retains the polypeptide of interest. If desirable, the wash buffer may contain a detergent, as described above, such as a polysorbate. Any suitable buffer at a pH compatible with the selected resin can be used for purifying the protein of interest, such as the buffers described above. Selection of pH and conductivity of the wash buffer are important for removal of host cell proteins (HCPs) and other contaminants without significantly eluting the protein of interest. The conductivity and pH can be reduced, or maintained or increased in wash buffers used in subsequent wash steps for the HCIC and cation exchange chromatography after loading the mixture in order to remove more hydrophilic and more acidic or basic contaminants than that of the protein of interest and to reduce the conductivity of the system prior to the elution step. In a particular embodiment, only the conductivity is decreased for the HCIC chromatography, and post-load washes for cation exchange chromatography do not include any change in either pH or conductivity of the buffers used for equilibration, load and post-load wash.

The term "elution buffer" refers to a buffer used to elute the protein of interest from the solid phase. The term "elute", and grammatical variations thereof, refers to the removal of a molecule, for example, but not limited to the polypeptide of interest, from a chromatography material by using appropriate conditions, for example, altering the ionic strength or pH of the buffer surrounding the chromatography material, by addition of a competitive molecule for the ligand, by altering the hydrophobicity of the molecule or by changing a chemical property of the ligand, such that the protein of interest is unable to bind the resin and is therefore eluted from the chromatography column. The term "eluate" refers to the effluent off the column containing the polypeptide of interest when the elution is applied onto the column. After elution of the polypeptide of interest the column can be regenerated, sanitized and stored as needed.

The term "residence time" used in the present invention is defined as the time from initiation of production through the end of purification. The "residence time" includes any hold steps after cell culture and prior to purification.

### 4. Detailed description of the invention

The inventors found that an anti-interferon alpha antibody lost binding activity over time during the production, purification, and storage of the antibody. Upon further investigation, it was determined that the anti-interferon alpha antibody exhibited multiple peaks by ion-exchange chromatography, which were determined to be a result of deamidation of the antibody. Further examination of the antibody sequence revealed that the Asn-Gly motif was present in the VHCDR2, therefore predisposing the antibody to deamidation. Not being bound by a particular hypothesis, it is believed that the presence of this potential deamidation site in a critical binding region of the antibody led to the loss of activity exhibited. Thus, to retain stability of the anti-interferon alpha antibody, the inventors have developed methods of producing, purifying and storing antibodies, as well as stable antibody compositions, with a decreased deamidation profile. Selected embodiments of the invention are described in the following sections.

Deamidation sites are referenced as the asparagine residue preceding (read N-terminus to C-terminus) to an amino acid residue such as glycine (Gly or G), serine (Ser or S), threonine (Thr or T), or aspartic acid (Asp or D).

In the embodiments to follow, it is understood that they collectively represent "methods of the invention".

Using the methods described in sections 4.1-4.6 below, the deamidation profile of an antibody predisposed to deamidation may be reduced as compared to a control antibody predisposed to deamidation not subjected to the methods described below.

In one embodiment of the invention, the deamidation profile of the antibody predisposed to deamidation is reduced by about 70%, about 60%, about 50%, about 40%, about 30%, about 20%, about 10% or about 5% to a control deamidation profile. In another embodiment of the invention, the deamidation profile of the antibody predisposed to deamidation is reduced by about 5% to about 70%, about 5% to about 60%, about 5% to about 50%, about 5% to about 40%, about 5% to about 30%, about 5% to about 20% or about 5% to about 10% to a control deamidation profile. In another embodiment of the invention, the deamidation profile of the antibody predisposed to deamidation is reduced by about 10% to about 70%, about 10% to about 60%, about 10% to about 50%, about 10% to about 40%, about 10% to about 30%, or about 10% to about 20% to a control deamidation profile. In another embodiment of the invention the deamidation profile of the antibody predisposed to deamidation is reduced by about 20% to about 70%, about 20% to about 60%, about 20% to about 50%, about 20% to about 40%, or about 20% to about 30% to a control deamidation profile. In another embodiment of the invention the deamidation profile of the antibody predisposed to deamidation is reduced by about 30% to about 70%, about 30% to about 60%, about 30% to about 50%, or about 30% to about 40% to a control deamidation profile.

In one embodiment of the invention, the deamidation profile of the antibody predisposed to deamidation is reduced by 70%, 60%, 50%, 40%, 30%, 20%, 10% or 5% to a control deamidation profile. In another embodiment of the invention, the deamidation profile of the antibody predisposed to deamidation is reduced by 5% to 70%, 5% to 60%, 5% to 50%, 5% to 40%, 5% to 30%, 5% to 20% or 5% to 10% to a control deamidation profile. In another embodiment of the invention, the deamidation profile of the antibody predisposed to deamidation is reduced by 10% to 70%, 10% to 60%, 10% to 50%, 10% to 40%, 10% to 30%, or 10% to 20% to a control deamidation profile. In another embodiment of the invention the deamidation profile of the antibody predisposed to deamidation is reduced by 20% to 70%, 20% to 60%, 20% to 50%, 20% to 40%, or 20% to 30% to a control deamidation profile. In another embodiment of the invention the deamidation profile of the antibody predisposed to deamidation is reduced by 30% to 70%, 30% to 60%, 30% to 50%, or 30% to 40% to a control deamidation profile.

In one embodiment of the invention, the deamidation profile of the antibody predisposed to deamidation is reduced by at least 70%, at least 60%, at least 50%, at least 40%, at least 30%, at least 20%, at least 10% or at least5 % to a control deamidation profile. In another embodiment of the invention, the deamidation profile of the antibody predisposed to deamidation is reduced by at least 5% to at least 70%, at least 5% to at least 60%, at least 5% to at least 50%, at least 5% to at least 40%, at least 5% to at least 30%, at least 5% to at least 20% or at least 5% to at least 10% to a control deamidation profile. In another embodiment of the invention, the deamidation profile of the antibody predisposed to deamidation is reduced by at least 10% to at least 70%, at least 10% to at least 60%, at least 10% to at least 50%, at least 10% to at least 40%, at least 10% to at least 30%, or at least 10% to at least 20% to a control deamidation profile. In another embodiment of the invention the deamidation profile of the antibody predisposed to deamidation is reduced by at least 20% to at least 70%, at least 20% to at least 60%, at least 20% to at least 50%, at least 20% to at least 40%, or at least 20% to at least 30% to a control deamidation profile. In another embodiment of the invention the deamidation profile of the antibody predisposed to deamidation is reduced by at least 30% to at least 70%, at least 30% to at least 60%, at least 30% to at least 50%, or at least 30% to at least 40% to a control deamidation profile.

The level of deamidation may also be represented as a percentage of the total concentration of an antibody. In certain embodiments, antibodies predisposed to deamidation subjected to the methods described in sections 4.1-4.6 exhibit a reduced deamidation profile as measured by a percentage of the total concentration of antibody present. In one embodiment, methods of the invention produce antibodies predisposed to deamidation which exhibit deamidation profiled of about 1%, about 5%, about 10%, about 15%, about 20%, about 25%, about 30%, or about 35%, of the total amount of antibody present in the sample. In certain embodiments, methods of the invention produce antibodies predisposed to deamidation which exhibit deamidation profiles of less than 35%, less than 30%, less than 25%, less than 20%, less than 15%, less than 10%, less than 5%, or less than 1% of the total amount of antibody present in the sample.

### 4.1 Cell culture production of antibodies

### 4.1.1 Recombinant expression of an antibody

Recombinant expression of an antibody of the invention, derivative, analog or fragment thereof, (for example, a heavy or light chain of an antibody of the invention or a portion thereof or a single chain antibody of the invention), requires construction of an expression vector containing a polynucleotide that encodes the antibody. Once a polynucleotide encoding an antibody molecule or a heavy or light chain of an antibody, or portion thereof has been obtained, the vector for the production of the antibody molecule may be produced by recombinant DNA technology using techniques well known in the art (also see section 4.7.4 below).

Thus, methods for preparing a protein by expressing a polynucleotide containing an antibody encoding nucleotide sequence are described herein. Methods which are well known to those skilled in the art can be used to construct expression vectors containing antibody coding sequences and appropriate transcriptional and translational control signals. These methods include, for example, *in vitro* recombinant DNA techniques, synthetic techniques, and *in vivo* genetic recombination.

The invention also provides replicable vectors comprising a nucleotide sequence encoding an antibody molecule of the invention, a heavy or light chain of an antibody, a heavy or light chain variable domain of an antibody or a portion thereof, or a heavy or light chain CDR, operably linked to a promoter. Such vectors may include the nucleotide sequence encoding the constant region of the antibody molecule (see, for example, International Publication No. WO 86/05807; International Publication No. WO 89/01036; and U.S. Pat. No. 5,122,464) and the variable domain of the antibody maybe cloned into such a vector for expression of the entire heavy, the entire light chain, or both the entire heavy and light chains.

The expression vector is transferred to a host cell by conventional techniques and the transfected cells are then cultured by conventional techniques to produce an antibody of the invention. Thus, the invention includes host cells containing a polynucleotide encoding an antibody of the invention or fragments thereof, or a heavy or light chain thereof, or portion thereof, or a single chain antibody of the invention, operably linked to a heterologous promoter. In other embodiments for the expression of double-chained antibodies, vectors encoding both the heavy and light chains may be co-expressed in the host cell for expression of the entire immunoglobulin molecule, as detailed below.

A variety of host-expression vector systems may be utilized to express the antibody molecules of the invention (see, for example, U.S. Pat. No. 5,807,715). Such host-expression systems represent vehicles by which the coding sequences of interest may be produced and subsequently purified, but also represent cells which may, when transformed or transfected with the appropriate nucleotide coding sequences, express an antibody molecule of the invention *in situ*. These include, but are not limited to, microorganisms such as bacteria (for example, *E. coli* and *B. subtilis*) transformed with recombinant bacteriophage DNA, plasmid DNA or cosmid DNA expression vectors containing antibody coding sequences; yeast (for example, *Saccharomyces Pichia*) transformed with recombinant yeast expression vectors containing antibody coding sequences; insect cell systems infected with recombinant virus expression vectors (for example, baculovirus) containing antibody coding sequences; plant cell systems infected with recombinant virus expression vectors (for example, cauliflower mosaic virus, CaMV; tobacco mosaic virus, TMV) or transformed with recombinant plasmid expression vectors (for example Ti plasmid) containing antibody coding sequences; or mammalian cell systems (for example, COS, HEK, 293, MDCK, CHO, BHK, NS0, and 3T3 cells) harboring recombinant expression constructs containing promoters derived from the genome of mammalian cells (for example, metallothionein promoter) or from mammalian viruses (for example, the adenovirus late promoter; the vaccinia virus 7.5K promoter). For example bacterial cells such as *Escherichia coli*, and eukaryotic cells, especially for the expression of whole recombinant antibody molecule, are used for the expression of a recombinant antibody molecule. For example, mammalian cells such as Chinese hamster ovary cells (CHO), in conjunction with a vector such as the major intermediate early gene promoter element from human cytomegalovirus is an effective expression system for antibodies (Foecking et al., 1986, Gene 45:101; and Cockett et al., 1990, Bio/Technology 8:2). In a specific embodiment, the expression of nucleotide sequences encoding antibodies or fragments thereof which specifically bind to IFN alpha are regulated by a constitutive promoter, inducible promoter or tissue specific promoter.

In bacterial systems, a number of expression vectors may be advantageously selected depending upon the use intended for the antibody molecule being expressed. For example, when a large quantity of such a protein is to be produced, for the generation of pharmaceutical compositions of an antibody molecule, vectors which direct the expression of high levels of fusion protein products that are readily purified may be desirable. Such vectors include, but are not limited to, the *E. coli* expression vector pUR278 (Ruther et al., 1983, EMBO 12:1791), in which the antibody coding sequence may be ligated individually into the vector in frame with the lac Z coding region so that a fusion protein is produced; pIN vectors (Inouye & Inouye, 1985, Nucleic Acids Res. 13:3101-3109; Van Heeke & Schuster, 1989, J. Biol. Chem. 24:5503-5509); and the like. pGEX vectors may also be used to express foreign polypeptides as fusion proteins with glutathione 5-transferase (GST). In general, such fusion proteins are soluble and can easily be purified from lysed cells by adsorption and binding to matrix glutathione agarose beads followed by elution in the presence of free glutathione. The pGEX vectors are designed to include thrombin or factor Xa protease cleavage sites so that the cloned target gene product can be released from the GST moiety.

In an insect system, Autographa californica nuclear polyhedrosis virus (AcNPV) is used as a vector to express foreign genes. The virus grows in *Spodoptera frugiperda* cells. The antibody coding sequence may be cloned individually into non-essential regions (for example the polyhedrin gene) of the virus and placed under control of an AcNPV promoter (for example the polyhedrin promoter).

In mammalian host cells, a number of viral-based expression systems may be utilized. In cases where an adenovirus is used as an expression vector, the antibody coding sequence of interest may be ligated to an adenovirus transcription/translation control complex, for example, the late promoter and tripartite leader sequence. This chimeric gene may then be inserted in the adenovirus genome by *in vitro* or *in vivo* recombination. Insertion in a non-essential region of the viral genome (for example, region El or E3) will result in a recombinant virus that is viable and capable of expressing the antibody molecule in infected hosts (for example, see Logan & Shenk, 1984, Proc. Natl. Acad. Sci. USA 8 1:355-359). Specific initiation signals may also be required for efficient translation of inserted antibody coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (see, for example, Bittner et al., 1987, Methods in Enzymol. 153:516-544).

In addition, a host cell strain may be chosen which modulates the expression of the inserted sequences, or modifies and processes the gene product in the specific fashion desired. Such modifications (for example, glycosylation) and processing (for example, cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include but are not limited to CHO, MDCK, VERY, BHK, Hela, COS, 293, 3T3, W138, BT483, Hs578T, HTB2, BT2O and T47D, NS0, CRL7O3O and HsS78Bst cells.

For long-term, high-yield production of recombinant proteins, stable expression is preferred. For example, cell lines which stably express the antibody molecule may be engineered. Rather than using expression vectors which contain viral origins of replication, host cells can be transformed with DNA controlled by appropriate expression control elements (for example, promoter, enhancer, sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the antibody molecule. Such engineered cell lines may be particularly useful in screening and evaluation of compositions that interact directly or indirectly with the antibody molecule.

A number of selection systems may be used, including but not limited to, the herpes simplex virus thymidine kinase (Wigler et al., 1977, Cell 11:223), hypoxanthineguanine phosphoribosyltransferase (Szybalska & Szybalski, 1992, Proc.Natl. Acad. Sci. USA 48:202), and adenine phosphoribosyltransferase (Lowy et al., 1980, Cell 22:8-17) genes can be employed in *tk*-, *hgprt*- or *aprt*- cells, respectively. Also, anti-metabolite resistance can be used as the basis of selection for the following genes: *dhfr,* which confers resistance to methotrexate (Wigler et al., 1980, Natl. Acad. Sci. USA 77:357; O'Hare et al., 1981, Proc. Natl. Acad. Sci. USA 78:1527); *gpt*, which confers resistance to mycophenolic acid (Mulligan & Berg, 1981, Proc. Natl. Acad. Sci. USA 78:2072); *neo,* which confers resistance to the aminoglycoside G-418 (Wu and Wu, 1991, Biotherapy 3:87-95; Tolstoshev, 1993, Ann. Rev. Pharmacol. Toxicol. 32:573-596; Mulligan, 1993, Science 260:926-932; and Morgan and Anderson, 1993, Ann. Rev. Biochem. 62: 191-217; May, 1993, TIB TECH 11(5):155-2 15); and *hygro*, which confers resistance to hygromycin (Santerre et al., 1984, Gene 30:147). Methods commonly known in the art of recombinant DNA technology may be routinely applied to select the desired recombinant clone, and such methods are described, for example, in Ausubel et al. (eds.), Current Protocols in Molecular Biology, John Wiley & Sons, NY (1993); Kriegler, Gene Transfer and Expression, A Laboratory Manual, Stockton Press, NY (1990); and in Chapters 12 and 13, Dracopoli et al. (eds), Current Protocols in Human Genetics, John Wiley & Sons, NY (1994); Colberre-Garapin et al., 1981, J. Mol. Biol. 150: 1, which are incorporated by reference herein in their entireties.

The expression levels of an antibody molecule can be increased by vector amplification (for a review, see Bebbington and Hentschel, The use of vectors based on gene amplification for the expression of cloned genes in mammalian cells in DNA cloning, Vol.3. (Academic Press, New York, 1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., 1983, Mol. Cell. Biol. 3:257).

The host cell may be co-transfected with two expression vectors of the invention, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes, and is capable of expressing, both heavy and light chain polypeptides. In such situations, the light chain should be placed before the heavy chain to avoid an excess of toxic free heavy chain (Proudfoot, 1986, Nature 322:52; and Kohler, 1980, Proc. Natl. Acad. Sci. USA 77:2 197). The coding sequences for the heavy and light chains may comprise cDNA or genomic DNA.

Once an antibody molecule of the invention has been produced by recombinant expression, it may be purified by any method known in the art for purification of an immunoglobulin molecule, for example, by chromatography (for example, ion exchange, affinity, particularly by affinity for the specific antigen after Protein A, and sizing column chromatography), centrifugation, differential solubility, or by any other standard technique for the purification of proteins. Further, the antibodies of the present invention or fragments thereof may be fused to heterologous polypeptide sequences described herein or otherwise known in the art to facilitate purification.

### 4.1.2 Cell culture processes

A number of cell culture processes may be used in the scale up synthesis of the desired product. Cells are generally started in small tissue culture flasks with a capacity of less than 500 mls. The cell culture, once reaching a desired concentration could be transferred to a larger flask for expansion. Once the cells have expanded in the larger shaking flasks, the culture is often aseptically transferred to a bioreactor for production of the desired product. These bioreactors can range in size from 5 litres to 5000 litres. Often a first bioreactor termed the 'seed' bioreactor is operated to capacity and then the culture is transferred to a 'production' bioreactor to obtain product. During the bioreactor runs, many physiological parameters such as pH, temperature, and dissolved oxygen are continuously monitored and adjusted as needed. Throughout the process, samples are routinely collected and tested for pH, viable cell density (VCD) and percent cell viability. In addition, microscopic evaluation for microbial contaminants are performed.

In one embodiment, methods of the invention comprise cells grown in media with a pH value range of about 6.0 to about 7.5. In another embodiment of the invention, cells are grown in media with a pH value range of about 7.0 to about 7.5. In other embodiments of the invention, cells are grown in media with a pH value range of about 6.0 to about 7.0, about 6.1 to about 7.0, about 6.2 to about 7.0, about 6.3 to about 7.0, about 6.4 to about 7.0, about 6.5 to about 7.0, about 6.6 to about 7.0, about 6.7 to about 7.0, about 6.8 to about 7.0, or about 6.9 to about 7.0. In another embodiment of the invention, cells are grown in media with a pH value range of about 6.0 to about 7.2, about 6.0 to about 7.0, about 6.0 to about 6.9, about 6.0 to about 6.8, about 6.0 to about 6.7, about 6.0 to about 6.6, about 6.0 to about 6.5, about 6.0 to about 6.4, about 6.0 to about 6.3, or about 6.0 to about 6.2. In another embodiment of the invention, the cells are grown in media having a pH value range of about 6.0 to about 6.1, about 6.1 to about 6.2, about 6.2 to about 6.3, about 6.3 to about 6.4, about 6.4 to about 6.5, about 6.5 to about 6.6, about 6.6 to about 6.7, about 6.7 to about 6.8, about 6.8 to about 6.9, about 6.9 to about 7.0, about 7.0 to about 7.1, about 7.1 to about 7.2, about 7.2 to about 7.3, about 7.3 to about 7.4 or about 7.4 to about 7.5. In another embodiment of the invention, cells are grown in media having a pH value of about 6.0, about 6.1, about 6.2, about 6.3, about 6.4, about 6.5, about 6.6, about 6.7, about 6.8, about 6.9, about 7.0, about 7.1, about 7.2, about 7.3, about 7.4, or about 7.5.

In one embodiment, methods of the invention comprise cells grown in media with a pH value range of 6.0 to 7.5. In another embodiment of the invention, cells are grown in media with a pH value range of 7.0 to 7.5. In other embodiments of the invention, cells are grown in media with a pH value range of 6.0 to 7.0, 6.1 to 7.0, 6.2 to 7.0, 6.3 to 7.0, 6.4 to 7.0, 6.5 to 7.0, 6.6 to 7.0, 6.7 to 7.0, 6.8 to 7.0, or 6.9 to 7.0. In another embodiment of the invention, cells are grown in media with a pH value range of 6.0 to 7.2, 6.0 to 7.0, 6.0 to 6.9, 6.0 to 6.8, 6.0 to 6.7, 6.0 to 6.6, 6.0 to 6.5, 6.0 to 6.4, 6.0 to 6.3, or 6.0 to 6.2. In another embodiment of the invention, the cells are grown in media having a pH value range of 6.0 to 6.1, 6.1 to 6.2, 6.2 to 6.3, 6.3 to 6.4, 6.4 to 6.5, 6.5 to 6.6, 6.6 to 6.7, 6.7 to 6.8, 6.8 to 6.9, 6.9 to 7.0, 7.0 to 7.1, 7.1 to 7.2, 7.2 to 7.3, 7.3 to 7.4 or 7.4 to 7.5. In another embodiment of the invention, cells are grown in media having a pH value of 6.0, 6.1, 6.2, 6.3, 6.4, 6.5, 6.6, 6.7, 6.8, 6.9, 7.0, 7.1, 7.2, 7.3, 7.4, or 7.5.

In one embodiment, methods of the invention comprise cells grown at a temperature range of about 28°C to about 37°C. In another embodiment of the invention, cells are grown at a temperature range of about 28°C to about 32°C, about 32°C to about 34°C, or about 34°C to about 37°C. In another embodiment of the invention, cells are grown at a temperature range of about 28°C to about 36°C, about 28°C to about 35°C about 28°C to about 34°C, about 28°C to about 33°C, about 28°C to about 31°C, or about 28°C to about 30°C. In another embodiment of the invention, cells are grown at a temperature range of about 29°C to about 37°C, about 30°C to about 37°C, about 31°C to about 37°C, about 32°C to about 37°C, about 33°C to about 37°C, about 34°C to about 37°C, about 35°C to about 37°C, or about 36°C to about 37°C. In another embodiment of the invention, cells are grown at a temperature range of about 28°C to about 29°C, about 29°C to about 30°C, about 30°C to about 31°C, about 31°C to about 32°C, about 32°C to about 33°C, about 33°C to about 34°C, about 34°C to about 35°C, about 35°C to about 36°C, or about 36°C to about 37°C. In another embodiment of the invention, cells are grown at a temperature of about 28°C, about 29°C, about 30°C, about 31°C, about 32°C, about 33°C, about 34°C, about 35°C, about 36°C, or about 37°C. In another embodiment of the invention, cells are grown at a temperature range of 0.1 degree increments between about 28°C and about 37°C. In another embodiment of the invention, cells are grown at a temperature range of 0.1 degree increments between about 28°C and about 33°C. In another embodiment of the invention, cells are grown at a temperature range of 0.1 degree increments between about 28°C and about 34°C. In another embodiment of the invention, cells are grown at a temperature range of 0.1 degree increments between about 30°C and about 33°C. In another embodiment of the invention, cells are grown at a temperature range of 0.1 degree increments between about 30°C and about 34°C. In another embodiment of the invention, cells are grown at a temperature range of 0.1 degree increments between about 33°C and about 36°C. In another embodiment of the invention, cells are grown at a temperature range of 0.1 degree increments between about 28°C and about 29°C. In another embodiment of the invention, cells are grown at a temperature range of 0.1 degree increments between about 28°C and about 29°C. In another embodiment of the invention, cells are grown at a temperature range of 0.1 degree increments between about 29°C and about 30°C. In another embodiment of the invention, cells are grown at a temperature range of 0.1 degree increments between about 30°C and about 31°C. In another embodiment of the invention, cells are grown at a temperature range of 0.1 degree increments between about 31°C and about 32°C. In another embodiment of the invention, cells are grown at a temperature range of 0.1 degree increments between about 32°C and about 33°C. In another embodiment of the invention, cells are grown at a temperature range of 0.1 degree increments between about 33°C and about 34°C. In another embodiment of the invention, cells are grown at a temperature range of 0.1 degree increments between about 34°C and about 35°C. In another embodiment of the invention, cells are grown at a temperature range of 0.1 degree increments between about 35°C and about 36°C. In another embodiment of the invention, cells are grown at a temperature range of 0.1 degree increments between about 36°C and about 37°C.

In one embodiment, methods of the invention comprise cells grown at a temperature range of 28°C to 37°C. In another embodiment of the invention, cells are grown at a temperature range of 28°C to 32°C, 32°C to 34°C, or 34°C to 37°C. In another embodiment of the invention, cells are grown at a temperature range of 28°C to 36°C, 28°C to 35°C, 28°C to 34°C, 28°C to 33°C, 28°C to 31°C, or 28°C to 30°C. In another embodiment of the invention, cells are grown at a temperature range of 29°C to 37°C, 30°C to 37°C, 31°C to 37°C, 32°C to 37°C, 33°C to 37°C, 34°C to 37°C, 35°C to 37°C, or 36°C to 37°C. In another embodiment of the invention, cells are grown at a temperature range of 28°C to 29°C, 29°C to 30°C, 30°C to 31°C, 31°C to 32°C, 32°C to 33°C, 33°C to 34°C, 34°C to 35°C, 35°C to 36°C, or 36°C to 37°C. In another embodiment of the invention, cells are grown at a temperature of 28°C, 29°C, 30°C, 31°C, 32°C, 33°C, 34°C, 35°C, 36°C, or 37°C. In another embodiment of the invention, cells are grown at a temperature range of 0.1 degree increments between 28°C and 37°C. In another embodiment of the invention, cells are grown at a temperature range of 0.1 degree increments between 28°C and 33°C. In another embodiment of the invention, cells are grown at a temperature range of 0.1 degree increments between 28°C and 34°C. In another embodiment of the invention, cells are grown at a temperature range of 0.1 degree increments between 30°C and 33°C. In another embodiment of the invention, cells are grown at a temperature range of 0.1 degree increments between 30°C and 34°C. In another embodiment of the invention, cells are grown at a temperature range of 0.1 degree increments between 33°C and 36°C. In another embodiment of the invention, cells are grown at a temperature range of 0.1 degree increments between 28°C and 29°C. In another embodiment of the invention, cells are grown at a temperature range of 0.1 degree increments between 28°C and 29°C. In another embodiment of the invention, cells are grown at a temperature range of 0.1 degree increments between 29°C and 30°C. In another embodiment of the invention, cells are grown at a temperature range of 0.1 degree increments between 30°C and 31°C. In another embodiment of the invention, cells are grown at a temperature range of 0.1 degree increments between 31°C and 32°C. In another embodiment of the invention, cells are grown at a temperature range of 0.1 degree increments between 32°C and 33°C. In another embodiment of the invention, cells are grown at a temperature range of 0.1 degree increments between 33°C and 34°C. In another embodiment of the invention, cells are grown at a temperature range of 0.1 degree increments between 34°C and 35°C. In another embodiment of the invention, cells are grown at a temperature range of 0.1 degree increments between 35°C and 36°C. In another embodiment of the invention, cells are grown at a temperature range of 0.1 degree increments between 36°C and 37°C.

In one embodiment, methods of the invention comprise cells cultured for a total run period of greater than about 8 days, greater than about 9 days, greater than about 10 days, greater than about 11 days, greater than about 12 days, greater than about 13 days, greater than about 14 days, greater than about 15 days, greater than about 16 days, or greater than about 17 days. In another embodiment, cells can be cultured for a total run period of about 9 to about 17 days, about 9 to about 14 days, or about 14 to about 17 days, or more. In another embodiment, cells can be cultured for about 9 to about 11, about 11 to about 3, about 13 to about 15, about 15 to about 17 days or more. In another embodiment of the invention, cells can be cultured for a total run period of about 9, about 10, about 11, about 12, about 13, about 14, about 15, about 16, about 17 days or more. In another embodiment, cells may be cultured for a total run period of 9, 10, 11, 12, 13, 14, 15, 16, or 17 days.

In certain embodiments of the invention, the parameters of temperature of the cell culture and pH of the media are lowered concurrently as described above. In another embodiment, the temperature of the cell culture, pH of the media and harvest timing are lowered concurrently as described above. In another embodiment of the invention, the cells are grown at a temperature comprising a range from about 28°C to about 37°C in 0.1 degree increments and comprising a pH range from about 6.0 to about 7.2 in 0.1 increments. In another embodiment of the invention, the cells are grown at a temperature comprising a range from about 28°C to about 37°C in 0.1 degree increments and comprising a pH range from about 6.0 to about 7.2 in 0.1 increments and harvested on a day after inoculation from the range comprising from about 0 to about 17 days in single day increments.

In other embodiments of the invention, the cells are grown at a temperature comprising a range from 28°C to 37°C in 0.1 degree increments and comprising a pH range from 6.0 to 7.2 in 0.1 increments. In another embodiment of the invention, the cells are grown at a temperature comprising a range from 28°C to 37°C in 0.1 degree increments and comprising a pH range from 6.0 to 7.2 in 0.1 increments and harvested on a day after inoculation from the range comprising from 0 to 17 days in single day increments.

In another embodiment of the invention, cells are grown at a temperature of about 33°C to about 35°C in media with a pH of about 6.7 to about 7.1 pH units for about 17 days. In another embodiment of the invention, cells are grown at a temperature of about 36°C to about 38°C in media with a pH of about 6.8 to about 7.5 pH units. In a specific embodiment of the invention, cells are grown at a temperature of about 34°C in media at a pH value of about 6.9 pH units for about 17 days. In another specific embodiment of the invention, cells are grown at a temperature of 34°C in media at a pH value of 6.9 pH units for 17 days.

### 4.2 Temperature shift - Biphasic culture conditions

In accordance with the cell culturing methods and processes of this invention, cells cultured in conjunction with one or more temperature shifts during a culturing run can produce a high quantity and quality of product, as measured by the end titer. The high quantity and quality of protein production associated with the methods of this invention are obtained relative to methods in which no temperature shift, or at most, one temperature shift is used, regardless of whether a culture run is carried out for a total run time of about 8 to about 17 days. Moreover, as a result of the one or more temperature shifts during the culturing process, cells can be maintained in culture for a period of time that essentially extends the standard or initial production phase. A standard or initial production phase is typically about 6 to 17 days. Increased production of high quality protein, as well as sustained cell viability, are achieved during the extended production phase of the present culturing methods involving two or more temperature shifts.

In an embodiment of the invention, cells may be cultured for a temperature shift period of greater than about 8 days, greater than about 9 days, greater than about 10 days, greater than about 11 days, greater than about 12 days, greater than about 13 days, greater than about 14 days, greater than about 15 days, greater than 16 days, or greater than about 17 days. In another embodiment, cells may be cultured for a temperature shift period of about 9 to about 17 days, about 9 to about 14 days, or about 14 to about 17 days, or more. In another embodiment, cells may be cultured for a temperature shift period of about 9 to about 11, about 11 to about 3, about 13 to about 15, about 15 to about 17 days or more. In another embodiment of the invention, cells may be cultured for a temperature shift period of about 9, about 10, about 11, about 12, about 13, about 14, about 15, about 16, about 17 days or more.

In other embodiments, cells may be cultured for a temperature shift period of 9 to 17 days, 9 to 14 days, or 14 to 17 days, or more. In another embodiment, cells may be cultured for a temperature shift period of 9 to 11, 11 to 3, 13 to 15, 15 to 17 days or more. In another embodiment of the invention, cells may be cultured for a temperature shift period of 9, 10, 11, 12, 13, 14, 15, 16, 17 days or more.

The timing of the shift of cell culture temperature may be assessed as a function of cell count within the culture vessel. In some embodiments the temperature shift occurs after the cell count has reached about 1 x 10⁵ cells/ml to about 1 x 10⁶ cells/ml, about 1 x 10⁵ cells/ml to about 5 x 10⁵ cells/ml, about 5 x 10⁵ cells/ml to about 1 x 10⁶ cells/ml, or about 4 x 10⁵ cells/ml to about 8 x 10⁵ cells/ml. In other embodiments, temperature shift occurs after the cell count has reached about 1, about 2, about 3, about 4, about 5, about 6, about 7, about 8, about 9, about or about 10 x 10⁵ cells/ml. In a specific embodiment, the temperature shift occurs when the cell density reaches 1 x 10⁶ cells/ml.

In some embodiments the temperature shift occurs after the cell count has reached 1 x 10⁵ cells/ml to 1 x 10⁶ cells/ml, 1 x 10⁵ cells/ml to 5 x 10⁵ cells/ml, 5 x 10⁵ cells/ml to 1 x 10⁶ cells/ml, or 4 x 10⁵ cells/ml to 8 x 10⁵ cells/ml. In other embodiments, temperature shift occurs after the cell count has reached 1, 2, 3, 4, 5, 6, 7, 8, 9, or 10 x 10⁵ cells/ml.

In one embodiment, one or more temperature shifts may occur from about 28°C, about 29°C, about 30°C, about 31°C, about 32°C, about 33°C, about 34°C, about 35°C, about 36°C, or about 37°C. In another embodiment of the invention, one of more temperature shifts may occur to about 28°C, about 29°C, about 30°C, about 31°C, about 32°C, about 33°C, about 34°C, about 35°C, about 36°C, or about 37°C. In another embodiment of the invention, one or more temperature shifts may occur to or from a temperature range of 0.1 degree increments between about 28°C to about 37°C. In another embodiment of the invention, one or more temperature shifts may occur to or from a temperature range of 0.1 degree increments between about 28°C to about 33°C. In another embodiment of the invention, one or more temperature shifts may occur to or from a temperature range of 0.1 degree increments between about 32°C to about 34°C. In another embodiment of the invention, one or more temperature shifts may occur to or from a temperature range of 0.1 degree increments between about 34°C to about 36°C. In a specific embodiment, the cell culture temperature is shifted from about 34°C to about 32°C.

In one embodiment, one or more temperature shifts may occur from 28°C, 29°C, 30°C, 31°C, 32°C, 33°C, 34°C, 35°C, 36°C, or 37°C. In another embodiment of the invention, one of more temperature shifts may occur to 28°C, 29°C, 30°C, 31°C, 32°C, 33°C, 34°C, 35°C, 36°C, or 37°C. In another embodiment of the invention, one or more temperature shifts may occur to or from a temperature range of 0.1 degree increments between 28°C to 37°C. In another embodiment of the invention, one or more temperature shifts may occur to or from a temperature range of 0.1 degree increments between 28°C to 33°C. In another embodiment of the invention, one or more temperature shifts may occur to or from a temperature range of 0.1 degree increments between 32°C to 34°C. In another embodiment of the invention, one or more temperature shifts may occur to or from a temperature range of 0.1 degree increments between 34°C to 36°C. In a specific embodiment, the cell culture temperature is shifted from 34°C to 32°C.

### 4.3 Higher seeding density

In an effort to optimize cell growth conditions for cell viability, protein production, culture time and other factors, the seeding cell density can be adjusted. In an embodiment of the invention, cells are seeded at a density from about 1 x 10⁵ cells/ml to about 1 x 10⁶ cells/ml. In another embodiment of the invention, cells are seeded at a density of about 1 x 10⁵ cells/ml to about 5 x 10⁵ cells/ml. In another embodiment of the invention, cells are seeded at a density of about 5 x 10⁵ cells/ml to about 1 x 10⁶ cells/ml. In another embodiment of the invention, cells are seeded at a density from about 4 x 10⁵ cells/ml to about 8 x 10⁵ cells/ml. In another embodiment of the invention, cells are seeded at a density of about 1, about 2, about 3, about 4, about 5, about 6, about 7, about 8, about 9, about or about 10 x 10⁵ cells/ml. In another embodiment of the invention, cells are seeded at a density between about 1 to about 2, about 2 to about 3, about 3 to about 4, about 4 to about 5, about 5 to about 6, about 6 to about 7, about 7 to about 8, about 8 to about 9, about 9 to about 10 x 10⁵ cells/ml.

In other embodiments, cells are seeded at a density from 1 x 10⁵ cells/ml to 1 x 10⁶ cells/ml. In another embodiment of the invention, cells are seeded at a density of 1 x 10⁵ cells/ml to 5 x 10⁵ cells/ml. In another embodiment of the invention, cells are seeded at a density of 5 x 10⁵ cells/ml to 1 x 10⁶ cells/ml. In another embodiment of the invention, cells are seeded at a density from 4 x 10⁵ cells/ml to 8 x 10⁵ cells/ml. In another embodiment of the invention, cells are seeded at a density of 1, 2, 3, 4, 5, 6, 7, 8, 9, or 10 x 10⁵ cells/ml. In another embodiment of the invention, cells are seeded at a density between 1 to 2, 2 to 3, 3 to 4, 4 to 5, 5 to 6, 6 to 7, 7 to 8, 8 to 9, 9 to 10 x 10⁵ cells/ml.

### 4.4 Harvest pH change

In an effort to further minimize deamidation of the desired protein product, the pH of the harvested material may be adjusted at the time of harvest. The pH of the harvested material may be adjusted up or down with the addition of base or acid respectfully. In an embodiment of the invention, the pH of the harvested material is adjusted downwards. In another embodiment of the invention the pH of the harvested material is adjusted upwards. In another embodiment of the invention the pH of the harvested material is adjusted to value from about 6.0 to about 7.5. In one embodiment of the invention, the pH of the harvested material is adjusted to a value of about 7.0 to about 7.5. In one embodiment of the invention, the pH of the harvested material is adjusted to a value of about 6.0 to about 7.0, about 6.1 to about 7.0, about 6.2 to about 7.0, about 6.3 to about 7.0, about 6.4 to about 7.0, about 6.5 to about 7.0, about 6.6 to about 7.0, about 6.7 to about 7.0, about 6.8 to about 7.0, or about 6.9 to about 7.0. In another embodiment of the invention, the pH of the harvested material is adjusted to a value of about 6.0 to about 7.2, about 6.0 to about 7.0, about 6.0 to about 6.9, about 6.0 to about 6.8, about 6.0 to about 6.7, about 6.0 to about 6.6, about 6.0 to about 6.5, about 6.0 to about 6.4, about 6.0 to about 6.3, or about 6.0 to about 6.2. In another embodiment of the invention, the pH of the harvested material is adjusted to a value of about 6.0 to about 6.1, about 6.1 to about 6.2, about 6.2 to about 6.3, about 6.3 to about 6.4, about 6.4 to about 6.5, about 6.5 to about 6.6, about 6.6 to about 6.7, about 6.7 to about 6.8, about 6.8 to about 6.9, about 6.9 to about 7.0, about 7.0 to about 7.1, about 7.1 to about 7.2, about 7.2 to about 7.3, about 7.3 to about 7.4 or about 7.4 to about 7.5. In another embodiment of the invention, the pH of the harvested material is adjusted to a value of about 6.0, about 6.1, about 6.2, about 6.3, about 6.4, about 6.5, about 6.6, about 6.7, about 6.8, about 6.9, about 7.0, about 7.1, about 7.2, about 7.3, about 7.4, or about 7.5.

In another embodiment of the invention the pH of the harvested material is adjusted to value from 6.0 to 7.5. In one embodiment of the invention, the pH of the harvested material is adjusted to a value of 7.0 to 7.5. In one embodiment of the invention, the pH of the harvested material is adjusted to a value of 6.0 to 7.0, 6.1 to 7.0, 6.2 to 7.0, 6.3 to 7.0, 6.4 to 7.0, 6.5 to 7.0, 6.6 to 7.0, 6.7 to 7.0, 6.8 to 7.0, or 6.9 to 7.0. In another embodiment of the invention, the pH of the harvested material is adjusted to a value of 6.0 to 7.2, 6.0 to 7.0, 6.0 to 6.9, 6.0 to 6.8, 6.0 to 6.7, 6.0 to 6.6, 6.0 to 6.5, 6.0 to 6.4, 6.0 to 6.3, or 6.0 to 6.2. In another embodiment of the invention, the pH of the harvested material is adjusted to a value of 6.0 to 6.1, 6.1 to 6.2, 6.2 to 6.3, 6.3 to 6.4, 6.4 to 6.5, 6.5 to 6.6, 6.6 to 6.7, 6.7 to 6.8, 6.8 to 6.9, 6.9 to 7.0, 7.0 to 7.1, 7.1 to 7.2, 7.2 to 7.3, 7.3 to 7.4 or 7.4 to 7.5. In another embodiment of the invention, the pH of the harvested material is adjusted to a value of 6.0, 6.1, 6.2, 6.3, 6.4, 6.5, 6.6, 6.7, 6.8, 6.9, 7.0, 7.1, 7.2, 7.3, 7.4, or 7.5.

### 4.5 Harvest Hold pH change

During the production and purification process, there may be a hold step between harvest of the media with the desired protein product and the purification process. This step is often referred to as the 'harvest hold' step. The duration of the harvest hold step may be anywhere from hours to days post cell culture. In one embodiment of the invention, the harvest hold step may be 0 to about 21 days or longer.

In an effort to minimize deamidation of the desired protein product, the pH of the harvest hold material may be adjusted at any time post harvest. In another embodiment of the invention, the pH of the harvest hold material is adjusted at any time post harvest. In another embodiment of the invention, the pH of the harvest hold material may be adjusted from 0 to about 21 days post harvest. The pH of the harvest hold material may be adjusted up or down with the addition of base or acid respectfully. In an embodiment of the invention, the pH of the harvest hold material is adjusted downwards. In another embodiment of the invention the pH of the harvest hold material is adjusted upwards.

In another embodiment of the invention the pH of the harvest hold material is adjusted to a value of about 6.0 to about 7.5. In one embodiment of the invention, the pH of the harvest hold material is adjusted to a value of about 7.0 to about 7.5. In one embodiment of the invention, the pH of the harvest hold material is adjusted to a value of about 6.0 to about 7.0, about 6.1 to about 7.0, about 6.2 to about 7.0, about 6.3 to about 7.0, about 6.4 to about 7.0, about 6.5 to about 7.0, about 6.6 to about 7.0, about 6.7 to about 7.0, about 6.8 to about 7.0, or about 6.9 to about 7.0. In another embodiment of the invention, the pH of the harvest hold material is adjusted to a value of about 6.0 to about 7.2, about 6.0 to about 7.0, about 6.0 to about 6.9, about 6.0 to about 6.8, about 6.0 to about 6.7, about 6.0 to about 6.6, about 6.0 to about 6.5, about 6.0 to about 6.4, about 6.0 to about 6.3, or about 6.0 to about 6.2.

In another embodiment of the invention the pH of the harvest hold material is adjusted to a value of 6.0 to 7.5. In one embodiment of the invention, the pH of the harvest hold material is adjusted to a value of 7.0 to 7.5. In one embodiment of the invention, the pH of the harvest hold material is adjusted to a value of 6.0 to 7.0, 6.1 to 7.0, 6.2 to 7.0, 6.3 to 7.0, 6.4 to 7.0, 6.5 to 7.0, 6.6 to 7.0, 6.7 to 7.0, 6.8 to 7.0, or 6.9 to 7.0. In another embodiment of the invention, the pH of the harvest hold material is adjusted to a value of 6.0 to 7.2, 6.0 to 7.0, 6.0 to 6.9, 6.0 to 6.8, 6.0 to 6.7, 6.0 to 6.6, 6.0 to 6.5, 6.0 to 6.4, 6.0 to 6.3, or 6.0 to 6.2.

In another embodiment of the invention, the pH of the harvest hold material is adjusted to a value of about 6.0 to about 6.1, 6.1 to about 6.2, about 6.2 to about 6.3, about 6.3 to about 6.4, about 6.4 to about 6.5, about 6.5 to about 6.6, about 6.6 to about 6.7, about 6.7 to about 6.8, about, 6.8 to about 6.9, about 6.9 to about 7.0, about 7.0 to about 7.1, about 7.1 to about 7.2, about 7.2 to about 7.3, about 7.3 to about 7.4 or about 7.4 to about 7.5. In another embodiment of the invention, the pH of the harvest hold material is adjusted to a value of about 6.0, about 6.1, about 6.2, about 6.3, about 6.4, about 6.5, about 6.6, about 6.7, about 6.8, about 6.9, about 7.0, about 7.1, about 7.2, about 7.3, about 7.4, or about 7.5.

In another embodiment of the invention, the pH of the harvest hold material is adjusted to a value of 6.0 to 6.1, 6.1 to 6.2, 6.2 to 6.3, 6.3 to 6.4, 6.4 to 6.5, 6.5 to 6.6, 6.6 to 6.7, 6.7 to 6.8, about, 6.8 to 6.9, 6.9 to 7.0, 7.0 to 7.1, 7.1 to 7.2, 7.2 to 7.3, 7.3 to 7.4 or 7.4 to 7.5. In another embodiment of the invention, the pH of the harvest hold material is adjusted to a value of 6.0, 6.1, 6.2, 6.3, 6.4, 6.5, 6.6, 6.7, 6.8, 6.9, 7.0, 7.1, 7.2, 7.3, 7.4, or 7.5.

In an embodiment of the invention the cell culture temperature is adjusted. In another embodiment, the cell culture temperature is adjusted and the pH of the media is adjusted. In another embodiment, the cell culture temperature is adjusted, the pH of the media is adjusted, and the length of cell culture run is adjusted. In another embodiment of the invention, any one of the cell culture parameters described above could be manipulated concurrently.

### 4.6 Purification of antibodies

Once a peptide, polypeptide, protein or a fusion protein of the invention has been produced by recombinant expression, it may be purified by any method known in the art for purification of a protein, for example, by chromatography (for example, ion exchange, affinity, particularly by affinity for the specific antigen after Protein A, and sizing column chromatography), centrifugation, differential solubility, or by any other standard technique for the purification of proteins.

When using recombinant techniques, the antibody can be produced intracellularly or directly secreted into the medium. If the antibody is produced intracellularly, as a first step, the particulate debris, either host cells or lysed fragments, is removed, for example, by centrifugation or ultrafiltration. Cell debris can be removed by centrifugation. Where the antibody is secreted into the medium, supernatants from such expression systems are generally first concentrated using a commercially available protein concentration filter, for example, an AMICON or MILLIPORE Pellicon® ultrafiltration unit. Similarly, the cell debris can be removed by tangential flow hollow fiber microfiltration (TFF). The resulting conditioned media (CM) is subjected to further purification. A protease inhibitor such as PMSF may be included in any of the foregoing steps to inhibit proteolysis and antibiotics may be included to prevent the growth of adventitious contaminants.

The antibody composition prepared from the cells is subjected to at least one purification step. Examples of suitable purification steps include hydroxyapatite chromatography, cation chromatography, anion chromatography, hydrophobic charge induction chromatography (HCIC), gel electrophoresis, dialysis, and affinity chromatography. The suitability of protein A as an affinity ligand depends on the species and isotype of any immunoglobulin Fc domain that is present in the antibody. Protein A can be used to purify antibodies that are based on human γ1, γ2, or γ4 heavy chains (Lindmark et al., J. Immunol. Meth. 62:1-13 [1983]). Protein G is recommended for all mouse isotypes and for human γ3 (Guss et al., EMBO J. 5:15671575 [1986]). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Other techniques for protein purification such as fractionation on an ion-exchange column, ethanol precipitation, Reverse Phase HPLC, chromatography on silica, chromatography on heparin Sepharose™, chromatography on an anion or cation exchange resin (such as a polyaspartic acid column), chromatofocusing, SDS-PAGE, and ammonium sulfate precipitation are also available depending on the antibody to be recovered.

Following any preliminary purification step(s), the mixture comprising the antibody of interest and contaminant(s) is subjected to a viral inactivation step. Often, the antibody composition to be purified will be present in a buffer from the previous purification step. However, it may be necessary to add a buffer to the antibody composition prior to the viral inactivation step. Many buffers are available and can be selected by routine experimentation. The pH of the mixture comprising the antibody to be purified and at least one contaminant (viral particles) is adjusted to a pH of about 2.5-4.5 using either an acid or base, depending on the starting pH and routinely incubated for 60-75 min at RT.

The mixture may be loaded on an ion exchange column. Ion Exchange Chromatography relies on charge-charge interactions between the proteins in a sample and the charges immobilized on the resin of choice. Ion exchange chromatography can be subdivided into cation exchange chromatography, in which positively charged ions bind to a negatively charged resin; and anion exchange chromatography, in which the binding ions are negative, and the immobilized functional group is positive. Once the solutes are bound, the column is washed to equilibrate it in the starting buffer, which should be of low ionic strength, then the bound molecules are eluted off using a gradient of a second buffer which steadily increases the ionic strength of the eluent solution. Alternatively, the pH of the eluent buffer can be modified as to give the protein or matrix a charge at which they will not interact and your molecule of interest elutes from the resin.

In certain embodiments of the invention, the harvested material is loaded onto a cation exchange column. A non-limiting example of a suitable cation exchange resin is a HS50 resin, commercially available for a variety of sources. Another non-limiting example of a suitable cation exchange resin is Fractogel^{®}EMD media (Merck KGa).

The amount of material loaded on to a chromatography column may affect the efficient recovery of intact material. More specifically, some columns may be overloaded to the point where resolution of intact and deamidated species overlap, leading to inefficient recovery. To alleviate this problem, it is understood that the protein concentrations of loaded material (protein load) need to be optimized. Accordingly, in some embodiments, the protein load concentration is less than 100 mg/ml, less than 75 mg/ml, less than 50 mg/ml, less than 25 mg/ml, less than 20 mg/ml, or less than 15 mg/ml. In other embodiments, the protein load concentration is about 5mg/ml to about 15 mg/ml, about 10mg/ml to about 20 mg/ml, about 15 mg/ml to about 50 mg/ml, about 20 mg/ml to about 50 mg/ml, about 25 mg/ml to about 50 mg/ml, about 30 mg/ml to about 50 mg/ml, or about 50 mg/ml to about 100 mg/ml. In a specific embodiment, the protein load concentration is 15 mg/ml or less. In yet another specific embodiment, the protein load concentration is 50 mg/ml or less.

After loading of the harvested material, it is well understood that a range of washes must take place to remove many of the impurities present in the loaded material. The wash steps would need to be optimized for each specific application. Some of the parameters that can be adjusted are buffer choice, pH, osmolality, and surfactant (such as polysorbate 80) concentration. More specifically, the osmolality can be increased by an increasing amount of a solute including but not limited to NaCl. The solute concentration can be adjusted from 0 mM to 2 M in the wash buffer to aide in the purification of the desired product. In one embodiment of the invention, the number of wash steps ranges from 1, 2, 3, 4, 5, or more steps to purify the desired product. In another embodiment of the invention, the buffer choice is adjusted to aide in the purification of the desired product. Exemplary buffers include, but are not limited to, sodium phosphate, HEPES, Tris, potassium phosphate, or sodium phosphate. In another embodiment of the invention, the pH is adjusted to aide in the purification of the desired product. In another embodiment of the invention, the concentration of surfactant is adjusted to aide in the purification of the desired product. In another embodiment of the invention, the osmolality of the wash buffer is adjusted to aide in the purification of the desired product. In another embodiment, the solute concentration in the wash buffer is in a range from about 0 mM to about 2M, about 0 mM to about 1 M, about 0 mM to about 500 mM, or about 0 mM to about 100 mM. In another embodiment of the invention, the solute concentration in the wash buffer is in a range from about 5mM to about 500 mM. In another embodiment of the invention, the solute concentration in the wash buffer is about 5mM, about 10 mM, about 15 mM, about 20 mM, about 25 mM, about 30 mM, about 35 mM, about 40 mM, about 45 mM, or about 50 mM. In another embodiment of the invention, the wash buffer comprises sodium phosphate as a buffering agent and sodium chloride as an adjustable solute agent. Another embodiment of the invention comprises eluting the desired product off the cation exchange column with an increasing gradient of solute.

In an embodiment of the invention, the NaCl concentration in the wash buffer is in a range from about 0 mM to about 100 mM. In another embodiment, the NaCl concentration in the wash buffer is about 5mM, about 10 mM, about 15 mM, about 20 mM, about 25 mM, about 30 mM, about 35 mM, about 40 mM, about 45 mM, or about 50 mM. In another embodiment, the NaCl concentration in the wash buffer is 35 mM. In a specific embodiment, the NaCl concentration in the wash buffer is 30 mM.

In certain embodiments, the mixture may be loaded on a HCIC column. HCIC columns normally comprise a base matrix (for example, cross-linked cellulose or synthetic copolymer material) to which hydrophobic ligands are coupled. Many HCIC columns are available commercially. A non-limiting example is MEP Hypercel® (Pall, New York). HCIC is controlled on the basis of pH rather than salt concentration. Antibody elution is conducted at low ionic strength, eliminating the need for extensive diafiltration in applications where ion exchange chromatography will follow capture. Compared to chromatography on Protein A sorbents, elution from HCIC columns is achieved under relatively mild conditions (pH 4.0). Under such conditions, antibody molecules also carry a positive charge. Electrostatic repulsion is induced and antibody is desorbed.

The antibody is eluted from the HCIC column using an elution buffer which is normally the same as the loading buffer. The elution buffer can be selected using routine experimentation. The pH of the elution buffer is between about 2.5-6.5 and has a low salt concentration (*i.e.* less than about 0.25 M salt). It has been discovered previously that it is not necessary to use a salt gradient to elute the antibody of interest as the desired product may be recovered in the flow through fraction which does not bind significantly to the column.

### 4.7 Additional embodiments

An embodiment of the invention is a method of producing an antibody with a decreased deamidation profile, wherein said antibody would otherwise be predisposed to an elevated deamidation profile. In a further embodiment, the antibody contains an asparagine residue preceding a deamidation trigger residue such as glycine, serine, threonine or an aspartic acid residue. In a further embodiment, the antibody contains an asparagine followed by a deamidation trigger residue both of which are located in at least one of the VHCDR1, VHCDR2, VHCDR3, VLCDR1, VLCDR2, or VLCDR3 regions of the antibody. In yet another further embodiment, the antibody contains an asparagine followed by a deamidation trigger residue located within the VHCDR2 of the antibody. In a further specific embodiment, the antibody is 13H5. In other embodiments, the antibody is 13H7 or 7H9.

An embodiment of the invention is a method of producing an antibody with a decreased deamidation profile, wherein said antibody would otherwise be predisposed to an elevated deamidation profile, wherein said antibody deamidation profile is reduced by about 60%, about 50%, about 40%, about 30%, about 20%, or about 10% as compared to a control deamidation profile.

An embodiment of the invention is a method of producing an antibody with a decreased deamidation profile, wherein said antibody would otherwise be predisposed to an elevated deamidation profile, wherein said method comprises production of an antibody from cells grown at a temperature from the range consisting of 30 to about 37 °C. In a further embodiment, the antibody producing cells are grown at 34°C. In a further embodiment, the antibody producing cells are grown in media at a pH from the range consisting of 6.0 to about 7.2 pH units. In a further embodiment, the antibody producing cells are grown in media with a pH of 6.9 pH units.

An embodiment of the invention is a method of producing an antibody with a decreased deamidation profile, wherein the antibody would otherwise be predisposed to an elevated deamidation profile, wherein the antibody producing cells are grown in a biphasic culture.

An embodiment of the invention is a method of producing an antibody with a decreased deamidation profile, wherein the antibody would otherwise be predisposed to an elevated deamidation profile, wherein the method includes a pH change of the media at the time of harvest. In a further embodiment, the pH is adjusted to a range consisting of 5.0 to about 7.0 pH units at the time of harvest. In a further embodiment, the pH is adjusted to 6.9 pH units at the time of harvest.

An embodiment of the invention is a method of producing an antibody with a decreased deamidation profile, wherein the antibody would otherwise be predisposed to an elevated deamidation profile, wherein the method comprises a hold step after cell harvest including a pH change. In a further embodiment, the pH is adjusted to a range consisting of 5.0 to about 7.0 pH units. In a further embodiment, the pH is adjusted to 6.0 pH units during the harvest hold step.

An embodiment of the invention is a method of producing an antibody with a decreased deamidation profile, wherein the antibody would otherwise be predisposed to an elevated deamidation profile, wherein the method includes a dilution step. In a further embodiment, the dilution step is an in-line dilution or a tank dilution step. In a further embodiment, the method does not include an ultrafiltration step.

An embodiment of the invention is a method of producing an antibody with a decreased deamidation profile, wherein the antibody would otherwise be predisposed to an elevated deamidation profile, wherein the method includes a residence time of less than 17 days.

An embodiment of the invention is a method of producing an antibody with a decreased deamidation profile, wherein the antibody would otherwise be predisposed to an elevated deamidation profile, wherein the antibody is specific for interferon alpha. In a further embodiment, the antibody is 13H5.

An embodiment of the invention is a method of producing an antibody with a decreased deamidation profile, wherein the antibody would otherwise be predisposed to an elevated deamidation profile, wherein the method includes the following steps: producing the antibody from cells grown at a temperature from about 33 °C to about 35 °C, the cells are grown in media with a pH value of about 6.7 to about 7.1 pH units, and the culturing the cells takes 15-19 days. In a further embodiment of the invention, the culturing of the cells takes 17 days. In a further embodiment, the antibody is 13H5.

An embodiment of the invention is a stable anti-IFN alpha monoclonal antibody composition with a decreased deamidation profile, wherein the antibody contains amino acid sequences that predispose said antibody to an elevated deamidation profile. In a further embodiment, the antibody contains adjacent an asparagine residue the deamidation trigger residues: glycine, serine, threonine or an aspartic acid residue. In a further embodiment, the asparagine and deamidation trigger residue are located in at least one of the VHCDR1, VHCDR2, VHCDR3, VLCDR1, VLCDR2, or VLCDR3 regions of the antibody. In a further embodiment, the asparagine and deamidation trigger residue are located in the VHCDR2 of the antibody. In a further embodiment, the antibody is 13H5.

An embodiment of the invention is a stable anti-IFN alpha monoclonal antibody composition with a decreased deamidation profile, wherein the antibody contains amino acid sequences that predispose said antibody to an elevated deamidation profile wherein the antibody deamidation profile is reduced by about 60%, about 50%, about 40%, about 30%, about 20%, or about 10% as compared to a control deamidated profile. In a further embodiment, the antibody is an antibody fragment. In a further embodiment, the antibody fragment is selected from the group consisting of a Fab fragment, a F(ab')₂ fragment, a Fab' fragment, and an scFv.

An embodiment of the invention is a stable anti-IFN alpha monoclonal antibody composition with a decreased deamidation profile, wherein the antibody contains amino acid sequences that predispose said antibody to an elevated deamidation profile, wherein the antibody composition is produced by a process comprising growing antibody producing cells at a temperature of about 34 °C, wherein the antibody producing cells are grown in media with a pH of about 6.9 pH units. In a further embodiment, the antibody is 13H5.

An embodiment of the invention is an antibody composition with a decreased deamidation profile, wherein the antibody is otherwise predisposed to an elevated deamidation profile, produced by the process comprising, growing antibody producing cells at about 34 °C, wherein the antibody producing cells are grown in media with a pH of about 6.9 pH units. In a further embodiment, the antibody is 13H5.

An embodiment of the invention is an antibody composition with a decreased deamidation profile, wherein the antibody is otherwise predisposed to an elevated deamidation profile, produced by the process comprising growing antibody producing cells at about 33 °C to about 35 °C, wherein the cells are grown in a media with a pH of about 6.7 to about 7.1 units, and culturing the antibody producing cells for about 15 to about 19 days. In a further embodiment, the cells are grown at 34 °C. In a further embodiment, the cells are grown in a media with a pH of 6.9 pH units. In a further embodiment, the cells are cultured for 17 days. In a further embodiment, the antibody is 13H5.

An embodiment of the invention is a method of purifying an antibody predisposed to an elevated deamidation profile, wherein the method comprises a wash step during purification for removal of the deamidated species of the antibody. In a further embodiment, the wash step comprises a buffer with a salt concentration of about 0 mM to 100 mM. In a further embodiment, the salt concentration is 35 mM. In a further embodiment, the antibody is 13H5.

### 4.7 Antibodies

### 4.7.1 Antibody types

Antibodies of the invention include, but are not limited to, synthetic antibodies, monoclonal antibodies, recombinantly produced antibodies, intrabodies, multispecific antibodies (including bi-specific antibodies), human antibodies, humanized antibodies, chimeric antibodies, synthetic antibodies, single-chain Fvs (scFv) (including bi-specific scFvs), BiTE® molecules, single chain antibodies Fab fragments, F(ab') fragments, disulfide-linked Fvs (dsFv), and anti-idiotypic (anti-Id) antibodies, and epitope-binding fragments of any of the above. In particular, antibodies of the present invention include immunoglobulin molecules and immunologically active portions of immunoglobulin molecules. Furthermore, the antibodies of the invention can be of any isotype. In one embodiment, antibodies of the invention are of the IgG1, IgG2, IgG3 or IgG4 isotype. The antibodies of the invention can be full-length antibodies comprising variable and constant regions, or they can be antigen-binding fragments thereof, such as a single chain antibody, or a Fab or Fab'₂ fragment.

In other embodiments, the invention also provides an immunoconjugate comprising an antibody of the invention, or antigen-binding portion thereof, linked to a therapeutic agent, such as a cytotoxin or a radioactive isotope. In certain embodiments, the invention also provides a bispecific molecule comprising an antibody, or antigen-binding portion thereof, of the invention, linked to a second functional moiety having a different binding specificity than said antibody, or antigen binding portion thereof.

Compositions comprising an antibody, or antigen-binding portion thereof, or immunoconjugate or bispecific molecule of the invention and a pharmaceutically acceptable carrier are also provided.

### 4.7.2 Antibodies specific for IFN alpha

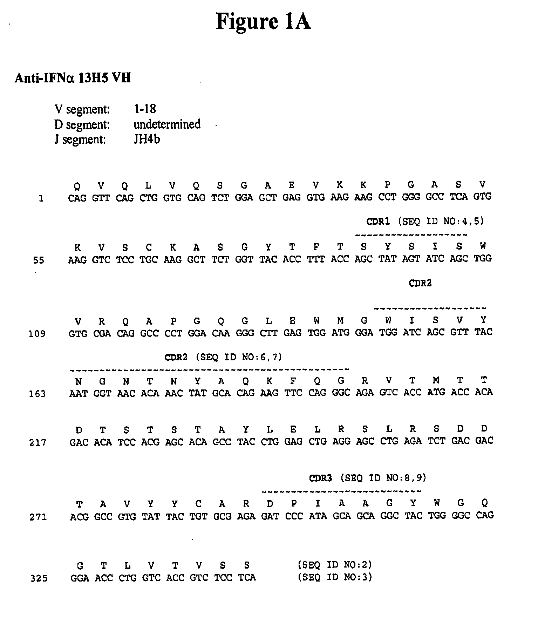

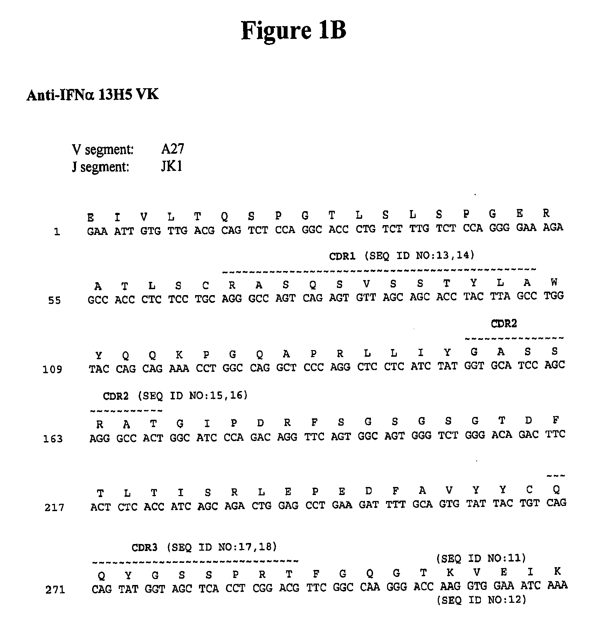

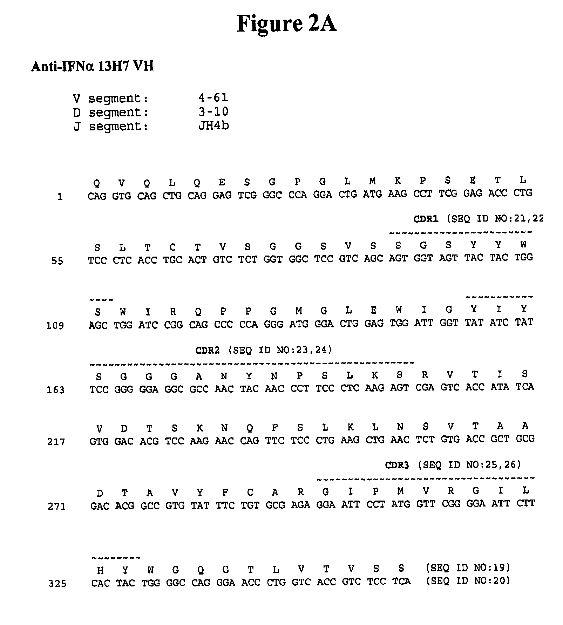

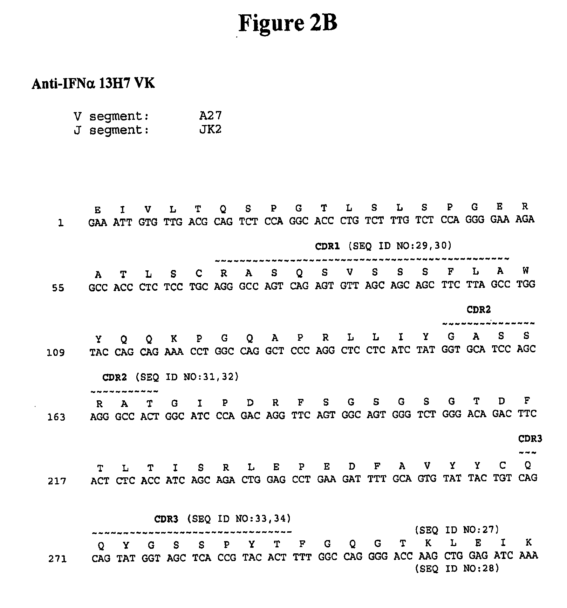

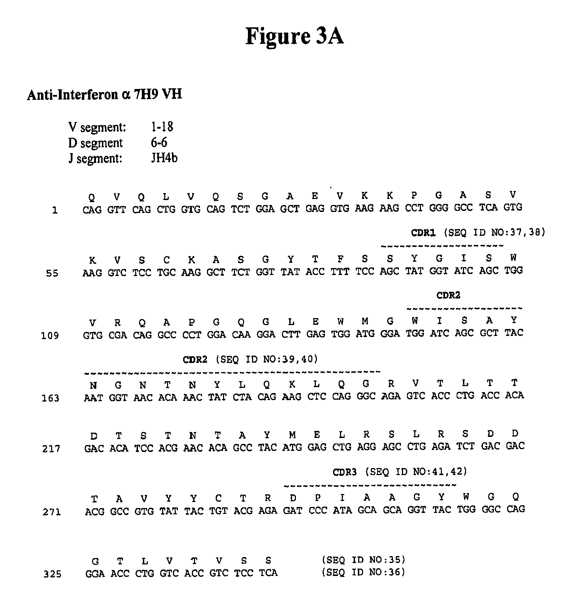

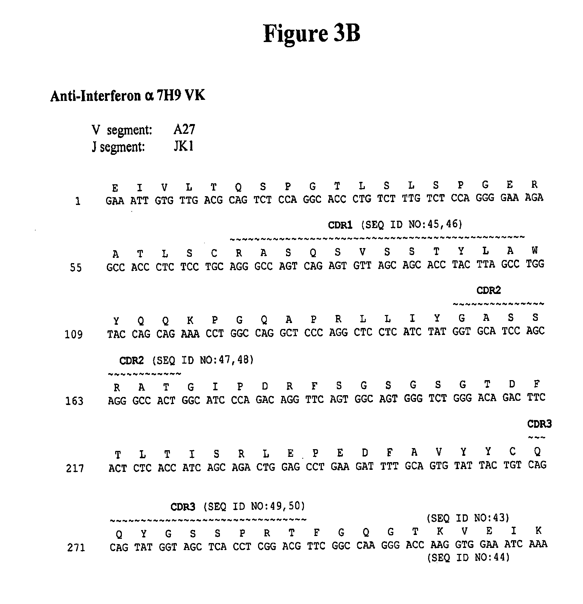

In a specific embodiment, the invention provides antibodies specific for IFN alpha. In certain embodiments, the anti-IFN alpha antibodies of the invention comprise 13H5 (Figure 1A, B, 13H7 (Figure 2A, B), and 7H9 (Figure 3A, B). In other embodiments, anti-IFN alpha antibodies of the invention are also exemplified in the publications WO 2005/059106 and US 2007/0014724 and the US application serial No. 11/009,410 all entitled "Interferon alpha antibodies and their uses".

In an embodiment of the invention, the anti-interferon alpha antibody is specific for the interferon alpha subtypes: alpha1, alpha2, alpha4, alpha5, alpha8, alpha10, and alpha21. In other embodiments, anti-interferon alpha antibodies are specific for at least one interferon alpha subtype selected from the group consisting of alpha1, alpha2, alpha4, alpha5, alpha8, alpha10, and alpha21. In other embodiments, anti-interferon alpha antibodies are specific for at least two, at least three, at least four, at least five, at least six or at least seven interferon alpha subtypes selected from the group consisting of alpha1, alpha2, alpha4, alpha5, alpha8, alpha10, and alpha21. In alternative embodiments, anti-interferon alpha antibodies are not specific for at least one interferon alpha subtype selected from the group consisting of alpha1, alpha2, alpha4, alpha5, alpha8, alpha 10, and alpha21.

### 4.7.3 Antibody Conjugates

The present invention encompasses the use of antibodies or fragments thereof conjugated or fused to one or more moieties, including but not limited to, peptides, polypeptides, proteins, fusion proteins, nucleic acid molecules, small molecules, mimetic agents, synthetic drugs, inorganic molecules, and organic molecules.

The present invention encompasses the use of antibodies or fragments thereof recombinantly fused or chemically conjugated (including both covalent and noncovalent conjugations) to a heterologous protein or polypeptide (or fragment thereof, for example, a polypeptide of at least 10, at least 20, at least 30, at least 40, at least 50, at least 60, at least 70, at least 80, at least 90 or at least 100 amino acids) to generate fusion proteins. The fusion does not necessarily need to be direct, but may occur through linker sequences. For example, antibodies may be used to target heterologous polypeptides to particular cell types, either *in vitro* or *in vivo,* by fusing or conjugating the antibodies to antibodies specific for particular cell surface receptors. Antibodies fused or conjugated to heterologous polypeptides may also be used in *in vitro* immunoassays and purification methods using methods known in the art. See for example, International publication No. WO 93/21232; European Patent No. EP 439,095; Naramura et al., 1994, Immunol. Lett. 39:91-99; U.S. Pat. No. 5,474,981; Gillies et al., 1992, PNAS 89:1428-1432; and Fell et al., 1991, J. Immunol. 146:2446-2452, which are incorporated by reference in their entireties.

The present invention further includes compositions comprising heterologous proteins, peptides or polypeptides fused or conjugated to antibody fragments. For example, the heterologous polypeptides may be fused or conjugated to a Fab fragment, Fd fragment, Fv fragment, F(ab)₂ fragment, a VH domain, a VL domain, a VH CDR, a VL CDR, or fragment thereof. Methods for fusing or conjugating polypeptides to antibody portions are well-known in the art. See, for example, U.S. Pat. Nos. 5,336,603, 5,622,929, 5,359,046, 5,349,053, 5,447,851, and 5,112,946; European Patent Nos. EP 307,434 and EP 367,166; International publication Nos. WO 96/04388 and WO 91/06570; Ashkenazi et al., 1991, Proc. Natl. Acad. Sci. USA 88: 10535-10539; Zheng et al., 1995, J. Immunol. 154:5590-5600; and Vil et al., 1992, Proc. Natl. Acad. Sci. USA 89:11337-11341 (said references incorporated by reference in their entireties).

Additional fusion proteins may be generated through the techniques of gene-shuffling, motif-shuffling, exon-shuffling, and/or codon-shuffling (collectively referred to as "DNA shuffling"). DNA shuffling may be employed to alter the activities of antibodies of the invention or fragments thereof (for example, antibodies or fragments thereof with higher affinities and lower dissociation rates). See, generally, U.S. Pat. Nos. 5,605,793; 5,811,238; 5,830,721; 5,834,252; and 5,837,458, and Patten et al., 1997, Curr. Opinion Biotechnol. 8:724-33; Harayama, 1998, Trends Biotechnol. 16(2):76-82; Hansson, et al., 1999, J. Mol. Biol. 287:265-76; and Lorenzo and Blasco, 1998, Biotechniques 24(2):308-313 (each of these patents and publications are hereby incorporated by reference in its entirety). Antibodies or fragments thereof, or the encoded antibodies or fragments thereof, may be altered by being subjected to random mutagenesis by error-prone PCR, random nucleotide insertion or other methods prior to recombination. One or more portions of a polynucleotide encoding an antibody or antibody fragment, which portions specifically bind to IFN alpha may be recombined with one or more components, motifs, sections, parts, domains, fragments, etc. of one or more heterologous molecules.

Moreover, the antibodies or fragments thereof can be fused to marker sequences, such as a peptide, to facilitate purification. In other embodiments, the marker amino acid sequence is a hexa-histidine peptide, such as the tag provided in a pQE vector (QIAGEN, Inc., 9259 Eton Avenue, Chatsworth, Calif., 91311), among others, many of which are commercially available. As described in Gentz et al., 1989, Proc. Natl. Acad. Sci. USA 86:821-824, for instance, hexa-histidine provides for convenient purification of the fusion protein. Other peptide tags useful for purification include, but are not limited to, the hemagglutinin "HA" tag, which corresponds to an epitope derived from the influenza hemagglutinin protein (Wilson et al., 1984, Cell 37:767) and the "FLAG" tag.

In other embodiments, antibodies of the present invention or fragments, analogs or derivatives thereof conjugated to a diagnostic or detectable agent. Such antibodies can be useful for monitoring or prognosing the development or progression of an inflammatory disorder as part of a clinical testing procedure, such as determining the efficacy of a particular therapy. Such diagnosis and detection can be accomplished by coupling the antibody to detectable substances including, but not limited to various enzymes, such as but not limited to horseradish peroxidase, alkaline phosphatase, beta-galactosidase, or acetylcholinesterase; prosthetic groups, such as but not limited to streptavidin/biotin and avidin/biotin; fluorescent materials, such as but not limited to, umbelliferone, fluorescein, fluorescein isothiocynate, rhodamine, dichlorotriazinylamine fluorescein, dansyl chloride or phycoerythrin; luminescent materials, such as, but not limited to, luminol; bioluminescent materials, such as but not limited to, luciferase, luciferin, and aequorin; radioactive materials, such as but not limited to iodine (¹³¹I, ¹²⁵I, ¹²³I, ¹²¹I), carbon (¹⁴C), sulfur (³⁵S), tritium (³H), indium (¹¹⁵In, ¹¹³In, ¹¹²In, ¹¹¹In,), and technetium (⁹⁹Tc), thallium (²⁰¹Ti), gallium (⁶⁸Ga, ⁶⁷Ga), palladium (¹⁰³Pd), molybdenum (⁹⁹Mo) xenon (¹³³X8) fluorine (¹⁸F), ¹⁵³Sm, ¹⁷⁷Lu, ¹⁵⁹Gd, ¹⁴⁹Pm, ¹⁴⁰La, ¹⁷⁵Yb, ¹⁶⁶Ho, ⁹⁰Y, ⁴⁷Sc, ¹⁸⁶Re, ¹⁸⁸Re, ¹⁴²Pr, ¹⁰⁵Rh, ⁹⁷Ru, ⁶⁸Ge, ⁵⁷Co, ⁶⁵Zn, ⁸⁵Sr, ³²P, ¹⁵³Gd, ¹⁶⁹Yb, ⁵¹Cr, ⁵⁴Mn, ⁷⁵Se, ¹¹³Sn, and ¹¹⁷Tin; positron emitting metals using various positron emission tomographies, nonradioactive paramagnetic metal ions, and molecules that are radiolabeled or conjugated to specific radioisotopes.

The present invention further encompasses uses of antibodies or fragments thereof conjugated to a therapeutic moiety. An antibody or fragment thereof may be conjugated to a therapeutic moiety such as a cytotoxin, for example, a cytostatic or cytocidal agent, a therapeutic agent or a radioactive metal ion, for example, alpha-emitters. A cytotoxin or cytotoxic agent includes any agent that is detrimental to cells. Therapeutic moieties include, but are not limited to, antimetabolites (for example, methotrexate, 6-mercaptopurine, 6-thioguanine, cytarabine, 5-fluorouracil decarbazine), alkylating agents (for example, mechlorethamine, thioepa chlorambucil, melphalan, carmustine (BCNU) and lomustine (CCNU), cyclothosphamide, busulfan, dibromomannitol, streptozotocin, mitomycin C, and cisdichlorodiamine platinum (II) (DDP) cisplatin), anthracyclines (for example, daunorubicin (formerly daunomycin) and doxorubicin), antibiotics (for example, dactinomycin (formerly actinomycin), bleomycin, mithramycin, and anthramycin (AMC)), Auristatin molecules (for example, auristatin PHE, bryostatin 1, and solastatin 10; see Woyke et al., Antimicrob. Agents Chemother. 46:3802-8 (2002), Woyke et al., Antimicrob. Agents Chemother. 45:3580-4 (2001), Mohammad et al., Anticancer Drugs 12:735-40 (2001), Wall et al., Biochem. Biophys. Res. Commun. 266:76-80 (1999), Mohammad et al., Int. J. Oncol. 15:367-72 (1999), all of which are incorporated herein by reference), hormones (for example, glucocorticoids, progestins, androgens, and estrogens), DNA-repair enzyme inhibitors (for example, etoposide or topotecan), kinase inhibitors (for example, compound ST1571, imatinib mesylate (Kantarjian et al., Clin Cancer Res. 8(7):2167-76 (2002)), cytotoxic agents (for example, paclitaxel, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, procaine, tetracaine, lidocaine, propranolol, and puromycin and analogs or homologs thereof) and those compounds disclosed in U.S. Pat. Nos. 6,245,759, 6,399,633, 6,383,790, 6,335,156, 6,271,242, 6,242,196, 6,218,410, 6,218,372, 6,057,300 6,034,053, 5,985,877, 5,958,769, 5,925,376, 5,922,844, 5,911,995, 5,872,223, 5,863,904, 5,840,745, 5,728,868, 5,648,239, 5,587,459), famesyl transferase inhibitors (for example, R115777, BMS-214662 and those disclosed by, for example, U.S. Pat. Nos. 6,458,935, 6,451,812, 6,440,974, 6,436,960, 6,432,959, 6,420,387, 6,414,145, 6,410,541, 6,410,539, 6,403,581, 6,399,615, 6,387,905, 6,372,747, 6,369,034, 6,362,188, 6,342,765, 6,342,487, 6,300,501, 6,268,363, 6,265,422, 6,248,756, 6,239,140, 6,232,338, 6,228,865, 6,228,856, 6,225,322, 6,218,406, 6,211,193, 6,187,786, 6,169,096, 6,159,984, 6,143,766, 6,133,303, 6,127,366, 6,124,465, 6,124,295, 6,103,723, 6,093,737, 6,090,948, 6,080,870, 6,077,853, 6,071,935, 6,066,738, 6,063,930, 6,054,466, 6,051,582, 6,051,574, and 6,040,305), topoisomerase inhibitors (for example, camptothecin; irinotecan; SN-38; topotecan; 9-aminocamptothecin; GG-211 (GI 147211); DX-8951f; IST-622; rubitecan; pyrazoloacridine; XR-5000; saintopin; UCE6; UCE1022; TAN-1518A; TAN-1518B; KT6006; KT6528; ED-110; NB-506; ED-110; NB-506; and rebeccamycin); bulgarein; DNA minor groove binders such as Hoescht dye 33342 and Hoechst dye 33258; nitidine; fagaronine; epiberberine; coralyne; beta-lapachone; BC-4-1; bisphosphonates (for example, alendronate, cimadronte, clodronate, tiludronate, etidronate, ibandronate, neridronate, olpandronate, risedronate, piridronate, pamidronate, zolendronate) HMG-CoA reductase inhibitors, (for example, lovastatin, simvastatin, atorvastatin, pravastatin, fluvastatin, statin, cerivastatin, lescol, lupitor, rosuvastatin and atorvastatin) and pharmaceutically acceptable salts, solvates, clathrates, and prodrugs thereof. See, for example, Rothenberg, M. L., Annals of Oncology 8:837-855(1997); and Moreau, P., et al., J. Med. Chem. 41:1631-1640(1998), antisense oligonucleotides (for example, those disclosed in the U.S. Pat. Nos. 6,277,832, 5,998,596, 5,885,834, 5,734,033, and 5,618,709), immunomodulators (for example, antibodies and cytokines), antibodies, and adenosine deaminase inhibitors (for example, Fludarabine phosphate and 2-Chlorodeoxyadenosine). Examples include paclitaxel, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof.

Therapeutics include, but are not limited to, antimetabolites (for example, methotrexate, 6-mercaptopurine, 6-thioguanine, cytarabine, 5-fluorouracil decarbazine), alkylating agents (for example, mechlorethamine, thioepa chlorambucil, melphalan, carmustine (BCNU) and lomustine (CCNU), cyclothosphamide, busulfan, dibromomannitol, streptozotocin, mitomycin C and cisdichlorodiamine platinum (II) (DDP) cisplatin), anthracyclines (for example, daunorubicin (formerly daunomycin) and doxorubicin), antibiotics (for example, dactinomycin (formerly actinomycin), bleomycin, mithramycin, and anthramycin (AMC)), Auristatin molecules (for example, auristatin PHE, bryostatin 1, solastatin 10, see Woyke et al., Antimicrob. Agents Chemother. 46:3802-8 (2002), Woyke et al., Antimicrob. Agents Chemother. 45:3580-4 (2001), Mohammad et al., Anticancer Drugs 12:735-40 (2001), Wall et al., Biochem. Biophys. Res. Commun. 266:76-80 (1999), Mohammad et al., Int. J. Oncol. 15:367-72 (1999), all of which are incorporated herein by reference), anti-mitotic agents (for example, vincristine and vinblastine), hormones (for example, glucocorticoids, progestatins, androgens, and estrogens), DNA-repair enzyme inhibitors (for example, etoposide or topotecan), kinase inhibitors (for example, compound ST1571, imatinib mesylate (Kantarjian et al., Clin Cancer Res. 8(7):2167-76 (2002)), and those compounds disclosed in U.S. Pat. Nos. 6,245,759, 6,399,633, 6,383,790, 6,335,156, 6,271,242, 6,242,196, 6,218,410, 6,218,372, 6,057,300, 6,034,053, 5,985,877, 5,958,769, 5,925,376, 5,922,844, 5,911,995, 5,872,223, 5,863,904, 5,840,745, 5,728,868, 5,648,239, 5,587,459), farnesyl transferase inhibitors (for example, R115777, BMS-214662, and those disclosed by, for example, U.S. Pat. Nos. 6,458,935, 6,451,812, 6,440,974, 6,436,960, 6,432,959, 6,420,387, 6,414,145, 6,410,541, 6,410,539, 6,403,581, 6,399,615, 6,387,905, 6,372,747, 6,369,034, 6,362,188, 6,342,765, 6,342,487, 6,300,501, 6,268,363, 6,265,422, 6,248,756, 6,239,140, 6,232,338, 6,228,865, 6,228,856, 6,225,322, 6,218,406, 6,211,193, 6,187,786, 6,169,096, 6,159,984, 6,143,766, 6,133,303, 6,127,366, 6,124,465, 6,124,295, 6,103,723, 6,093,737, 6,090,948, 6,080,870, 6,077,853, 6,071,935, 6,066,738, 6,063,930, 6,054,466, 6,051,582, 6,051,574, and 6,040,305), topoisomerase inhibitors (for example, camptothecin; irinotecan; SN-38; topotecan; 9-aminocamptothecin; GG-211 (GI 147211); DX-8951f; IST-622; rubitecan; pyrazoloacridine; XR-5000; saintopin; UCE6; UCE1022; TAN-1518A; TAN-1518B; KT6006; KT6528; ED-110; NB-506; ED-110; NB-506; and rebeccamycin; bulgarein; DNA minor groove binders such as Hoescht dye 33342 and Hoechst dye 33258; nitidine; fagaronine; epiberberine; coralyne; beta-lapachone; BC-4-1; and pharmaceutically acceptable salts, solvates, clathrates, and prodrugs thereof. See, for example, Rothenberg, M. L., Annals of Oncology 8:837-855(1997); and Moreau, P., et al., J. Med. Chem. 41:1631-1640(1998)), antisense oligonucleotides (for example, those disclosed in the U.S. Pat. Nos. 6,277,832, 5,998,596, 5,885,834, 5,734,033, and 5,618,709), immunomodulators (for example, antibodies and cytokines), antibodies (for example, rituximab (RITUXAN®), calicheamycin (MYLOTARG®, ibritumomab tiuxetan (ZEVALIN®), and tositumomab (BEXXAR®), TNF-inhibitors (including adalimumab (HUMIRA®), etanercept (ENBREL®) and infliximab (REMICADE®)), and adenosine deaminase inhibitors (for example, Fludarabine phosphate and 2-Chlorodeoxyadenosine).

Further, an antibody or fragment thereof may be conjugated to a therapeutic moiety or drug moiety that modifies a given biological response. Therapeutic moieties or drug moieties are not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, cholera toxin, or diphtheria toxin; a protein such as tumor necrosis factor, α-interferon, β-interferon, nerve growth factor, platelet derived growth factor, tissue plasminogen activator, an apoptotic agent, for example, TNF-α, TNF-β, AIM I (see, International publication No. WO 97/33899), AIM II (see, International Publication No. WO 97/34911), Fas Ligand (Takahashi et al., 1994, J. Immunol., 6:1567-1574), and VEGI (see, International publication No. WO 99/23105), a thrombotic agent or an anti-angiogenic agent, for example, angiostatin, endostatin or a component of the coagulation pathway (for example, tissue factor); or, a biological response modifier such as, for example, a lymphokine (for example, interleukin-1 ("IL-1"), interleukin-2 ("IL-2"), interleukin-6 ("IL-6"), granulocyte macrophage colony stimulating factor ("GM-CSF"), and granulocyte colony stimulating factor ("G-CSF")), a growth factor (for example, growth hormone ("GH")), or a coagulation agent (for example, calcium, vitamin K, tissue factors, such as but not limited to, Hageman factor (factor XII), high-molecular-weight kininogen (HMWK), prekallikrein (PK), coagulation proteins-factors II (prothrombin), factor V, XIIa, VIII, XIIIa, XI, XIa, IX, IXa, X, phospholipid. fibrinopeptides A and B from the α and β chains of fibrinogen, fibrin monomer).

Moreover, an antibody can be conjugated to therapeutic moieties such as a radioactive metal ion, such as alpha-emitters such as ²¹³Bi or macrocyclic chelators useful for conjugating radiometal ions, including but not limited to, ¹³¹In, ¹³¹LU, ¹³¹Y, ¹³¹Ho, ¹³¹Sm, to polypeptides. In certain embodiments, the macrocyclic chelator is 1,4,7,10-tetraazacyclododecane-N,N',N",N"'-tetra-acetic acid (DOTA) which can be attached to the antibody via a linker molecule. Such linker molecules are commonly known in the art and described in Denardo et al., 1998, Clin Cancer Res. 4(10):2483-90; Peterson et al., 1999, Bioconjug. Chem. 10(4):553-7; and Zimmerman et al., 1999, Nucl. Med. Biol. 26(8):943-50, each incorporated by reference in their entireties.

Techniques for conjugating therapeutic moieties to antibodies are well known, see, for example, Arnon et al., "Monoclonal Antibodies For Immunotargeting Of Drugs In Cancer Therapy", in Monoclonal Antibodies And Cancer Therapy, Reisfeld et al. (eds.), pp. 243-56. (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies 84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., 1982, Immunol. Rev. 62:119-58.

Alternatively, an antibody can be conjugated to a second antibody to form an antibody heteroconjugate as described by Segal in U.S. Pat. No. 4,676,980, which is incorporated herein by reference in its entirety.

The therapeutic moiety or drug conjugated to an antibody or fragment thereof that specifically binds to IFN alpha should be chosen to achieve the desired prophylactic or therapeutic effect(s) for a particular disorder in a subject. A clinician or other medical personnel should consider the following when deciding on which therapeutic moiety or drug to conjugate to an antibody or fragment thereof that specifically binds to IFN alpha: the nature of the disease, the severity of the disease, and the condition of the subj ect.

Antibodies may also be attached to solid supports, which are particularly useful for immunoassays or purification of the target antigen. Such solid supports include, but are not limited to, glass, cellulose, polyacrylamide, nylon, polystyrene, polyvinyl chloride or polypropylene.

### 4.7.4 Methods of generating antibodies

The antibodies or fragments thereof can be produced by any method known in the art for the synthesis of antibodies, in particular, by chemical synthesis or by recombinant expression techniques.

Polyclonal antibodies to IFN alpha can be produced by various procedures well known in the art. For example, IFN alpha or immunogenic fragments thereof can be administered to various host animals including, but not limited to, rabbits, mice, rats, etc. to induce the production of sera containing polyclonal antibodies specific for IFN alpha. Various adjuvants may be used to increase the immunological response, depending on the host species, and include but are not limited to, Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (bacille Calmette-Guerin) and corynebacterium parvum. Such adjuvants are also well known in the art.

Monoclonal antibodies can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al., Antibodies: A Laboratory Manual, (Cold Spring Harbor Laboratory Press, 2nd ed. 1988); Hammerling, et al., in: Monoclonal Antibodies and T-Cell Hybridomas 563-681 (Elsevier, N.Y., 1981) (said references incorporated by reference in their entireties). The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced.

Methods for producing and screening for specific antibodies using hybridoma technology are routine and well known in the art. Briefly, mice can be immunized with IFN alpha and once an immune response is detected, for example, antibodies specific for IFN alpha are detected in the mouse serum, the mouse spleen is harvested and splenocytes isolated. The splenocytes are then fused by well known techniques to any suitable myeloma cells, for example cells from cell line SP20 available from the ATCC. Hybridomas are selected and cloned by limited dilution. The hybridoma clones are then assayed by methods known in the art for cells that secrete antibodies capable of binding a polypeptide of the invention. Ascites fluid, which generally contains high levels of antibodies, can be generated by immunizing mice with positive hybridoma clones.

Accordingly, monoclonal antibodies can be generated by culturing a hybridoma cell secreting an antibody of the invention wherein, the hybridoma is generated by fusing splenocytes isolated from a mouse immunized with IFN alpha with myeloma cells and then screening the hybridomas resulting from the fusion for hybridoma clones that secrete an antibody able to bind IFN alpha.

Antibody fragments which recognize specific IFN alpha epitopes may be generated by any technique known to those of skill in the art. For example, Fab and F(ab')₂ fragments of the invention may be produced by proteolytic cleavage of immunoglobulin molecules, using enzymes such as papain (to produce Fab fragments) or pepsin (to produce F(ab')₂ fragments). F(ab')₂ fragments contain the variable region, the light chain constant region and the CH1 domain of the heavy chain. Further, the antibodies of the present invention can also be generated using various phage display methods known in the art.

In phage display methods, functional antibody domains are displayed on the surface of phage particles which carry the polynucleotide sequences encoding them. In particular, DNA sequences encoding VH and VL domains are amplified from animal cDNA libraries (for example, human or murine cDNA libraries of lymphoid tissues). The DNA encoding the VH and VL domains are recombined together with an scFv linker by PCR and cloned into a phagemid vector (for example, p CANTAB 6 or pComb 3 HSS). The vector is electroporated in E. coli and the E. coli is infected with helper phage. Phage used in these methods are typically filamentous phage including fd and M13 and the VH and VL domains are usually recombinantly fused to either the phage gene III or gene VIII. Phage expressing an antigen binding domain that binds to the IFN alpha epitope of interest can be selected or identified with antigen, for example, using labeled antigen or antigen bound or captured to a solid surface or bead. Examples of phage display methods that can be used to make the antibodies of the present invention include those disclosed in Brinkman et al., 1995, J. Immunol. Methods 182:41-50; Ames et al., 1995, J. Immunol. Methods 184:177-186; Kettleborough et al., 1994, Eur. J. Immunol. 24:952-958; Persic et al., 1997, Gene 187:9-18; Burton et al., 1994, Advances in Immunology 57:191-280; International Application No. PCT/GB91/01134; International Publication Nos. WO 90/02809, WO 91/10737, WO 92/01047, WO 92/18619, WO 93/11236, WO 95/15982, WO 95/20401, and WO97/13844; and U.S. Pat. Nos. 5,698,426, 5,223,409, 5,403,484, 5,580,717, 5,427,908, 5,750,753, 5,821,047, 5,571,698, 5,427,908, 5,516,637, 5,780,225, 5,658,727, 5,733,743 and 5,969,108; each of which is incorporated herein by reference in its entirety.

As described in the above references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria, for example, as described below. Techniques to recombinantly produce Fab, Fab' and F(ab')₂ fragments can also be employed using methods known in the art such as those disclosed in International Publication No. WO 92/22324; Mullinax et al., 1992, BioTechniques 12(6):864-869; Sawai et al., 1995, AJRI 34:26-34; and Better et al., 1988, Science 240:1041-1043 (said references incorporated by reference in their entireties).

To generate whole antibodies, PCR primers including VH or VL nucleotide sequences, a restriction site, and a flanking sequence to protect the restriction site can be used to amplify the VH or VL sequences in scFv clones. Utilizing cloning techniques known to those of skill in the art, the PCR amplified VH domains can be cloned into vectors expressing a VH constant region, for example the human gamma 4 constant region, and the PCR amplified VL domains can be cloned into vectors expressing a VL constant region, for example, human kappa or lamba constant regions. In one embodiment, the vectors for expressing the VH or VL domains comprise an EF-1α promoter, a secretion signal, a cloning site for the variable domain, constant domains, and a selection marker such as neomycin. The VH and VL domains may also be cloned into one vector expressing the necessary constant regions. The heavy chain conversion vectors and light chain conversion vectors are then co-transfected into cell lines to generate stable or transient cell lines that express full-length antibodies, for example, IgG, using techniques known to those of skill in the art.

For some uses, including *in vivo* use of antibodies in humans and *in vitro* detection assays, it may be preferable to use human or chimeric antibodies. Completely human antibodies are particularly desirable for therapeutic treatment of human subjects. Human antibodies can be made by a variety of methods known in the art including phage display methods described above using antibody libraries derived from human immunoglobulin sequences. See also U.S. Pat. Nos. 4,444,887 and 4,716,111; and International Publication Nos. WO 98/46645, WO 98/50433, WO 98/24893, WO98/16654, WO 96/34096, WO 96/33735, and WO 91/10741; each of which is incorporated herein by reference in its entirety.

Human antibodies can also be produced using transgenic mice which are incapable of expressing functional endogenous immunoglobulins, but which can express human immunoglobulin genes. For example, the human heavy and light chain immunoglobulin gene complexes may be introduced randomly or by homologous recombination into mouse embryonic stem cells. Alternatively, the human variable region, constant region, and diversity region may be introduced into mouse embryonic stem cells in addition to the human heavy and light chain genes. The mouse heavy and light chain immunoglobulin genes may be rendered non-functional separately or simultaneously with the introduction of human immunoglobulin loci by-homologous recombination. In particular, homozygous deletion of the JH region prevents endogenous antibody production. The modified embryonic stem cells are expanded and microinjected into blastocysts to produce chimeric mice. The chimeric mice are then bred to produce homozygous offspring which express human antibodies. The transgenic mice are immunized in the normal fashion with a selected antigen, for example, all or a portion of a polypeptide of the invention. Monoclonal antibodies directed against the antigen can be obtained from the immunized, transgenic mice using conventional hybridoma technology.

The human immunoglobulin transgenes harbored by the transgenic mice rearrange during B cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg and Huszar (1995, Int. Rev. Immunol. 13:65-93). For a detailed discussion of this technology for producing human antibodies and human monoclonal antibodies and protocols for producing such antibodies, see, for example, International Publication Nos. WO 98/24893, WO 96/34096, and WO 96/33735; and U.S. Pat. Nos. 5,413,923, 5,625,126, 5,633,425, 5,569,825, 5,661,016, 5,545,806, 5,814,318, and 5,939,598, which are incorporated by reference herein in their entirety. In addition, companies such as Medarex (Princeton, N.J.) can be engaged to provide human antibodies directed against a selected antigen using technology similar to that described above.

A chimeric antibody is a molecule in which different portions of the antibody are derived from different immunoglobulin molecules. Methods for producing chimeric antibodies are known in the art. See for example, Morrison, 1985, Science 229:1202; Oi et al., 1986, BioTechniques 4:214; Gillies et al., 1989, J. Immunol. Methods 125:191-202; and U.S. Pat. Nos. 5,807,715, 4,816,567, 4,816,397, and 6,311,415, which are incorporated herein by reference in their entirety.

A humanized antibody is an antibody or its variant or fragment thereof which is capable of binding to a predetermined antigen and which comprises a framework region having substantially the amino acid sequence of a human immunoglobulin and a CDR having substantially the amino acid sequence of a non-human immuoglobulin. A humanized antibody comprises substantially all of at least one, and typically two, variable domains (Fab, Fab', F(ab')₂, Fabc, Fv) in which all or substantially all of the CDR regions correspond to those of a non-human immunoglobulin (donor antibody) and all or substantially all of the framework regions are those of a human immunoglobulin consensus sequence. In other embodiments, a humanized antibody also comprises at least a portion of an immunoglobulin constant region (Fc), typically that of a human immunoglobulin. Ordinarily, the antibody will contain both the light chain as well as at least the variable domain of a heavy chain. The antibody also may include the CH1, hinge, CH2, CH3, and CH4 regions of the heavy chain. The humanized antibody can be selected from any class of immunoglobulins, including IgM, IgG, IgD; IgA and IgE, and any isotype, including IgG1, IgG2, IgG3 and lgG4. Usually the constant domain is a complement fixing constant domain where it is desired that the humanized antibody exhibit cytotoxic activity, and the class is typically IgG₁. Where such cytotoxic activity is not desirable, the constant domain may be of the IgG₂ class. The humanized antibody may comprise sequences from more than one class or isotype, and selecting particular constant domains to optimize desired effector functions is within the ordinary skill in the art.

The framework and CDR regions of a humanized antibody need not correspond precisely to the parental sequences, for example, the donor CDR or the consensus framework may be mutagenized by substitution, insertion or deletion of at least one residue so that the CDR or framework residue at that site does not correspond to either the consensus or the import antibody. Such mutations, however, will not be extensive. Usually, at least 75% of the humanized antibody residues will correspond to those of the parental framework region (FR) and CDR sequences, more often 90%, and often greater than 95%. Humanized antibody can be produced using variety of techniques known in the art, including but not limited to, CDR-grafting (European Patent No. EP 239,400; International Publication No. WO 91/09967; and U.S. Pat. Nos. 5,225,539, 5,530,101, and 5,585,089), veneering or resurfacing (European Patent Nos. EP 592,106 and EP 519,596; Padlan, 1991, Molecular Immunology 28(4/5):489-498; Studnicka et al., 1994, Protein Engineering 7(6):805-814; and Roguska et al., 1994, PNAS 91:969-973), chain shuffling (U.S. Pat. No. 5,565,332), and techniques disclosed in, for example, U.S. Pat. Nos. 6,407,213, 5,766,886, WO 9317105, Tan et al., J. Immunol. 169:1119-25 (2002), Caldas et al., Protein Eng. 13(5):353-60 (2000), Morea et al., Methods 20(3):267-79 (2000), Baca et al., J. Biol. Chem. 272(16):10678-84 (1997), Roguska et al., Protein Eng. 9(10):895-904 (1996), Couto et al., Cancer Res. 55 (23 Supp):5973s-5977s (1995), Couto et al., Cancer Res. 55(8):1717-22 (1995), Sandhu J S, Gene 150(2):409-10 (1994), and Pedersen et al., J. Mol. Biol. 235(3):959-73 (1994). Often, framework residues in the framework regions will be substituted with the corresponding residue from the CDR donor antibody to alter or improve, antigen binding. These framework substitutions are identified by methods well known in the art, for example, by modeling of the interactions of the CDR and framework residues to identify framework residues important for antigen binding and sequence comparison to identify unusual framework residues at particular positions. (See, for example, Queen et al., U.S. Pat. No. 5,585,089; and Riechmann et al., 1988, Nature 332:323, which are incorporated herein by reference in their entireties).

Further, the antibodies of the invention can, in turn, be utilized to generate anti idiotype antibodies that "mimic" IFN alpha using techniques well known to those skilled in the art. (See, for example, Greenspan & Bona, 1989, FASEB J. 7(5):437-444; and Nissinoff, 1991, J. Immunol. 147(8):2429-2438). For example, antibodies of the invention which bind to and competitively inhibit the binding of IFN alpha (as determined by assays well known in the art) to its binding partners can be used to generate anti-idiotypes that "mimic" IFN alpha binding domains and, as a consequence, bind to and neutralize IFN alpha and/or its binding partners. Such neutralizing anti-idiotypes or Fab fragments of such anti-idiotypes can be used in therapeutic regimens to neutralize IFN alpha. The invention provides methods employing the use of polynucleotides comprising a nucleotide sequence encoding an antibody of the invention or a fragment thereof.

### 4.7.4 Polynucleotides Encoding an Antibody

The methods of the invention also encompass polynucleotides that hybridize under high stringency, intermediate or lower stringency hybridization conditions, to polynucleotides that encode an antibody of the invention.

The polynucleotides may be obtained, and the nucleotide sequence of the polynucleotides determined, by any method known in the art. Since the amino acid sequences of the antibodies are known, nucleotide sequences encoding these antibodies can be determined using methods well known in the art, such as, nucleotide codons known to encode particular amino acids are assembled in such a way to generate a nucleic acid that encodes the antibody or fragment thereof of the invention. Such a polynucleotide encoding the antibody maybe assembled from chemically synthesized oligonucleotides (for example, as described in Kutmejer *et al.*, 1994, BioTechniques 17:242), which, briefly, involves the synthesis of overlapping oligonucleotides containing portions of the sequence encoding the antibody, annealing and ligating of those oligonucleotides, and then amplification of the ligated oligonucleotides by PCR.

Alternatively, a polynucleotide encoding an antibody may be generated from nucleic acid from a suitable source. If a clone containing a nucleic acid encoding a particular antibody is not available, but the sequence of the antibody molecule is known, a nucleic acid encoding the immunoglobulin may be chemically synthesized or obtained from a suitable source (for example, an antibody cDNA library, or a cDNA library generated from, or nucleic acid, such as poly A+RNA, isolated from, any tissue or cells expressing the antibody, such as hybridoma cells selected to express an antibody of the invention) by PCR amplification using synthetic primers hybridizable to the 3' and 5' ends of the sequence or by cloning using an oligonucleotide probe specific for the particular gene sequence to identify, for example, a cDNA clone from a cDNA library that encodes the antibody. Amplified nucleic acids generated by PCR may then be cloned into replicable cloning vectors using any method well known in the art.

Once the nucleotide sequence of the antibody is determined, the nucleotide sequence of the antibody may be manipulated using methods well known in the art for the manipulation of nucleotide sequences, for example, recombinant DNA techniques, site directed mutagenesis, PCR, etc. (see, for example, the techniques described in Sambrook et al., 1990, Molecular Cloning, A Laboratory Manual, 2d Ed., Cold Spring Harbor Laboratory, Cold Spring Harbor, N.Y. and Ausubel et al., eds., 1998, Current Protocols in Molecular Biology, John Wiley & Sons, NY, which are both incorporated by reference herein in their entireties), to generate antibodies having a different amino acid sequence, for example to create amino acid substitutions, deletions, and/or insertions.

In a specific embodiment, one or more of the CDRs is inserted within framework regions using routine recombinant DNA techniques. The framework regions may be naturally occurring or consensus framework regions, such as human framework regions (see, for example, Chothia et al., 1998, J. Mol. Biol. 278: 457-479 for a listing of human framework regions).In certain embodiments, the polynucleotide generated by the combination of the framework regions and CDRs encodes an antibody that specifically binds to IFN alpha. Additionally, one or more amino acid substitutions may be made within the framework regions and the amino acid substitutions may improve binding of the antibody to its antigen. Additionally, such methods may be used to make amino acid substitutions or deletions of one or more variable region cysteine residues participating in an intrachain disulfide bond to generate antibody molecules lacking one or more intrachain disulfide bonds. Other alterations to the polynucleotide are encompassed by the present invention and within the skill of the art.

### 4.8 Peptides, Polypeptides and Fusion Proteins That Specifically Bind to IFN alpha

### 4.8.1 Peptide, Polypeptide and Fusion Protein Conjugates

The present invention also encompasses peptides, polypeptides and fusion proteins, which specifically bind to IFN alpha, fused to marker sequences, such as but not limited to, a peptide, to facilitate purification. In other embodiments, the marker amino acid sequence is a hexa-histidine peptide, such as the tag provided in a pQE vector (QIAGEN, Inc., 9259 Eton Avenue, Chatsworth, Calif., 91311), among others, many of which are commercially available. As described in Gentz et al., 1989, Proc. Natl. Acad. Sci. USA 86:821-824, for instance, hexa-histidine provides for convenient purification of the fusion protein. Other peptide tags useful for purification include, but are not limited to, the hemagglutinin "HA" tag, which corresponds to an epitope derived from the influenza hemagglutinin protein (Wilson et al., 1984, Cell 37:767) and the "FLAG" tag.

The present invention further encompasses peptides, polypeptides and fusion proteins that specifically bind to IFN alpha conjugated to a therapeutic moiety. A peptide, a polypeptide or a fusion protein that specifically binds to IFN alpha may be conjugated to a therapeutic moiety such as a cytotoxin, for example, a cytostatic or cytocidal agent, an agent which has a potential therapeutic benefit, or a radioactive metal ion, for example, alpha-emitters. A cytotoxin or cytotoxic agent includes any agent that is detrimental to cells. Examples of a cytotoxin or cytotoxic agent include, but are not limited to, paclitaxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1 - dehydrotestosterone, glucocorticoids, procaine, tetracaine, lidocaine, propranolol, and puromycin and analogs or homologs thereof. Other agents which have a potential therapeutic benefit include, but are not limited to, antimetabolites (for example, methotrexate, 6-mercaptopurine, 6-thioguanine, cytarabine, 5-fluorouracil decarbazine), alkylating agents (for example, mechlorethamine, thioepa chlorambucil, melphalan, carmustine (BSNU) and lomustine (CCNU), cyclothosphamide, busulfan, dibromomannitol, streptozotocin, mitomycin C, and cisdichlorodiamine platinum (II) (DDP) cisplatin), anthracyclines (for example, daunorubicin (formerly daunomdycin) and doxorubicin), antibiotics (for example, dactinomycin (formerly actinomycin), bleomycin, mithramycin, and anthramycin (AMC)), and anti-mitotic agents (for example, vincristine and vinblastine).

Furthermore, a peptide, a polypeptide or a fusion protein that specifically binds to IFN alpha may be conjugated to a therapeutic moiety or drug moiety that modifies a given biological response. Agents which have a potential therapeutic benefit or drug moieties are not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumor necrosis factor, IFN-α IFN-β, NGF, PDGF, TPA, an apoptotic agent, for example, TNF-α, TNF-β, AIM I (see, International Publication No. WO 97/33899), AIM II (see, International Publication No. WO 97/34911), Fas Ligand (Takahashi et al., 1994, J. Immunol., 6:1567-1574), and VEGF (see, International Publication No. WO 99/23105), a thrombotic agent or an anti-angiogenic agent, for example, angiostatin or endostatin; or, a biological response modifier such as, for example, a lymphokine (for example, IL-1, IL-2, IL-6, IL-10, GM-CSF, and G-CSF), or a growth factor (for example, GH).

### 4.8.2 Methods of Producing Polypeptides and Fusion Proteins

Peptides, polypeptides, proteins and fusion proteins can be produced by standard recombinant DNA techniques or by protein synthetic techniques, for example, by use of a peptide synthesizer. For example, a nucleic acid molecule encoding a peptide, polypeptide, protein or a fusion protein can be synthesized by conventional techniques including automated DNA synthesizers. Alternatively, PCR amplification of gene fragments can be carried out using anchor primers which give rise to complementary overhangs between two consecutive gene fragments which can subsequently be annealed and reamplified to generate a chimeric gene sequence (see, for example, Current Protocols in Molecular Biology, Ausubel et al., eds., John Wiley & Sons, 1992). Moreover, a nucleic acid encoding a bioactive molecule can be cloned into an expression vector containing the Fc domain or a fragment thereof such that the bioactive molecule is linked in-frame to the Fc domain or Fc domain fragment.

Methods for fusing or conjugating polypeptides to the constant regions of antibodies are known in the art. See, for example, U.S. Pat. Nos. 5,336,603, 5,622,929, 5,359,046, 5,349,053, 5,447,851, 5,723,125, 5,783,181, 5,908,626, 5,844,095, and 5,112,946; EP 307,434; EP 367,166; EP 394,827; International Publication Nos. WO 91/06570, WO 96/04388, WO 96/22024, WO 97/34631, land WO 99/04813; Ashkenazi et al., 1991, Proc. Natl. Acad. Sci. USA 88: 10535-10539; Traunecker et al, 1988, Nature, 331:84-86; Zheng et al., 1995, J. Immunol. 154:5590-5600; and Vil et al., 1992, Proc. Natl. Acad. Sci. USA 89:11337-11341, which are incorporated herein by reference in their entireties.

The nucleotide sequences encoding a bioactive molecule and an Fc domain or fragment thereof may be obtained from any information available to those of skill in the art (for example, from Genbank, the literature, or by routine cloning). The nucleotide sequences encoding IFN alpha ligands may be obtained from any available information, for example, from Genbank, the literature or by routine cloning. See, for example, Xiong et al., Science, 12;294(5541):339-45 (2001). The nucleotide sequence coding for a polypeptide a fusion protein can be inserted into an appropriate expression vector,. i.e., a vector which contains the necessary elements for the transcription and translation of the inserted protein-coding sequence. A variety of host-vector systems may be utilized in the present invention to express the protein-coding sequence. These include but are not limited to mammalian cell systems infected with virus (for example, vaccinia virus, adenovirus, etc.); insect cell systems infected with virus (for example, baculovirus); microorganisms such as yeast containing yeast vectors; or bacteria transformed with bacteriophage, DNA, plasmid DNA, or cosmid DNA. The expression elements of vectors vary in their strengths and specificities. Depending on the host-vector system utilized, any one of a number of suitable transcription and translation elements may be used.

The expression of a peptide, polypeptide, protein or a fusion protein may be controlled by any promoter or enhancer element known in the art. Promoters which may be used to control the expression of the gene encoding fusion protein include, but are not limited to, the SV40 early promoter region (Bemoist and Chambon, 1981, Nature 290:304-310), the promoter contained in the 3' long terminal repeat of Rous sarcoma virus (Yamamoto, et al., 1980, Cell 22:787-797), the herpes thymidine kinase promoter (Wagner et al., 1981, Proc. Natl. Acad. Sci. U.S.A. 78:1441-1445), the regulatory sequences of the metallothionein gene (Brinster et al., 1982, Nature 296:39-42), the tetracycline (Tet) promoter (Gossen et al., 1995, Proc. Nat. Acad. Sci. USA 89:5547-5551); prokaryotic expression vectors such as the β-lactamase promoter (Villa-Kamaroff et al., 1978, Proc. Natl. Acad. Sci. U.S.A. 75:3727-3731), or the tac promoter (DeBoer et al., 1983, Proc. Natl. Acad. Sci. U.S.A. 80:21-25; see also "Useful proteins from recombinant bacteria" in Scientific American, 1980, 242:74-94); plant expression vectors comprising the nopaline synthelase promoter region (Herrera-Estrella et al., Nature 303:209-213) or the cauliflower mosaic virus 35S RNA promoter (Gardner et al., 1981, Nucl. Acids Res. 9:2871), and the promoter of the photosynthetic enzyme ribulose biphosphate carboxylase (Herrera-Estrella et al., 1984, Nature 310:115-120); promoter elements from yeast or other fungi such as the Gal 4 promoter, the ADC (alcohol dehydrogenase) promoter, PGK (phosphoglycerol kinase) promoter, alkaline phosphatase promoter, and the following animal transcriptional control regions, which exhibit tissue specificity and have been utilized in transgenic animals: elastase I gene control region which is active in pancreatic acinar cells (Swift et al., 1984, Cell 38:639-646; Omitz et al., 1986, Cold Spring Harbor Symp. Quant. Biol. 50:399-409; MacDonald, 1987, Hepatology 7:425-515); insulin gene control region which is active in pancreatic beta cells (Hanahan, 1985, Nature 315:115-122), immunoglobulin gene control region which is active in lymphoid cells (Grosschedl et al., 1984, Cell 38:647-658; Adames et al., 1985, Nature 318:533-538; Alexander et al., 1987, Mol. Cell. Biol. 7:1436-1444), mouse mammary tumor virus control region which is active in testicular, breast, lymphoid and mast cells (Leder et al., 1986, Cell 45:485-495), albumin gene control region which is active in liver (Pinkert et al., 1987, Genes and Devel. 1:268-276), alpha-fetoprotein gene control region which is active in liver (Krumlauf et al., 1985, Mol. Cell. Biol. 5:1639-1648; Hammer et al., 1987, Science 235:53-58; alpha 1-antitrypsin gene control region which is active in the liver (Kelsey et al., 1987, Genes and Devel. 1:161-171), beta-globin gene control region which is active in myeloid cells (Mogram et al., 1985, Nature 315:338-340; Kollias et al., 1986, Cell 46:89-94; myelin basic protein gene control region which is active in oligodendrocyte cells in the brain (Readhead et al., 1987, Cell 48:703-712); myosin light chain-2 gene control region which is active in skeletal muscle (Sani, 1985, Nature 314:283-286); neuronal-specific enolase (NSE) which is active in neuronal cells (Morelli et al., 1999, Gen. Virol. 80:571-83); brain-derived neurotrophic factor (BDNF) gene control region which is active in neuronal cells (Tabuchi et al., 1998, Biochem. Biophysic. Res. Corn. 253:818-823); glial fibrillary acidic protein (GFAP) promoter which is active in astrocytes (Gomes et al., 1999, Braz J Med Biol Res 32(5):619-631; Morelli et al., 1999, Gen. Virol. 80:571-83) and gonadotropic releasing hormone gene control region which is active in the hypothalamus (Mason et al., 1986, Science 234:1372-1378).

In a specific embodiment, the expression of a peptide, polypeptide, protein or a fusion protein is regulated by a constitutive promoter. In another embodiment, the expression of a peptide, polypeptide, protein or a fusion protein is regulated by an inducible promoter. In another embodiment, the expression of a peptide, polypeptide, protein or a fusion protein is regulated by a tissue-specific promoter.

In a specific embodiment, a vector is used that comprises a promoter operably linked to a peptide-, polypeptide-, protein- or a fusion protein-encoding nucleic acid, one or more origins of replication, and, optionally, one or more selectable markers (for example, an antibiotic resistance gene).

In mammalian host cells, a number of viral-based expression systems may be utilized. In cases where an adenovirus is used as an expression vector, the polypeptide or fusion protein coding sequence may be ligated to an adenovirus transcription/translation control complex, for example, the late promoter and tripartite leader sequence. This chimeric gene may then be inserted in the adenovirus genome by *in vitro* or *in vivo* recombination. Insertion in a non-essential region of the viral genome (for example, region E1 or E3) will result in a recombinant virus that is viable and capable of expressing the antibody molecule in infected hosts (for example, see Logan & Shenk, 1984, Proc. Natl. Acad. Sci. USA 81:355-359). Specific initiation signals may also be required for efficient translation of inserted fusion protein coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (see Bittner et al., 1987, Methods in Enzymol. 153:51-544).

Expression vectors containing inserts of a gene encoding a peptide, polypeptide, protein or a fusion protein can be identified by three general approaches: (a) nucleic acid hybridization, (b) presence or absence of "marker" gene functions, and (c) expression of inserted sequences. In the first approach, the presence of a gene encoding a peptide, polypeptide, protein or a fusion protein in an expression vector can be detected by nucleic acid hybridization using probes comprising sequences that are homologous to an inserted gene encoding the peptide, polypeptide, protein or the fusion protein, respectively. In the second approach, the recombinant vector/host system can be identified and selected based upon the presence or absence of certain "marker" gene functions (for example, thymidine kinase activity, resistance to antibiotics, transformation phenotype, occlusion body formation in baculovirus, etc.) caused by the insertion of a nucleotide sequence encoding a polypeptide or a fusion protein in the vector. For example, if the nucleotide sequence encoding the fusion protein is inserted within the marker gene sequence of the vector, recombinants containing the gene encoding the: fusion protein insert can be identified by the absence of the marker gene function. In the third approach, recombinant expression vectors can be identified by assaying the gene product (for example, fusion protein) expressed by the recombinant. Such assays can be based, for example, on the physical or functional properties of the fusion protein in an *in vitro* assay systems, for example, binding with anti-bioactive molecule antibody.

In addition, a host cell strain may be chosen which modulates the expression of the inserted sequences, or modifies and processes the gene product in the specific fashion desired. Expression from certain promoters can be elevated in the presence of certain inducers; thus, expression of the genetically engineered fusion protein may be controlled. Furthermore, different host cells have characteristic and specific mechanisms for the translational and post-translational processing and modification (for example, glycosylation, phosphorylation of proteins). Appropriate cell lines or host systems can be chosen to ensure the desired modification and processing of the foreign protein expressed. For example, expression in a bacterial system will produce an unglycosylated product and expression in yeast will produce a glycosylated product. Eukaryotic host cells which possess the cellular machinery for proper processing of the primary transcript, glycosylation, and phosphorylation of the gene product may be used. Such mammalian host cells include, but are not limited to, CHO, VERY, BHK, Hela, COS, MDCK, 293, 3T3, W138, NS0, and in particular, neuronal cell lines such as, for example, SK-N-AS, SK-N-FI, SK-N-DZ human neuroblastomas (Sugimoto et al., 1984, J. Natl. Cancer Inst. 73: 51-57), SK-N-SH human neuroblastoma (Biochim. Biophys. Acta, 1982, 704: 450-460), Daoy human cerebellar medulloblastoma (He et al., 1992, Cancer Res. 52: 1144-1148) DBTRG-05MG glioblastoma cells (Kruse et al., 1992, In vitro Cell. Dev. Biol. 28A: 609-614), IMR-32 human neuroblastoma (Cancer Res., 1970, 30: 2110-2118), 1321N1 human astrocytoma (Proc. Natl Acad. Sci. USA, 1977, 74: 4816), MOG-G-CCM human astrocytoma (Br. J. Cancer, 1984, 49: 269), U87MG human glioblastoma-astrocytoma (Acta Pathol. Microbiol. Scand., 1968, 74: 465-486), A172 human glioblastoma (Olopade et al., 1992, Cancer Res. 52: 2523-2529), C6 rat glioma cells (Benda et al., 1968, Science 161: 370-371), Neuro-2a mouse neuroblastoma (Proc. Natl. Acad. Sci. USA, 1970, 65: 129-136), NB41A3 mouse neuroblastoma (Proc. Natl. Acad. Sci. USA, 1962, 48: 1184-1190), SCP sheep choroid plexus (Bolin et al., 1994, J. Virol. Methods 48: 211-221), G355-5, PG-4 Cat normal astrocyte (Haapala et al., 1985, J. Virol. 53: 827-833), Mpf ferret brain (Trowbridge et al., 1982, In vitro 18: 952-960), and normal cell lines such as, for example, CTX TNA2 rat normal cortex brain (Radany et al., 1992, Proc. Natl. Acad. Sci. USA 89: 6467-6471) such as, for example, CRL7030 and Hs578Bst. Furthermore, different vector/host expression systems may effect processing reactions to different extents.

For long-term, high-yield production of recombinant proteins, stable expression is preferred. For example, cell lines which stably express a polypeptide or a fusion protein may be engineered. Rather than using expression vectors which contain viral origins of replication, host cells can be transformed with DNA controlled by appropriate expression control elements (for example, promoter, enhancer, sequences, transcription termina-tors, polyadenylation sites, etc.), and a selectable marker. Following the introduction of the foreign DNA, engineered cells may be allowed to grow for 1-2 days in an enriched medium, and then are switched to a selective medium. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express a polypeptide or a fusion protein that specifically binds to IFN alpha. Such engineered cell lines may be particularly useful in screening and evaluation of compounds that affect the activity of a polypeptide or a fusion protein that specifically binds to IFN alpha. Selection systems, as discussed above may be used.

### 4.9 Therapeutic uses of the invention

Type I interferons are known to be immunoregulatory cytokines that are involved in T cell differentiation, antibody production and activity and survival of memory T cells. Moreover, increased expression of Type I interferons has been described in numerous autoimmune diseases, in HIV infection, in transplant rejection and in graft versus host disease (GVHD). Accordingly, the anti-IFN alpha antibodies of the invention or fragments thereof can be used in a variety of clinical indications involving aberrant or undesired Type I interferon activity. The invention encompasses methods of preventing, treating, maintaining, ameliorating, or inhibiting a Type I interferon-mediated disease or disorder, wherein the methods comprise administering antibodies, or antigen-binding portions thereof, of the invention.

Specific examples of autoimmune conditions in which antibodies of the invention can be used include, but are not limited to, the following: systemic lupus erythematosus (SLE), insulin dependent diabetes mellitus (IDDM), inflammatory bowel disease (IBD) (including Crohn's Disease, Ulcerative Colitis and Celiac's Disease), multiple sclerosis (MS), psoriasis, autoimmune thyroiditis, rheumatoid arthritis (RA) and glomerulonephritis. Furthermore, the antibody compositions of the invention can be used for inhibiting or preventing transplant rejection or in the treatment of graft versus host disease (GVHD) or in the treatment of HIV infection/AIDS.

High levels of IFNα have been observed in the serum of patients with systemic lupus erythematosus (SLE) (see *e.g*., Kim et al. (1987) Clin. Exp. Immunol. 70:562-569). Moreover, administration of IFNα, for example in the treatment of cancer or viral infections, has been shown to induce SLE (Garcia-Porrua et al. (1998) Clin. Exp. Rheumatol. 16:107-108). Accordingly, in another embodiment, anti-IFN alpha antibodies of the invention can be used in the treatment of SLE by administering the antibody to a subject in need of treatment.

Other methods of treating SLE are described in U.S. Patent Applications entitled "Methods of treating SLE" with the following serial numbers; 60/907, 767, filed April 16, 07; 60/966,174, filed November 5, 2007 and PCT application serial number PCT/US2007/02494, filed December 9, 2007 each of which are incorporated by reference in their entireties.

IFNα also has been implicated in the pathology of Type I diabetes. For example, the presence of immunoreactive IFNα in pancreatic beta cells of Type I diabetes patients has been reported (Foulis et al. (1987) Lancet 2:1423-1427). Prolonged use of IFNα in anti-viral therapy also has been shown to induce Type I diabetes (Waguri *et al.* (1994) Diabetes Res. Clin. Pract. 23:33-36). Accordingly, in another embodiment, the anti-IFN alpha antibodies or fragments thereof of the invention can be used in the treatment of Type I diabetes by administering the antibody to a subject in need of treatment. The antibody can be used alone or in combination with other anti-diabetic agents, such as insulin.

Treatment with IFNα has also been observed to induce autoimmune thyroiditis (Monzani et al. (2004) Clin. Exp. Med. 3:199-210; Prummel and Laurberg (2003) Thyroid 13:547-551). Accordingly, in another embodiment, anti-IFN alpha antibodies of the invention can be used in the treatment of autoimmune thyroid disease, including autoimmune primary hypothyroidism, Graves Disease, Hashimoto's thyroiditis and destructive thyroiditis with hypothyroidism, by administering an antibody of the invention to a subject in need of treatment. Antibodies of the invention can be used alone or in combination with other agents or treatments, such as anti-thyroid drugs, radioactive iodine and subtotal thyroidectomy.

High levels of IFNα also have been observed in the circulation of patients with HIV infection and its presence is a predictive marker of AIDS progression (DeStefano et al. (1982) J. Infec. Disease 146:451; Vadhan-Raj et al. (1986) Cancer Res. 46:417). Thus, in another embodiment, anti-IFN alpha antibodies of the invention may be used in the treatment of HIV infection or AIDS by administering the antibody of the invention to a subject in need of treatment. In another embodiment, antibodies of the invention can be used alone or in combination with other anti-HIV agents, such as nucleoside reverse transcriptase inhibitors, non-nucleoside reverse transcriptase inhibitors, protease inhibitors and fusion inhibitors.

Antibodies to IFNAR1 have been demonstrated to be effective in inhibiting allograft rejection and prolonging allograft survival (see *e.g.,* Tovey et al. (1996) J. Leukoc. Biol. 59:512-517; Benizri et al. (1998) J. Interferon Cytokine Res. 18:273-284). Accordingly, the anti-IFN alpha antibodies of the invention also can be used in transplant recipients to inhibit allograft rejection and/or prolong allograft survival. The invention provides a method of inhibiting transplant rejection by administering anti-IFN alpha antibodies of the invention to a transplant recipient in need of treatment. Examples of tissue transplants that can be treated include, but are not limited to, liver, lung, kidney, heat, small bowel, and pancreatic islet cells, as well as the treatment of graft versus host disease (GVHD). Antibodies of the invention can be used alone or in combination with other agents for inhibiting transplant rejection, such as immunosuppressive agents (*e.g*., cyclosporine, azathioprine, methylprednisolone, prednisolone, prednisone, mycophenolate mofetil, sirilimus, rapamycin, tacrolimus), anti-infective agents (*e.g*., acyclovir, clotrimazole, ganciclovir, nystatin, trimethoprimsulfarnethoxazole), diuretics (*e.g*., bumetanide, furosemide, metolazone) and ulcer medications (*e.g*., cimetidine, farnotidine, lansoprazole, omeprazole, ranitidine.

In another embodiment, the compositions of the invention are used to treat and prevent a wide range of inflammatory conditions including both chronic and acute conditions, such as appendicitis, peptic, gastric and duodenal ulcers, peritonitis, pancreatitis, ulcerative, pseudomembranous, acute and ischemic colitis, diverticulitis, epiglottitis, achalasia, cholangitis, cholecystitis, hepatitis, Crohn's disease, enteritis, Whipple's disease, asthma, allergy, anaphylactic shock, immune complex disease, organ ischemia, reperfusion injury, organ necrosis, hay fever, sepsis, septicemia, endotoxic shock, cachexia, hyperpyrexia, eosinophilic granuloma, granulomatosis, sarcoidosis, septic abortion, epididymitis, vaginitis, prostatitis, urethritis, bronchitis, emphysema, rhinitis, cystic fibrosis, pneumonitis, pneumoultramicroscopicsilicovolcanoconiosis, alvealitis, bronchiolitis, pharyngitis, pleurisy, sinusitis, influenza, respiratory syncytial virus infection, herpes infection, HIV infection, hepatitis B virus infection, hepatitis C virus infection, disseminated bacteremia, Dengue fever, candidiasis, malaria, filariasis, amebiasis, hydatid cysts, bums, dermatitis, dermatomyositis, sunburn, urticaria, warts, wheals, vasulitis, angiitis, endocarditis, arteritis, atherosclerosis, thrombophlebitis, pericarditis, myocarditis, myocardial ischemia, periarteritis nodosa, rheumatic fever, Alzheimer's disease, coeliac disease, congestive heart failure, restenosis, COPD adult respiratory distress syndrome, meningitis, encephalitis, multiple sclerosis, cerebral infarction, cerebral embolism, Guillame-Barre syndrome, neuritis, neuralgia, spinal cord injury, paralysis, uveitis, arthritides, arthralgias, osteomyelitis, fasciitis, Paget's disease, gout, periodontal disease, rheumatoid arthritis, synovitis, myasthenia gravis, thryoiditis, systemic lupus erythematosus, Goodpasture's syndrome, Behcets's syndrome, allograft rejection, graft-versus-host disease, Type I diabetes, ankylosing spondylitis, Berger's disease, Retier's syndrome, and Hodgkins disease.

In another embodiment, the compositions of the invention are used to may be useful in the prevention, treatment, amelioration of symptoms associated with the following conditions or disease states: Grave's disease, Hashimoto's thyroiditis, Crohn's disease, psoriasis, psoriatic arthritis, sympathetic opthalmitis, autoimmune oophoritis, autoimmune orchitis, autoimmune lymphoproliferative syndrome, antiphospholipid syndrome. Sjogren's syndrome, scleroderma, Addison's disease, polyendocrine deficiency syndrome, Guillan-Barre syndrome, immune thrombocytopenic purpura, pernicious anemia, myasthenia gravis, primary biliary cirrhosis, mixed connective tissue disease, vitiligo, autoimmune uveitis, autoimmune hemolytic anemia, autoimmune thrombopocytopenia, celiac disease, dermatitis herpetiformis, autoimmune hepatitis, pemphigus, pemphigus vulgaris, pemphigus foliaceus, bullous pemphigoid, autoimmune myocarditis, autoimmune vasculitis, alopecia areata, autoimmune artherosclerosis, Behcet's disease, autoimmune myelopathy, autoimmune hemophelia, autoimmune interstitial cystitis, autoimmune diabetes isipidus, autoimmune endometriosis, relapsing polychondritis, ankylosing spondylitis, autoimmune urticaria, dermatomyositis, Miller-Fisher syndrome, IgA nephropathy, goodpastures syndrome, and herpes gestationis.

In another embodiment, the compositions of the invention are used to may be useful in the prevention, treatment, amelioration of symptoms associated with the following conditions or disease states: Idiopathic inflammatory myopathies (IIM), Dermatomyositis (DM), Polymyositis (PM), and Inclusion body myositis (IBM).

In another embodiment, methods of administration and compositions of antibodies of the invention may be useful in the prevention, treatment, amelioration of symptoms associated with Sjogren's syndrome. Sjögren's syndrome is an autoimmune disorder in which immune cells attack and destroy the exocrine glands that produce tears and saliva. It is named after Swedish ophthalmologist Henrik Sjögren (1899-1986), who first described it. Sjögren's syndrome is also associated with rheumatic disorders such as rheumatoid arthritis, and it is rheumatoid factor positive in 90 percent of cases. The hallmark symptoms of the disorder are dry mouth and dry eyes. In addition, Sjögren's syndrome may cause skin, nose, and vaginal dryness, and may affect other organs of the body, including the kidneys, blood vessels, lungs, liver, pancreas, and brain. Nine out of ten Sjögren's patients are women and the average age of onset is late 40s, although Sjögren's occurs in all age groups in both women and men. It is estimated to strike as many as 4 million people in the United States alone, making it the second most common autoimmune rheumatic disease.

Myositis is general condition characterized by inflammation of skeletal muscle or voluntary muscle. Muscle inflammation may be caused by an allergic reaction, exposure to a toxic substance or medicine, another disease such as cancer or rheumatoid conditions, or a virus or other infectious agent. The chronic inflammatory myopathies are idiopathic, meaning they have no known cause. They are understood to be autoimmune disorders, in which the body's white blood cells (that normally fight disease) attack blood vessels, normal muscle fibers, and connective tissue in organs, bones, and joints.

Polymyositis affects skeletal muscles (involved with making movement) on both sides of the body. It is rarely seen in persons under age 18; most cases are in patients between the ages of 31 and 60. In addition to symptoms listed above, progressive muscle weakness leads to difficulty swallowing, speaking, rising from a sitting position, climbing stairs, lifting objects, or reaching overhead. Patients with polymyositis may also experience arthritis, shortness of breath, and heart arrhythmias.

Dermatomyositis is characterized by a skin rash that precedes or accompanies progressive muscle weakness. The rash looks patchy, with bluish-purple or red discolorations, and characteristically develops on the eyelids and on muscles used to extend or straighten joints, including knuckles, elbows, heels, and toes. Red rashes may also occur on the face, neck, shoulders, upper chest, back, and other locations, and there may be swelling in the affected areas. The rash sometimes occurs without obvious muscle involvement. Adults with dermatomyositis may experience weight loss or a low-grade fever, have inflamed lungs, and be sensitive to light. Adult dermatomyositis, unlike polymyositis, may accompany tumors of the breast, lung, female genitalia, or bowel. Children and adults with dermatomyositis may develop calcium deposits, which appear as hard bumps under the skin or in the muscle (called calcinosis). Calcinosis most often occurs 1-3 years after disease onset but may occur many years later. These deposits are seen more often in childhood dermatomyositis than in dermatomyositis that begins in adults. Dermatomyositis may be associated with collagen-vascular or autoimmune diseases.

Inclusion body myositis (IBM) is characterized by progressive muscle weakness and wasting. IBM is similar to polymyositis but has its own distinctive features. The onset of muscle weakness is generally gradual (over months or years) and affects both proximal and distal muscles. Muscle weakness may affect only one side of the body. Small holes called vacuoles are seen in the cells of affected muscle fibers. Falling and tripping are usually the first noticeable symptoms of IBM. For some patients the disorder begins with weakness in the wrists and fingers that causes difficulty with pinching, buttoning, and gripping objects. There may be weakness of the wrist and finger muscles and atrophy (thinning or loss of muscle bulk) of the forearm muscles and quadricep muscles in the legs. Difficulty swallowing occurs in approximately half of IBM cases. Symptoms of the disease usually begin after the age of 50, although the disease can occur earlier. Unlike polymyositis and dermatomyositis, IBM occurs more frequently in men than in women.

Juvenile myositis has some similarities to adult dermatomyositis and polymyositis. It typically affects children ages 2 to 15 years, with symptoms that include proximal muscle weakness and inflammation, edema (an abnormal collection of fluids within body tissues that causes swelling), muscle pain, fatigue, skin rashes, abdominal pain, fever, and contractures (chronic shortening of muscles or tendons around joints, caused by inflammation in the muscle tendons, which prevents the joints from moving freely). Children with juvenile myositis may also have difficulty swallowing and breathing, and the heart may be affected. Approximately 20 to 30 percent of children with juvenile dermatomyositis develop calcinosis. Juvenile patients may not show higher than normal levels of the muscle enzyme creatine kinase in their blood but have higher than normal levels of other muscle enzymes.

Accordingly, in other embodiments, antibodies of the invention may be useful in the prevention, treatment, or amelioration of myositis, inflammatory myositis, idiopathic myositis, polymyositis, dermatomyositis, inclusion body myositis (IBM), juvenile myositis or symptoms associated with these conditions.

In another embodiment, antibodies of the invention may be useful in the prevention, treatment, or amelioration of symptoms associated with vasculitis.

Antibodies of the invention may be useful for the treatment of scleroderma. Methods of treating Scleroderma are described in a U.S. patent application entitled "Methods Of Treating Scleroderma" with an application serial number of 60/996,175, filed on November 5, 2007 and incorporated by reference in its entirety for all purposes.

In another embodiment, antibodies of the invention may be useful in the prevention, treatment, or amelioration of symptoms associated with sarcoidosis. Sarcoidosis (also called sarcoid or Besnier-Boeck disease) is an immune system disorder characterized by non-necrotizing granulomas (small inflammatory nodules). Virtually any organ can be affected; however, granulomas most often appear in the lungs or the lymph nodes. Symptoms can occasionally appear suddenly but usually appear gradually. When viewing X-rays of the lungs, sarcoidosis can have the appearance of tuberculosis or lymphoma.

Antibodies and composition of the invention may be useful in the regulation of IFN-I responsive genes. IFN-I responsive genes have been identified in US Patent Applications entitled "IFN alpha-induced Pharmacodynamic Markers" with the following serial numbers; 60/873,008, filed December 6, 2006; 60/907,762, filed April 16, 2007; 60/924, 584, filed May 21, 2007; 60/960,187, filed September 19, 2007; 60/966, 176, filed November 5, 2007 and PCT application serial number PCT/US2007/02494, filed December 6, 2007 each of which are incorporated by reference in their entireties.

### 4.10 Combinations

Compositions of the invention also can be administered in combination therapy, such as, combined with other agents. For example, the combination therapy can include an anti-IFN alpha antibody of the present invention combined with at least one other immunosuppressent.

In some methods, two or more monoclonal antibodies with different binding specificities are administered simultaneously, in which case the dosage of each antibody administered falls within the ranges indicated. The antibody is usually administered on multiple occasions. Intervals between single dosages can be, for example, weekly, monthly, every three months or yearly. Intervals can also be irregular as indicated by measuring blood levels of antibody to the target antigen in the patient. In some methods, dosage is adjusted to achieve a plasma antibody concentration of about 1-1000 µg /ml and in some methods about 25-300 µg /ml.

When antibodies to IFN alpha are administered together with another agent, the two can be administered in either order or simultaneously. For example, an anti-IFN alpha antibody of the invention can be used in combination with one or more of the following agents: drugs containing mesalamine (including sulfasalazine and other agents containing 5-aminosalicylic acid (5-ASA), such as olsalazine and balsalazide), non-steroidal anti-inflammatory drugs (NSAIDs), analgesics, corticosteroids (*e.g*., predinisone, hydrocortisone), TNF-inhibitors (including adalimumab (HUMIRA®), etanercept (ENBREL®) and infliximab (REMICADE®)), immunosuppressants (such as 6-mercaptopurine, azathioprine and cyclosporine A), and antibiotics anti-IFNAR1 antibody, anti-IFNγ receptor antibody, and soluble IFNγ receptor.

In other embodiments, the compositions of the invention may also include agents useful in the treatment of SLE. Such agents include analgesics, corticosteroids (*e.g*., predinisone, hydrocortisone), immunosuppressants (such as cyclophosphamide, azathioprine, and methotrexate), antimalarials (such as hydroxychloroquine) and biologic drugs that inhibit the production of dsDNA antibodies (*e.g*., LJP 394).

### 4.11 Specific Embodiments

1. A method of producing an antibody with a decreased deamidation profile, wherein said antibody would otherwise be predisposed to an elevated deamidation profile.
2. The method of embodiment 1, wherein said method comprises the use of mammalian cells.
3. The method of embodiment 1, wherein said mammalian cells are selected from the group consisting of NS0, CHO, MDCK, or HEK cells.
4. The method of embodiment 1, wherein said antibody comprises an asparagine residue preceding and adjacent to a glycine, serine, threonine or an aspartic acid residue, as read N-terminus to C-terminus.
5. The method of embodiment 4, wherein said residues are located in at least one of the VHCDR1, VHCDR2, VHCDR3, VLCDR1, VLCDR2, or VLCDR3 regions of said antibody.
6. The method of embodiment 5, wherein said residues are located in the VHCDR2 of said antibody.
7. The method of any of embodiments 1-6, wherein said antibody deamidation profile is decreased by about 60%, about 50%, about 40%, about 30%, about 20%, or about 10% as compared to a control deamidation profile.
8. The method of any of embodiments 1-7, wherein said method comprises production of an antibody from cells grown at a temperature in the range of between about 30°C to about 37°C.
9. The method of any of embodiments 1-8, wherein said temperature is about 34°C.
10. The method of any of embodiments 1-9, wherein said method comprises production of an antibody from cells grown in media at a pH from the range of between about 6.0 to about 7.2 pH units.
11. The method of any of embodiments 1-10, wherein said pH is about 6.9 pH units.
12. The method of any of embodiments 1-11, wherein said method comprises production of an antibody from cells grown in a biphasic culture.
13. The method of embodiment 12, wherein said biphasic culture comprises at least one temperature shift.
14. The method of embodiment 13, wherein said temperature shift comprises a shift from about 34°C to about 32°C.
15. The method of embodiment 14, wherein said temperature shift occurs on or after the cell culture density has reached 1 x 10⁶ cells/ml.
16. The method of any of embodiments 1-15, wherein said method comprises a pH change of the media at the time of harvest.
17. The method of any of embodiments 1-16, wherein said pH is adjusted to a range of about 5.0 to about 7.0 pH units.
18. The method of any of embodiments 1-17, wherein said pH is adjusted to about 6.9 pH units.
19. The method of any of embodiments 1-18, wherein said method comprises a hold step after cell harvest, said hold step comprising a pH change.
20. The method of any of embodiments 1-19, wherein said pH is adjusted to a range of about 5.0 to about 7.0 pH units.
21. The method of any of embodiments 1-20, wherein said method comprises a dilution step.
22. The method of any of embodiments 1-21, wherein said dilution step is an in-line dilution or a tank dilution step.
23. The method of any of embodiments 1-22, wherein said method does not include an ultrafiltration step.
24. The method of any of embodiments 1-23, wherein said method has a residence time of less than about 17 days.
25. The method of embodiment 24, wherein said method has a residence time of about 13 days.
26. The method of any of embodiments 1-25, wherein said antibody is specific for interferon alpha.
27. The method of any of embodiments 1-26, wherein said antibody is 13H5.
28. A method of producing an antibody with a decreased deamidation profile, wherein said antibody would otherwise be predisposed to an elevated deamidation profile, said method comprising the following steps:
   a. producing said antibody from cells grown at a temperature from about 33°C to about 35°C, wherein said cells are grown in media with a pH value of about 6.7 to about 7.1 pH units; and
   b. culturing said cells for about 13 to about 19 days.
29. The method of embodiment 28, wherein said cells are cultured for 13 days.
30. The method of embodiment 28, wherein said antibody is 13H5.
31. A stable monoclonal antibody composition with a decreased deamidation profile, wherein said antibody comprises amino acid sequences that predispose said antibody to an elevated deamidation profile.
32. The composition of embodiment 31, wherein said antibody is an anti-interferon alpha antibody.
33. The composition of embodiment 31 or 32, wherein said antibody comprises an asparagine residue preceding and adjacent to a glycine, serine, threonine or an aspartic acid residue, as read N-terminus to C-terminus.
34. The composition of any of embodiments 31-33, wherein said residues are located in at least one of the VHCDR1, VHCDR2, VHCDR3, VLCDR1, VLCDR2, or VLCDR3 regions of said antibody.
35. The composition of any of embodiments 31-34, wherein said residues are located in the VHCDR2 of said antibody.
36. The composition of any of embodiments 31-35, wherein said antibody deamidation profile is decreased by about 60%, about 50%, about 40%, about 30%, about 20%, or about 10% as compared to a control deamidation profile.
37. The composition of any of embodiments 31-36, wherein said antibody is an antibody fragment.
38. The composition of any of embodiments 31-37, wherein said antibody fragment is selected from the group consisting of a Fab fragment, a F(ab')2 fragment, a Fab' fragment, and an scFv.
39. The antibody composition of any of embodiments 31-38, wherein said composition is produced by a process comprising growing antibody producing cells at a temperature of about 34°C, wherein said antibody producing cells are grown in media with a pH of about 6.9 pH units.
40. The antibody composition of any of embodiments 31-39, wherein said composition is produced by a process comprising;
   a. growing antibody producing cells at a first temperature of about 34°C;
   b. shifting said cells to a second temperature of about 32°C, when the cell density reaches about 1 x 10⁶ cells/ml; and
   c. said antibody producing cells are grown in media with a pH of about 6.9 pH units.
41. An antibody composition with a decreased deamidation profile, wherein said antibody is otherwise predisposed to an elevated deamidation profile, produced by the process comprising, growing antibody producing cells at about 34°C, wherein said antibody producing cells are grown in media with a pH of about 6.9 pH units.
42. The antibody composition of embodiment 41, wherein said composition is produced by the process further comprising shifting said temperature to about 32°C at or after the cell density reaches about 1 x 10⁶ cells/ml.
43. An antibody composition with a decreased deamidation profile, wherein said antibody is otherwise predisposed to an elevated deamidation profile, produced by the process comprising growing antibody producing cells at about 32°C to about 35°C, wherein said cells are grown in a media with a pH of about 6.7 to about 7.1 units, and culturing said antibody producing cells for about 12 to about 19 days.
44. The antibody composition of embodiment 43, wherein said cells are grown at about 34°C.
45. The antibody composition of embodiment 43 or 44, wherein said cells are grown in a media with a pH of about 6.9 pH units.
46. The antibody composition of any of embodiments 41-45, wherein said cells are cultured for about 13 days.
47. The composition of any of embodiments 41-46 wherein, said antibody is 13H5.
48. A method of purifying an antibody predisposed to an elevated deamidation profile, wherein said method comprises a wash step during purification for removal of the deamidated species of said antibody.
49. The method of embodiment 48, wherein said wash step comprises a buffer with a salt concentration of about 0 mM to about 100 mM.
50. The method of embodiment 48 or 49, wherein said salt concentration is about 30 mM.
51. The method of any of embodiments 48-50, wherein said buffer is sodium phosphate.
52. The method of any of embodiments 48-51, wherein said method comprises an ion-exchange chromatography step.
53. The method of any of embodiments 48-52, further comprising the method of any of embodiments 1-30.

### 4.12 Equivalents

Those skilled in the art will recognize, or be able to ascertain using no more than routine experimentation, many equivalents to the specific embodiments of the invention described herein. Such equivalents are intended to be encompassed by the following claims.

All publications, patents and patent applications mentioned in this specification are herein incorporated by reference into the specification to the same extent as if each individual publication, patent or patent application was specifically and individually indicated to be incorporated herein by reference. In addition, the following United States provisional patent applications: 60/909,117 and 60/909,232 both filed March 30, 2007 are hereby incorporated by reference herein in their entireties for all purposes.

### 5. Examples

### 5.1 Example 1: Deamidation is reduced by altering the cell culture conditions for production

Methods: Standard cell culture processes are well documented in the art. Altering certain parameters for growth and viability of the production cell line may yield a higher titre of product. In this example, cell culture conditions such as temperature and pH were adjusted to reduce the deamidation of the desired product. Specifically, the temperature of the cell culture was lowered from the standard 37°C to 34°C. In addition, the pH of the media the cells were cultured in was lowered from the standard pH 7.2 to 6.9. The deamidation profile of the desired product was analyzed by standard ion-exchange chromatography methods. The percent deamidation was determined by the area under the curve (AUC) method for the elution profile from the ion-exchange chromatography column.

**Table 1: Varying cell culture parameters affects deamidation of the desired product.**

| Cell culture Run # | Temperature (°C) | pH | Deamidation % |
|---|---|---|---|
| 1 | 37 | 7.2 | 50 |
| 2 | 34 | 7.2 | 61 |
| 3 | 34 | 7.2 | 70 |
| 4 | 34 | 6.9 | 17 |

Results: Documented in Table 1 are the results from cell culture production runs 1-4. Run 1 involved cells grown at the standard cell culture conditions, 37°C, pH 7.2. The resultant deamidation profile of the desired protein product was 50 %. Cell culture runs 2 and 3 involved lowering the temperature of the process to 34°C with no change in pH. The resultant deamidation percentages were 61% and 70% respectively. In cell culture run 4, two parameters were adjusted. The temperature was adjusted down to 34°C while the pH was also lowered to 6.9 for the duration of the growth and production phases. The deamidation profile of the resultant product was 17% using the combined temperature and pH shift. This reduction in deamidation percentage is a significant and unexpected improvement over the current standard cell culture run which resulted in a much higher deamidation profile. These results suggest that the combination of reduction in temperature and a reduction in pH lead to a surprisingly synergistic effect, with the end result being a dramatic reduction in deamidation percentage of the desired protein product.

### 5.2 Example 2: Varying the timing of harvest reduces the deamidation of the desired protein product

Standard protein production technologies suggest that harvesting on Day 14 of a cell culture run leads to an optimal recovery of desired protein product. In this example the harvesting time parameter was adjusted to determine the effect on the deamidation state of the desired protein product.

Methods: In the large scale production of proteins, the harvesting time runs can be varied over the course of the cell culture run. In this working example the harvest date was varied from day 9 day 14, and day 17 post inoculation. Cell culture runs were performed under similar conditions for each trial. The deamidation profile of the desired product was analyzed by standard ion-exchange chromatography methods. The percent deamidation was determined by the area under the curve method for the elution profile from the ion-exchange chromatography column.

**Table 2: Harvest timing alters the deamidation of the desired protein product**

| Cell culture run | Harvest day | Deamidation % |
|---|---|---|
| 5 | Day 9 | 17% |
| 6 | Day 14 | 21% |
| 7 | Day 17 | 24% |

Results: Documented in Table 2 are the results from independent cell culture runs harvested on various days to determine the effect on the deamidation profile of the desired protein. As demonstrated, the earlier the desired protein is harvested the lower the exhibited deamidation profile. Therefore these results suggest that altering the harvest timing affects the deamidation state of the desired product. Accordingly, the harvesting of the desired protein earlier in the production run leads to a surprisingly and unexpected lowered deamidation percentage.

### 5.3 Example 3: Adjusting the pH at harvest to decrease the deamidation of the desired protein product

Methods: Upon harvest, the conditioned media containing the protein of interest is subjected to a pH shift downwards with the addition of a suitable acid. The resultant pH would be less than the pH cells were cultured at. It is postulated that the resultant pH would be at or near 6.5 or lower. This pH adjustment downwards would slow the rate of deamidation and therefore increase the rate of recovery of the desired protein product. The actual deamidation percentage of the desired protein product could be determined using the percent area under the curve of standard ion-exchange chromatography.

### 5.4 Example 4: Adjusting the pH after harvest to decrease the deamidation of the desired protein product

Methods: After harvest, the conditioned media containing the protein of interest was subjected to a pH shift downwards with the addition of a suitable acid. Control samples were maintained at pH 7.0 whereas test samples were adjusted to pH 6.0. Both sets of samples were maintained at 2-8°C for the entire duration. During the duration of the experiment, samples were taken from the two conditions and analyzed for deamidation of the desired protein product. The actual deamidation percentage of the desired protein product was determined using the percent area under the curve of standard ion-exchange chromatography. The results of these experiments are presented in Table 3.

**Table 3: Lowering of pH reduces the rate of deamidation**

| Time Point (Days) | % Deamidation (% under the curve) | |
|---|---|---|
| | pH 6.0 | pH 7.0 |
| 0 | 24.5 | 24.5 |
| 7 | - | 31.4 |
| 33 | - | 33.5 |
| 56 | 28.3 | - |

Results: As shown in Table 3, as time progresses product maintained at pH 7.0 undergoes a faster rate of deamidation compared to product maintained at pH 6.0. This pH adjustment downwards slowed the rate of deamidation and therefore increases the rate of recovery of the desired protein product. These data suggest that the stability of the protein product is maintained at lower pH values after the cell culture run.

### 5.5 Example 5: Adjusting the temperature after harvest to decrease the deamidation of the desired product

Methods: After harvest, the conditioned media containing the protein of interest was subjected to a temperature shift downwards to 2-8°C. Control samples were maintained at 15-25°C. Samples were held at pH 7.2 for up to 8 weeks. The actual deamidation percentage of the desired protein product was determined using the percent area under the curve of standard ion-exchange chromatography. The results of these experiments are presented in Table 4

**Table 4.**

| Time Point (Weeks) | % Deamidation (Area under the curve) | |
|---|---|---|
| | 2-8 °C | 15-25 °C |
| 0 | 22.5 | 22.5 |
| 1 | - | 52.8 |
| 2 | 25.4 | 74.2 |
| 4 | 29.5 | - |
| 8 | 35.6 | - |

Results: As shown in Table 4, as time progresses product maintained at the higher temperature (15-25°C) undergoes a faster rate of deamidation compared to product maintained at the lower temperature (2-8°C). This temperature adjustment downwards slowed the rate of deamidation and therefore increases the rate of recovery of the desired protein product. These data suggest that the stability of the protein product is maintained at lower temperatures after the cell culture run.

### 5.6 Example 6: Adjusting the wash buffer steps to increase the recovery of the desired protein product

Methods: To increase the resolution of the cation exchange chromatography and the recover of the desired protein product, the wash steps within the protocol were adjusted. The resultant experimentation with the ionic strength of the wash buffer allowed for the selective removal of unwanted protein species, such as deamidated protein, from the desired protein product. To determine the optimal ionic strength to remove unwanted deamidated species from the desired protein product, variations of a linear solute gradient were tested. Columns were loaded with pH adjusted conditioned medium as outlined below. After wash2, bound 13H5 was eluted in a linear salt gradient (0 - 100 mM NaCl in 35 mM sodium phosphate pH 6.2) at various gradient slopes (gradient lengths 10, 20, 30, 40 column volumes (CV)). Elution peaks were fractionated and measured for percent deamidated content by analytical HPLC ion-exchange chromatography. IEC chromatograms corresponding to these fractions are shown together with a reference standard IEC profile. Early eluting peaks in these analytical chromatograms correspond to acidic or deamidated subspecies of the 13H5 antibody.

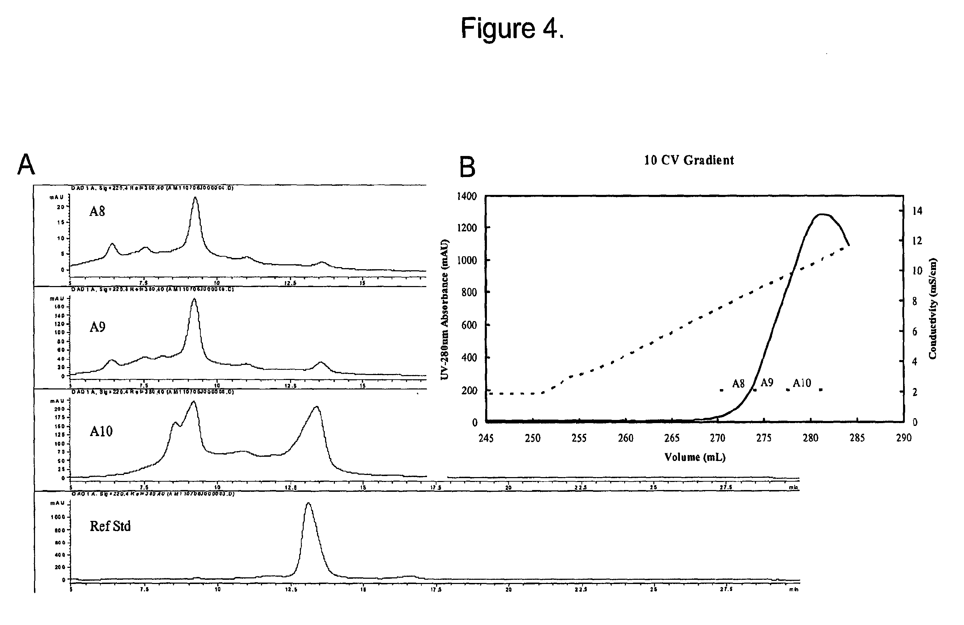

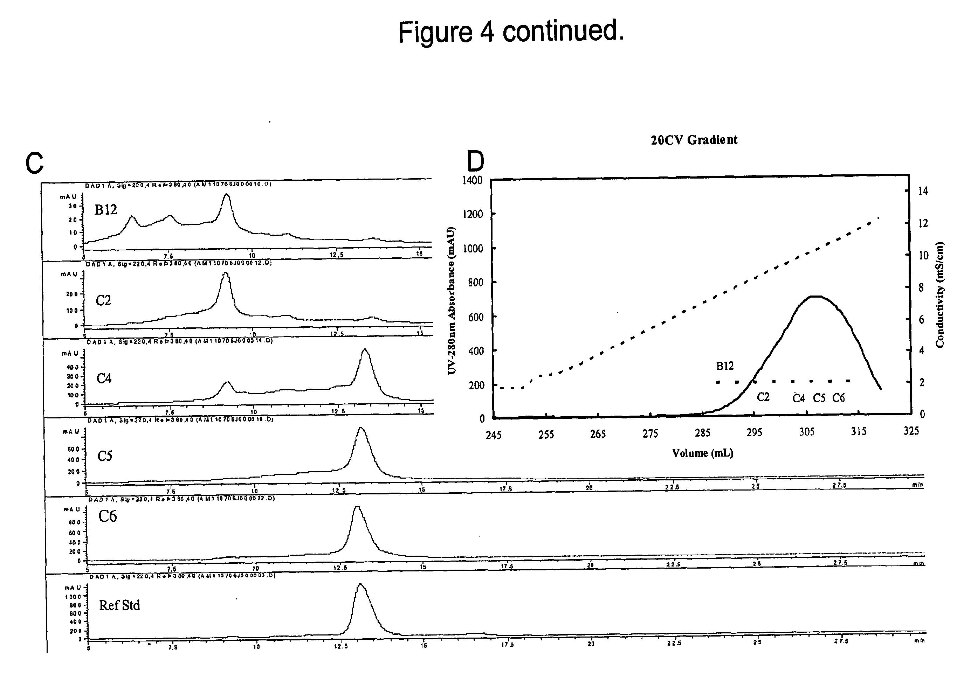

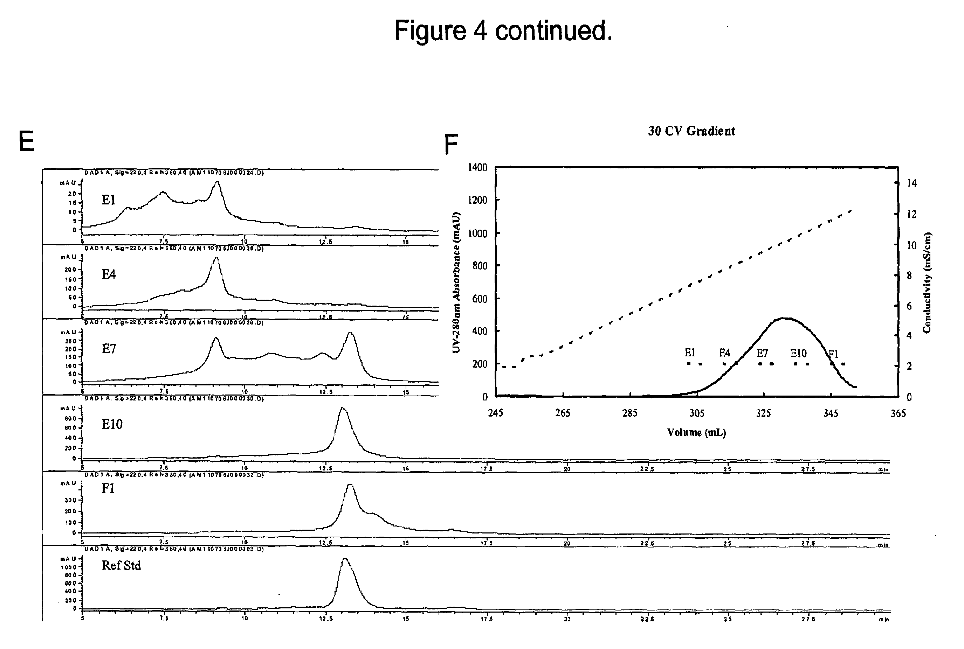

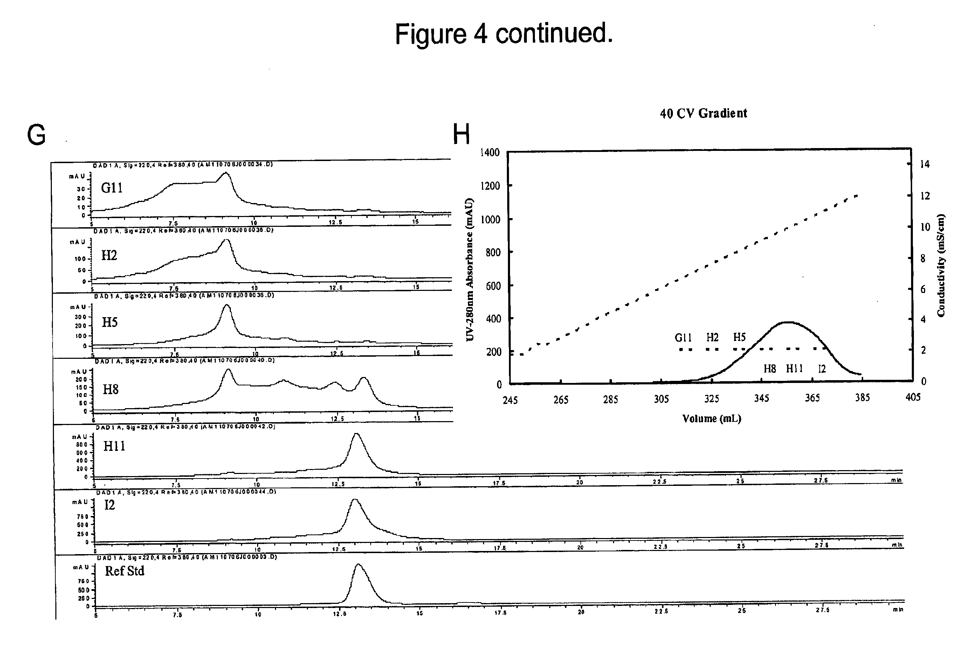

Results: As can be seen in these plots (Fig 4 A-H), by using a linear salt gradient, deamidated species can be resolved from the intact 13H5 molecule and resolution improves at lower gradient slopes (extended gradient length). By expanding these experiments (increase number of runs and number of analyzed peak fractions), these results can be extended to the application/optimization of step elution. Depending on the final desired yield and percent deamidated content in the eluted cation-exchange product, any salt concentration in the range of 0-100 mM NaCl (in sodium phosphate buffer) may be selected as Wash3 to target removal of deamidated species.

### 5.7 Example 7 - Estimation of optimal harvest day

During bioreactor production, 13H5 deamidation occurs at a rate that is primarily controlled by the pH and temperature of the cell culture broth, among other factors. Since the antibody is expected to be intact once excreted from the cell, the final percentage of deamidated 13H5 at harvest will depend on, among other factors, the overall bioreactor lifetime. In other words, at harvest, antibody produced earlier in the cycle is exposed to unfavorable conditions (high pH and temperature) for a considerably longer time than antibody produced later in the cycle. An experimental determination of the total percent deamidation at various bioreactor time-points has indeed shown that earlier samples contain less deamidated antibody than later samples. Therefore, the total percent deamidation can potentially be controlled by cutting back on the bioreactor harvest date to where more favorable conditions that minimize deamidation can then be established. However, because antibody production continues throughout the bioreactor lifetime, a trade-off between total productivity and deamidation becomes evident.

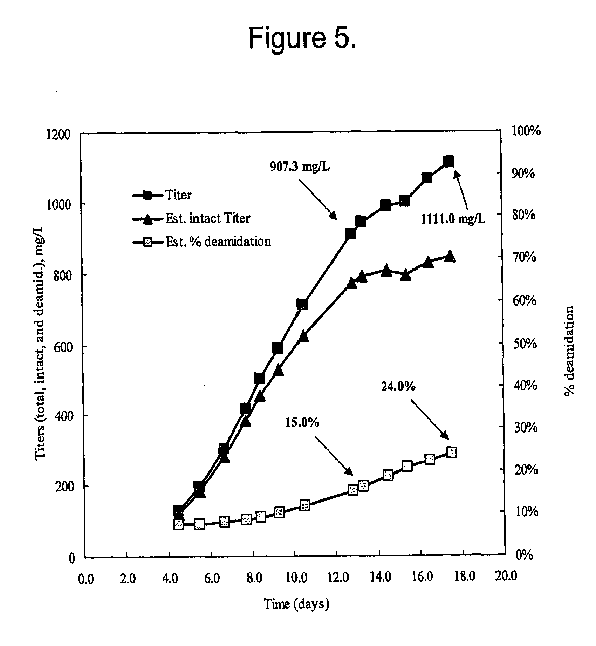

Results: Figure 5 shows the measured productivity of 13H5 as a function of bioreactor lifetime. In this particular case, the final titer after 18 days was measured as 1.1 g/L. This measurement includes both intact and deamidated 13H5. The estimated percent deamidation at each time point is estimated and consequently, the estimated "intact" 13H5 concentration is also shown. It should be noted that in this case, percent deamidation was measured only at day 18 (harvest). Earlier deamidation time points are estimates based on first order deamidation kinetics (fixed deamidation rate constant). This approach enables bioreactor analysis and optimization. For example, it becomes clear that although total 13H5 production continues beyond day 13 (increasing from 0.9 g/L at day 13 to 1.1 g/L at day 18), the intact antibody titer curve is virtually flat during this same time period. Thus in this example, harvesting the bioreactor at day 13 would result in a final percent deamidation that is considerably lower (15%) than at day 18 (24%) with a minimal loss in overall intact antibody productivity.

Here is the list of the requested sequences:

SEQUENCE LISTING

<110>  MedImmune, Inc.
       Ram, Kripa
       Venkat, Raghavan

<120>  Antibodies with Decreased Deamidation Profiles

<130>  IA151PCT

<140>  PCTUS0858133
<141>  2008-03-25

<150>  US 60/909,232
<151>  2007-03-30

<150>  US 60/909,117
<151>  2007-03-30

<160>  50

<170>  PatentIn version 3.3

<210>  1
<211>  446
<212>  PRT
<213>  Artificial

<220>
<223>  Unknown

<400>  1

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Ser Ile Ser Trp Val Arg Gln Ala Pro Gly Gln Gly Leu Glu Trp Met

        35                  40                  45

Gly Trp Ile Ser Val Tyr Asn Gly Asn Thr Asn Tyr Ala Gln Lys Phe
    50                  55                  60

Gln Gly Arg Val Thr Met Thr Thr Asp Thr Ser Thr Ser Thr Ala Tyr
65                  70                  75                  80

Leu Glu Leu Arg Ser Leu Arg Ser Asp Asp Thr Ala Val Tyr Tyr Cys
                85                  90                  95

Ala Arg Asp Pro Ile Ala Ala Gly Tyr Trp Gly Gln Gly Thr Leu Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Lys Val Glu Pro Lys Ser Cys Asp Lys Thr His Thr
    210                 215                 220

Cys Pro Pro Cys Pro Ala Pro Glu Leu Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
        260                 265                 270

Lys Phe Asn Trp Tyr Val Asp Gly Val Glu Val His Asn Ala Lys Thr
    275                 280                 285

Lys Pro Arg Glu Glu Gln Tyr Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Ile Glu Lys Thr Ile Ser
                325                 330                 335

Lys Ala Lys Gly Gln Pro Arg Glu Pro Gln Val Tyr Thr Leu Pro Pro
        340                 345                 350

Ser Arg Glu Glu Met Thr Lys Asn Gln Val Ser Leu Thr Cys Leu Val
    355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His
        420                 425                 430

Asn His Tyr Thr Gln Lys Ser Leu Ser Leu Ser Pro Gly Lys
        435                 440                 445

<210>  2
<211>  116
<212>  PRT
<213>  Artificial

<220>
<223>  Unknown

<400>  2

<210>  3
<211>  348
<212>  DNA
<213>  Artificial

<220>
<223>  Unknown

<400>  3
caggttcagc tggtgcagtc tggagctgag gtgaagaagc ctggggcctc agtgaaggtc      60

tcctgcaagg cttctggtta cacctttacc agctatagta tcagctgggt gcgacaggcc     120

cctggacaag ggcttgagtg gatgggatgg atcagcgttt acaatggtaa cacaaactat     180

gcacagaagt tccagggcag agtcaccatg accacagaca catccacgag cacagcctac     240

ctggagctga ggagcctgag atctgacgac acggccgtgt attactgtgc gagagatccc     300

atagcagcag gctactgggg ccagggaacc ctggtcaccg tctcctca                  348

<210>  4
<211>  5
<212>  PRT
<213>  Artificial

<220>
<223>  Unknown

<400>  4

Ser Tyr Ser Ile Ser
1               5

<210>  5
<211>  15
<212>  DNA
<213>  Artificial

<220>
<223>  Unknown

<400>  5
agctatagta tcagc                                                        15

<210> 6
<211> 17
<212> PRT
<213> Artificial

<220>
<223> Unknown

<400> 6

Trp Ile Ser Val Tyr Asn Gly Asn Thr Asn Tyr Ala Gln Lys Phe Gln
1               5                   10                  15

Gly

<210>  7
<211>  51
<212>  DNA
<213>  Artificial

<220>
<223>  Unknown

<400>  7
tggatcagcg tttacaatgg taacacaaac tatgcacaga agttccaggg c                51

<210>  8
<211>  7
<212>  PRT
<213>  Artificial

<220>
<223>  Unknown

<400>  8

Asp Pro Ile Ala Ala Gly Tyr
1               5

<210>  9
<211>  21
<212>  DNA
<213>  Artificial

<220>
<223>  Unknown

<400>  9
gatcccatag cagcaggcta c                                                21

<210>  10
<211>  215
<212>  PRT
<213>  Artificial

<220>
<223>  Unknown

<400>  10

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Thr
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Pro
                85                  90                  95

Arg Thr Phe Gly Gln Gly Thr Lys Val Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210>  11
<211>  108
<212>  PRT
<213>  Artificial

<220>
<223>  Unknown

<400>  11

<210>  12
<211>  324
<212>  DNA
<213>  Artificial

<220>
<223>  Unknown

<400>  12
gaaattgtgt tgacgcagtc tccaggcacc ctgtctttgt ctccagggga aagagccacc      60

ctctcctgca gggccagtca gagtgttagc agcacctact tagcctggta ccagcagaaa     120

cctggccagg ctcccaggct cctcatctat ggtgcatcca gcagggccac tggcatccca     180

gacaggttca gtggcagtgg gtctgggaca gacttcactc tcaccatcag cagactggag     240

cctgaagatt ttgcagtgta ttactgtcag cagtatggta gctcacctcg gacgttcggc     300

caagggacca aggtggaaat caaa                                            324

<210> 13
<211> 12
<212> PRT
<213> Artificial

<220>
<223> Unknown

<400> 13

Arg Ala Ser Gln Ser Val Ser Ser Thr Tyr Leu Ala
1               5                   10

<210>  14
<211>  36
<212>  DNA
<213>  Artificial

<220>
<223>  Unknown

<400>  14
agggccagtc agagtgttag cagcacctac ttagcc                                36

<210>  15
<211>  7
<212>  PRT
<213>  Artificial

<220>
<223>  Unknown

<400>  15

Gly Ala Ser Ser Arg Ala Thr
1               5

<210>  16
<211>  21
<212>  DNA
<213>  Artificial

<220>
<223>  Unknown

<400>  16
ggtgcatcca gcagggccac t                                                21

<210>  17
<211>  9
<212>  PRT
<213>  Artificial

<220>
<223>  Unknown

<400>  17

Gln Gln Tyr Gly Ser Ser Pro Arg Thr
1               5

<210>  18
<211>  27
<212>  DNA
<213>  Artificial

<220>
<223>  Unknown

<400>  18
cagcagtatg gtagctcacc tcggacg                                          27

<210>  19
<211>  121
<212>  PRT
<213>  Artificial

<220>
<223>  Unknown

<400>  19

Gln Val Gln Leu Gln Glu Ser Gly Pro Gly Leu Met Lys Pro Ser Glu
1               5                   10                  15

Thr Leu Ser Leu Thr Cys Thr Val Ser Gly Gly Ser Val Ser Ser Gly
            20                  25                  30

Ser Tyr Tyr Trp Ser Trp Ile Arg Gln Pro Pro Gly Met Gly Leu Glu
        35                  40                  45

Trp Ile Gly Tyr Ile Tyr Ser Gly Gly Gly Ala Asn Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Asn Ser Val Thr Ala Ala Asp Thr Ala Val Tyr Phe
                85                  90                  95

Cys Ala Arg Gly Ile Pro Met Val Arg Gly Ile Leu His Tyr Trp Gly
            100                 105                 110

Gln Gly Thr Leu Val Thr Val Ser Ser
        115                 120

<210>  20
<211>  363
<212>  DNA
<213>  Artificial

<220>
<223>  Unknown

<400>  20
caggtgcagc tgcaggagtc gggcccagga ctgatgaagc cttcggagac cctgtccctc      60

acctgcactg tctctggtgg ctccgtcagc agtggtagtt actactggag ctggatccgg     120

cagcccccag ggatgggact ggagtggatt ggttatatct attccggggg aggcgccaac     180

tacaaccctt ccctcaagag tcgagtcacc atatcagtgg acacgtccaa gaaccagttc     240

tccctgaagc tgaactctgt gaccgctgcg gacacggccg tgtatttctg tgcgagagga     300

attcctatgg ttcggggaat tcttcactac tggggccagg gaaccctggt caccgtctcc     360

tca                                                                   363

<210>  21
<211>  7
<212>  PRT
<213>  Artificial

<220>
<223>  Unknown

<400>  21

Ser Gly Ser Tyr Tyr Trp Ser
1               5

<210>  22
<211>  21
<212>  DNA
<213>  Artificial

<220>
<223>  Unknown

<400>  22
agtggtagtt actactggag c                                                21

<210>  23
<211>  16
<212>  PRT
<213>  Artificial

<220>
<223>  Unknown

<400>  23

Tyr Ile Tyr Ser Gly Gly Gly Ala Asn Tyr Asn Pro Ser Leu Lys Ser
1               5                   10                  15

<210>  24
<211>  48
<212>  DNA
<213>  Artificial

<220>
<223>  Unknown

<400>  24
tatatctatt ccgggggagg cgccaactac aacccttccc tcaagagt                48

<210>  25
<211>  11
<212>  PRT
<213>  Artificial

<220>
<223>  Unknown

<400>  25

Gly Ile Pro Met Val Arg Gly Ile Leu His Tyr
1               5                   10

<210>  26
<211>  33
<212>  DNA
<213>  Artificial

<220>
<223>  Unknown

<400>  26
ggaattccta tggttcgggg aattcttcac tac                                    33

<210>  27
<211>  108
<212>  PRT
<213>  Artificial

<220>
<223>  Unknown

<400>  27

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Phe Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Pro
                85                  90                  95

Tyr Thr Phe Gly Gln Gly Thr Lys Leu Glu Ile Lys
            100                 105

<210>  28
<211>  324
<212>  DNA
<213>  Artificial

<220>
<223>  Unknown

<400>  28
gaaattgtgt tgacgcagtc tccaggcacc ctgtctttgt ctccagggga aagagccacc      60

ctctcctgca gggccagtca gagtgttagc agcagcttct tagcctggta ccagcagaaa     120

cctggccagg ctcccaggct cctcatctat ggtgcatcca gcagggccac tggcatccca     180

gacaggttca gtggcagtgg gtctgggaca gacttcactc tcaccatcag cagactggag     240

cctgaagatt ttgcagtgta ttactgtcag cagtatggta gctcaccgta cacttttggc     300

caggggacca agctggagat caaa                                            324

<210> 29
<211> 12
<212> PRT
<213> Artificial

<220>
<223> Unknown

<400> 29

Arg Ala Ser Gln Ser Val Ser Ser Ser Phe Leu Ala
1               5                   10

<210>  30
<211>  36
<212>  DNA
<213>  Artificial

<220>
<223>  Unknown

<400>  30
agggccagtc agagtgttag cagcagcttc ttagcc                                36

<210>  31
<211>  7
<212>  PRT
<213>  Artificial

<220>
<223>  Unknown

<400>  31

Gly Ala Ser Ser Arg Ala Thr
1               5

<210> 32
<211> 21
<212> DNA
<213> Artificial

<220>
<223> Unknown

<400> 32
ggtgcatcca gcagggccac t 21

<210> 33
<211> 9
<212> PRT
<213> Artificial

<220>
<223> Unknown

<400> 33

Gln Gln Tyr Gly Ser Ser Pro Tyr Thr
1               5

<210>  34
<211>  27
<212>  DNA
<213>  Artificial

<220>
<223>  Unknown

<400>  34
cagcagtatg gtagctcacc gtacact                                          27

<210>  35
<211>  116
<212>  PRT
<213>  Artificial

<220>
<223>  Unknown

<400>  35

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ser Ser Tyr
            20                  25                  30

Gly Ile Ser Trp Val Arg Gln Ala Pro Gly Gln Gly Leu Glu Trp Met
        35                  40                  45

Gly Trp Ile Ser Ala Tyr Asn Gly Asn Thr Asn Tyr Leu Gln Lys Leu
    50                  55                  60

Gln Gly Arg Val Thr Leu Thr Thr Asp Thr Ser Thr Asn Thr Ala Tyr
65                  70                  75                  80

Met Glu Leu Arg Ser Leu Arg Ser Asp Asp Thr Ala Val Tyr Tyr Cys
                85                  90                  95

Thr Arg Asp Pro Ile Ala Ala Gly Tyr Trp Gly Gln Gly Thr Leu Val
            100                 105                 110

Thr Val Ser Ser
        115

<210>  36
<211>  348
<212>  DNA
<213>  Artificial

<220>
<223>  Unknown

<400>  36
caggttcagc tggtgcagtc tggagctgag gtgaagaagc ctggggcctc agtgaaggtc      60

tcctgcaagg cttctggtta tacctttacc agctatggta tcagctgggt gcgacaggcc     120

cctggacaag ggcttgagtg gatgggatgg atcagcgctt acaatggtaa cacaaactat     180

gcacagaagc tccagggcag agtcaccatg accacagaca catccacgag cacagcctac     240

atggagctga ggagcctgag atctgacgac acggccgtgt attactgtgc gagagatccc     300

atagcagcag gttactgggg ccagggaacc ctggtcaccg tctcctca                  348

<210>  37
<211>  5
<212>  PRT
<213>  Artificial

<220>
<223>  Unknown

<400>  37

Ser Tyr Gly Ile Ser
1               5

<210>  38
<211>  15
<212>  DNA
<213>  Artificial

<220>
<223>  Unknown

<400>  38
agctatggta tcagc                                                        15

<210>  39
<211>  17
<212>  PRT
<213>  Artificial

<220>
<223>  Unknown

<400>  39

Trp Ile Ser Ala Tyr Asn Gly Asn Thr Asn Tyr Leu Gln Lys Leu Gln
1               5                   10                  15

Gly

<210>  40
<211>  51
<212>  DNA
<213>  Artificial

<220>
<223>  Unknown

<400>  40
tggatcagcg cttacaatgg taacacaaac tatctacaga agctccaggg c                51

<210>  41
<211>  7
<212>  PRT
<213>  Artificial

<220>
<223>  Unknown

<400>  41

Asp Pro Ile Ala Ala Gly Tyr
1               5

<210>  42
<211>  21
<212>  DNA
<213>  Artificial

<220>
<223>  Unknown

<400>  42
gatcccatag cagcaggtta c                                                21

<210>  43
<211>  108
<212>  PRT
<213>  Artificial

<220>
<223>  Unknown

<400>  43

<210> 44
<211> 324
<212> DNA
<213> Artificial

<220>
<223> Unknown

<400> 44
gaaattgtgt tgacgcagtc tccaggcacc ctgtctttgt ctccagggga aagagccacc 60

ctctcctgca gggccagtca gagtgttagc agcacctact tagcctggta ccagcagaaa 120

cctggccagg ctcccaggct cctcatctat ggtgcatcca gcagggccac tggcatccca 180

gacaggttca gtggcagtgg gtctgggaca gacttcactc tcaccatcag cagactggag 240

cctgaagatt ttgcagtgta ttactgtcag cagtatggta gctcacctcg gacgttcggc 300

caagggacca aggtggaaat caaa 324

<210> 45
<211> 12
<212> PRT
<213> Artificial

<220>
<223> Unknown

<400> 45

Arg Ala Ser Gln Ser Val Ser Ser Thr Tyr Leu Ala
1               5                   10

<210>  46
<211>  36
<212>  DNA
<213>  Artificial

<220>
<223>  Unknown

<400>  46
agggccagtc agagtgttag cagcacctac ttagcc                                36

<210>  47
<211>  7
<212>  PRT
<213>  Artificial

<220>
<223>  Unknown

<400>  47

Gly Ala Ser Ser Arg Ala Thr
1               5

<210>  48
<211>  21
<212>  DNA
<213>  Artificial

<220>
<223>  Unknown

<400>  48
ggtgcatcca gcagggccac t                                                21

<210> 49
<211> 9
<212> PRT
<213> Artificial

<220>
<223> Unknown

<400> 49

Gln Gln Tyr Gly Ser Ser Pro Arg Thr
1               5

<210>  50
<211>  27
<212>  DNA
<213>  Artificial

<220>
<223>  Unknown

<400>  50
cagcagtatg gtagctcacc tcggacg                                          27

## Claims

1. A stable monoclonal antibody composition with a decreased deamidation profile, wherein the antibody comprises amino acid sequences that predispose the antibody to an elevated deamidation profile.

2. The composition of claim 1, wherein the antibody is an anti-interferon alpha antibody.

3. The composition of claim 1 or 2, wherein the antibody comprises an asparagine residue preceding and adjacent to a glycine, serine, threonine or an aspartic acid residue, as read N-terminus to C-terminus.

4. The composition of any one of claims 1-3, wherein the residues are located in at least one of the VHCDR1, VHCDR2, VHCDR3, VLCDR1, VLCDR2, or VLCDR3 regions of the antibody.

5. The antibody of any one of claims 1-4, wherein the residues are located in the VHCDR2 of the antibody.

6. The composition of any one of claims 1-5, wherein the antibody deamindation profile is decreased by about 60%, about 50%, about 40%, about 30%, about 20%, or about 10% as compared to a control deamidation profile.

7. The composition of any one of claims 1-6, wherein the antibody is an antibody fragment.

8. The composition of any one of claims 1-7, wherein the antibody fragment is selected from the group consisting of a Fab fragment, a F(ab')2 fragment, a Fab' fragment, and an scFv.

9. The composition of any one of claims 1-8, wherein the composition is produced by a process comprising:
a) growing antibody producing cells at a first temperature of about 34°C;
b) shifting the cells to a second temperature of about 32°C, when the cell density reaches about 1 x 10⁶ cells/ml; and
c) culturing the cells in media with a pH of about 6.9 pH units.

10. The composition of any one of claims 1-9, wherein the antibody is 13H5.

11. An antibody composition with a decreased deamidation profile, wherein the antibody is otherwise predisposed to an elevated deamidation profile, produced by a process comprising culturing the antibody producing cells at about 34°C in a growth media having a pH of about 6.9 pH units.

12. The antibody composition of claim 11, wherein the composition is produced by the process further comprising shifting the temperature to about 32°C at or after the cell density reaches about 1 x 10⁶ cells/ml.

13. The antibody composition of claim 11 or 12, wherein the antibody producing cells are cultured for about 12 to about 19 days.

14. The antibody composition of any one of claims 11-13, wherein the antibody is 13H5.
